(19) Europäisches Patentamt
European Patent Office
Office européen des brevets

(11) Veröffentlichungsnummer: **0 170 048 B1**

(12) # EUROPÄISCHE PATENTSCHRIFT

(45) Veröffentlichungstag der Patentschrift: **17.04.91**

(51) Int. Cl.5: **C07C 321/08**, A61K 31/195, C07C 323/14, C07K 5/06, A61K 37/02

(21) Anmeldenummer: **85107869.1**

(22) Anmeldetag: **25.06.85**

(54) **Aliphatische Thioether.**

(30) Priorität: **28.06.84 CH 3115/84**
**12.02.85 CH 617/85**

(43) Veröffentlichungstag der Anmeldung:
**05.02.86 Patentblatt 86/06**

(45) Bekanntmachung des Hinweises auf die Patenterteilung:
**17.04.91 Patentblatt 91/16**

(84) Benannte Vertragsstaaten:
**AT BE CH DE FR GB IT LI LU NL SE**

(56) Entgegenhaltungen:
**EP-A- 0 063 858**
**EP-A- 0 121 350**
**EP-A- 0 123 543**

**TETRAHEDRON LETTERS, Band 21, Nr. 33, 1980, Seiten 3143-3146, Pergamon Press Ltd., GB; E.J. COREY et al.: "Total synthesis of slow reacting substances (SRS)"**

(73) Patentinhaber: **CIBA-GEIGY AG**
**Klybeckstrasse 141**
**CH-4002 Basel(CH)**

(72) Erfinder: **Von Sprecher, Andreas, Dr.**
**Nelkenweg 5**
**CH-4104 Oberwil(CH)**
Erfinder: **Ernest, Ivan, Dr.**
**Rüttihard 10**
**CH-4127 Birsfelden(CH)**
Erfinder: **Main, Alan Joseph, Dr.**
**164 Meyersville Road**
**Chagham Township, NJ 07928(US)**
Erfinder: **Beck, Andreas, Dr.**
**Kohlerweg 23**
**W-7800 Freiburg(DE)**

(74) Vertreter: **Zumstein, Fritz, Dr. et al**
**Bräuhausstrasse 4**
**W-8000 München 2(DE)**

**EP 0 170 048 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neuartige unsymmetrische aliphatische Thioether abgeleitet vom Rest (A) einer Mercaptoalkancarbonsäure, wie der Mercaptoessigsäure, ß-Mercaptopropionsäure, eines am Stickstoffatom gegebenenfalls acylierten Cysteins oder Cysteinpeptids, oder eines Salzes oder eines am Carboxyl abgewandelten Derivates einer solchen Säure, deren Schwefelatom mit einem mindestens 12 Kohlenstoffatome aufweisenden olefinischen Rest (B) substituiert ist, welcher in α-Stellung zum Schwefelatom auf einer Seite seiner Kette ein in bezug auf das S-Atom trans -orientiertes Hydroxyl, auf der anderen Seite eine Doppelbindung trägt.

Insbesondere betrifft die Erfindung Verbindungen der Formel

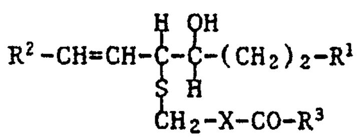

(I)

worin

- $R^1$ ein $C_{1-3}$-Alkyl oder ein $C_{1-3}$-Hydroxyalkyl, dessen Hydroxylgruppe in veresterter Form vorliegen kann,
- $R^2$ einen gegebenenfalls ungesättigten aliphatischen Rest mit 5-15 C-Atomen,
- $R^3$ Hydroxyl, Alkoxy oder eine gegebenenfalls substituierte Aminogruppe, und
- -X- eine Einfachbindung, eine Methylengruppe oder eine gegebenenfalls N-acylierte primäre Aminomethylengruppe darstellt, wobei das O-Atom dar Hydroxylgruppe mit dem S-Atom in der relativen trans -Konfiguration sind, sowie Salze solcher Verbindungen mit salzbildenden Eigenschaften.

Die Raumdarstellung in der obigen Formel I ist so zu verstehen, dass die Symbole der ersten Zeile oberhalb, die der dritten Zeile dann unterhalb der Darstellungsebene liegen (oder umgekehrt), was für die abgebildete Formel der gegenseitigen Konfiguration (RS)-(SR) beider centralen Kohlenstoffatome gemäss der Kahn-Ingold-Prelog-Konvention entspricht.

Gegenstand der Erfindung bilden auch Verfahren zur Herstellung der oben definierten erfindungsgemässen Verbindungen, sowie pharmazeutische Zusammensetzungen, welche diese Verbindungen als Wirkstoff enthalten, und entsprechende Herstellungsverfahren, mit welchen solche Zusammensetzungen auf nicht-chemischem Wege hergestellt werden. - Ein weiterer Gegenstand der Erfindung ist die therapeutische Anwendung der oben definierten Verbindungen und pharmazeutischen Zusammensetzungen, insbesondere bei Linderung und Behebung solcher krankhafter Zustände, bei welchen die ausgeprägte Leukotrien-antagonisierende Wirksamkeit der erfindungsgemässen Verbindungen zur Geltung kommt, wie bei Allergien verschiedener Art, vor allem bei Asthma.

Vor einigen Jahren wurde nachgewiesen, dass Isolate aus biologischem Material verschiedener Herkunft, welche als SRS (s low-r eacting s ubstance of anaphylaxis) von immunologischen Studien her bekannt waren, vgl. H.R. Morris et al. Nature 285, 1045-106 (May 1980) und L. Oerning, S. Hammarström und B. Samuelsson: Proc. Natl. Acad. Sci. USA 77 (4), 2014-2017 (1980), mit den vom Studium des Arachidonsäure-Metabolismus bekannten sogenannten Leukotrienen identisch sind. So geht z.B. aus beiden letztgenannten Arbeiten klar hervor, dass der als SRS-A bezeichnete Wirkstoff, welcher mit höchster Wahrscheinlichkeit als ein primärer Vermittler von sofort einsetzenden Ueberempfindlichkeits-Reaktionen für die Bronchokonstriktion bei Asthma verantwortlich ist, mit dem sogenannten Leukotrien D identischist (vgl. die nachstehende Formel LTD). Analog wirksam ist auch Leukotrien C, dessen Raumstruktur vor kurzem [E.J. Corey et al., J. Am. Chem. Soc. 102 (4), 1436-1439 (1980)] auch durch die Totalsynthese bestätigt wurde.

Das strukturelle Grundgerüst der Leukotriene allgemein wird durch eine mehrfach ungesättigte, lineare Eicosansäure gebildet, die charakteristische Substituenten in 1-, 5- und 6-Stellung trägt, wie es für die erwähnten wichtigsten Vertreter formelmässig abgebildet wird:

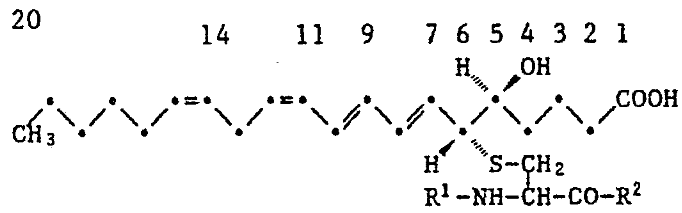

LTC-4: $R^1$ = $HOCOCH(NH_2)CH_2CH_2CO-$ ; $R^2$ = $-NHCH_2COOH$
LTD-4: $R^1$ = H- ; $R^2$ = $-NHCH_2COOH$
LTE-4: $R^1$ = H- ; $R^2$ = -OH

[Hier ist die Raumdarstellung so zu verstehen, dass die ganze olefinische Kette in der Darstellungsebene liegt und die mit Pfeil angegebenen Valenzstriche sich oberhalb der Darstellungsebene, die punktierten dagegen unterhalb der Ebene befinden.]

In ihren physiologischen Eigenschaften zeichnen sich Leukotriene im allgemeinen dadurch aus, dass sie eine markante Kontraktion von glatten Muskeln verschiedenster Art verursachen. Vom Standpunkt der Gesundheit ist ein solcher Effekt meistens unerwünscht, und dementsprechend steht die Suche nach geeigneten Leukotrien-Antagonisten im Vordergrund der Forschung auf diesem Gebiet.

Ueberraschenderweise hat sich nun gezeigt, dass die erfindungsgemässen Verbindungen der Formel I, obwohl sie mehrere Strukturmerkmale mit bekannten Leukotrienen gemeinsam haben, diesen gegenüber eine ausgeprägte antagonistische Wirkung ausüben. So wirken sie in verschiedenen Testanordnungen in vitro deutlich Leukotrienantagonisierend.

So hemmen sie z.B. im getesteten Konzentrationsbereich von etwa 0,1-25 µMol/1 die durch Leukotrien-$D_4$ ($LTD_4$ - siehe oben) induzierte Kontraktion eines glatten Muskels hemmen. Dieser sogenannte $LTD_4$-Antagonismus wird experimentell z.B. folgendermassen ermittelt: In Segmenten, die dem Ileum eines 300-400 g schweren Meerschweinchens entnommen wurden und in einem Organbad in Tyrode-Lösung bei 38°c und unter Begasung mit einem Gemisch von 95 % Sauerstoff und 5 % Kohlenstoffdioxid bei einer Belastung von 1 g inkubiert wurden, werden mit synthetischem Leukotrien $D_4$ (als Kaliumsalz) Kontraktionen ausgelöst und isotonisch registriert. Das Ausmass der Hemmung durch die Prüfsubstanz wird nach einer Vorinkubation von 2 Minuten ermittelt und als $IC_{50}$ , d.h. die Konzentration, welche die Testkontraktion um 50 % reduziert, ausgewertet. Der $LTD_4$-Antagonismus kann auch in vivo durch Bronchokonstriktions-Standardtest am Meerschweinchen bei Aerosol-Verabreichung nachgewiesen werden. (Die Beschreibung der Testmethode befindet sich im Anhang nach den Beispielen).

In einer anderen Testanordnung hemmen Verbindungen der Formel I im getesteten Konzentrationsbereich von etwa 1-100 µMol/l die durch Leukotrien $B_4$ ($LTB_4$ induzierte Aggregation von peritonealen Leukocyten der Ratte. Bei experimenteller Durchführung werden Wistar-Ratten (400-600 g) 24 Stunden nach i.p. Injektion von 16 ml 12%iger Natrium Kaseinat-Lösung getötet, Zellen werden vom Peritoneum mit gepuffertem Eagles minimal essential medium (E-MEM) ausgewaschen, je 0,5 ml Zellsuspension ($10^7$ Zellen in 1 ml E-MEM) wird in die Küvette eines Plättchenaggregometers gebracht und unter ständigem Rühren (800-900 rpm) auf 37°C erwärmt. Vier Minuten nach Zugabe der Prüfsubstanz (2 µl) wird die Aggregation durch 2 µl $LTB_4$(1 ng/ml Endkonzentration) ausgelöst und durchgehend registriert. Die Konzentration der Prüfsubstanz, welche die Kontrollaggregation ($LTB_4$ allein) um 50 % reduziert, wird als $IC_{50}$ bezeichnet.

Ueberraschenderweise üben Verbindungen der Formel I auch eine ausgeprägte Hemmwirkung auf andere physiologisch wichtige Enzymsysteme aus. So wurde die Hemmung von Phospholipase $A_2$ aus menschlichen Leukocyten im getesteten Konzentrationsbereich von etwa 0,5-50 µMol/l beobachtet. (Die experimentelle Anordnung für diese Bestimmung ist im Anhang nach den Beispielen näher beschrieben.) Ebenfalls wurde die Hemmung von Phospholipase C aus menschlichen Thrombocyten im getesteten Konzentrationsbereich von etwa 1-100 µMol/l beobachtet (für experimentelle Anordnung siehe den Anhang nach den Beispielen).

Die durch diese Methoden in vitro angedeuteten antiallergischen bzw. antiinflammatorischen Eigenschaften werden auch im Tierversuch in vivo bestätigt. So lässt sich die lokale antiinflammatorische Wirksamkeit beispielsweise nach der von G. Tonelli und L. Thibault [Endocrinology 77 , 625 (1965)] entwickelten Methode durch Hemmung des mit Crotonöl induzierten Rattenohroedems bei der Normalratte im Dosisbereich von etwa 1 bis etwa 100 mg/ml nachweisen.

Dank diesen wertvollen pharmakologischen Eigenschaften können die erfindungsgemässen Verbindungen der Formel I überall dort therapeutische Anwendung finden, wo die allergogene Wirkung der Leukotriene zu krankhaften Zuständen führt und zu mildern oder zu beheben ist. Demzufolge können sie beispielsweise zur Behandlung allergischer Zustände und Erkrankungen, wie insbesondere Asthma, aber auch Heufieber sowie obstruktiver Lungenkrankheiten einschliesslich zystischer Fibrose, verwendet werden. Ebenfalls sind sie, dank ihrer antiinflammatorischen Wirksamkeit, als entzündungshemmende Mittel, insbesondere als externe (topische) Hautphlogistatika für die Behandlung entzündlicher Dermatosen jeglicher Genese, wie bei leichten Hautirritationen, Kontaktdermatitis, Exanthemen und Verbrennungen, sowie als Schleimhautphlogostatika für die Behandlungvon Mukosaentzündungen, z.B. der Augen, Nase, Lippen, Mund und Genital-, bzw. Analregion, geeignet. Ferner können sie als Sonnenschutzmittel verwendet werden. Die hohe hemmende Wirksamkeit auf verschiedene Blutfaktoren weist zudem auf die Möglichkeit der therapeutischen Anwendung der Verbindungen der Formel I im Indikationsbereich Thrombose und Blutgerinnung hin.

Wie bereits oben erwähnt wurde, ist im Strukturaufbau der erfindungsgemässen Verbindungen der Formel I und Leukotrienen allgemeine Analogie zu finden, insbesondere in der eingangs besprochenen obligatorischen trans-Konfiguration des vicinalen S- und O-Atoms und der gesamten Struktur des Mercaptoalkansäure-Rests A (insbesondere in seiner typischen Form eines Cystein-Peptids). Von Leukotrienen unterscheiden sie sich dagegen wesentlich dadurch, dass bei ihnen im olefinischen Rest (B) die charakteristische terminale Carboxylgruppe fehlt. Im Unterschied zu Leukotrienen ist bei ihnen auch die Anzahl, Charakter und Raumordnung der Mehrfachbindungen sowie die Gesamtlänge des olefinischen Rests (B) für die Wirksamkeit in breiten Grenzen nebensächlich und sogar die absolute Konfiguration beider oben diskutierten asymmetrischen C-Atome ist für die Wirksamkeit nicht kritisch, wie man es am Beispiel des hochwirksamen N-[S-5(R),6(S)-5-Hydroxy-7,9-trans-11-cis-eicosatrien-6-yl-N-trifluoracetyl-cysteinyl]-glycin-Natriumsalzes zeigen kann, welches die gegenüber natürlichen Leukotirenen umgekehrte absolute Konfiguration der Kohlenstoffatome 5 und 6 der KW-Kette hat.

In der oben definierten Formel I steht Symbol $R^1$ vorzugsweise für ein Alkyl, wie Methyl, Propyl und vor allem Ethyl, oder auch für ein entsprechendes Hydroxyalkyl, vorzugsweise $\omega$-Hydroxyalkyl, wie insbesondere $\beta$-Hydroxyethyl, wobei die Hydroxylgruppe nicht nur in freier, sondern auch in veresterter Form vorliegen kann. Die veresterte Hydroxylgruppe ist vorzugsweise durch den Rest einer höchstens 12 C-Atome aufweisenden aliphatischen oder aromatischen Carbonsäure, wie Benzoesäure oder insbesondere einer $C_{1-7}$-Alkansäure, vor allem Essigsäure, verestert.

Der durch Symbol $R^2$ dargestellte aliphatische Rest ist vorzugsweise ein linearer Rest, z.B. ein Alkylrest, bestehend aus 5-15, vorzugsweise 7-12 C-Atomen, wie insbesondere Heptyl, Nonyl, Undecyl und Dodecyl, oder ein entsprechender einfach oder mehrfach ungesättigter Rest, der eine, zwei oder drei mehrfache Bindungen, wie Dreifachbindungen und vor allem Doppelbindungen beliebig in cis-oder trans -Konfiguration, in beliebigen Kombinationen trägt. Diese Mehrfachbindungen befinden sich vorzugsweise möglichst nahe dem Schwefelatom, d.h. konjugiert mit der ersten Doppelbindung, die in $\alpha,\beta$-Stellung zum schwefeltragenden C-Atom liegt. Bevorzugte Reste $R^2$ dieser Art sind z.B. 1-Alkenyl-, 1,3-Alkadienyl- und 1,3,6-Alkatrienyl-Reste, wie insbesondere 1-Heptenyl, 1-Octenyl, 1-Nonenyl, 1-Decenyl, 1-Undecenyl und 1-Dodecenyl bzw. 1,3-Octadienyl, 1,3-Decadienyl, 1,3-Dodecadienyl sowie 1,3,6-Dodecatrienyl, in welchen allen die Doppelbindungen je einzeln in cis-oder trans -Konfiguration vorliegen und beliebige Kombinationen bilden können.

Das in der eingangs angegebenen Formel I definierte Symbol $R^3$ bildet zusammen mit der benachbarten Carbonylgruppe -CO- einen freien oder funktionell abgewandelten Carboxylrest; falls $R^3$ für Hydroxyl steht, bildet es mit der Carbonylgruppe den Carboxylrest einer freien Carbonsäure, falls es ein Alkoxy bedeutet, insbesondere ein solches mit höchstens 7 C-Atomen, vor allem Methoxy, ergänzt es einen Carbonsäureester, und falls es für eine Aminogruppe steht, gehört es zur amidischen Bindung eines Carboxamids oder, falls die Aminogruppe geeignet substituiert ist, eines Peptids. Im letztgenannten Fall ist die substituierte Aminogruppe das Grundelement einer Aminosäure, wie einer $\alpha$-Aminocarbonsäure und insbesondere einer $\alpha$-Amino-$C_{2-7}$-Alkansäure, vorzugsweise einer solchen, die in der Natur vorkommt, wie Leucin, Valin, Alanin (insbesondere in der "natürlichen" L-Form) und vor allem Glycin. Das Carboxyl dieser Aminosäuren kann dann wiederum als freies Carboxyl vorliegen oder in der oben definierten Weise als eine Estergruppe, wie insbesondere ein $C_{1-7}$-Alkoxycarbonyl, oder die Carboxamidogruppe $-CONH_2$ funtkionell abgewandelt sein. Derartige bevorzugte Bedeutungen des Symbols $R^3$ entsprechen dann der Partialformel

$$\begin{array}{c} R^3_a \\ | \\ -NH-CH-CO-R^3_b \end{array} \qquad (R^3_o)$$

worin $R_a^3$ ein $C_{1-5}$-Alkyl oder vorzugsweise Wasserstoff und $R_b^3$ Hydroxyl, $C_{1-7}$-Alkoxy oder die primäre Aminogruppe $NH_2$ darstellt.

Das eingangs definierte Symbol -X- kann einerseits eine einfache C-C-Bindung darstellen, und somit zusammen mit den benachbarten Gruppen den Rest der Mercaptoessigsäure -S-$CH_2$-CO-$R^3$ bilden; in diesem Fall ist unter den obgenannten Bedeutungen von $R^3$ Hydroxyl besonders bevorzugt. Andererseits kann -X- eine gegebenenfalls am Stickstoffatom acylierte Aminomethylengruppe darstellen, die dann der Partialformel

$$R^4\text{-NH-CH-} \qquad (\text{-}X_o\text{-})$$

entspricht, worin $R^4$ Wasserstoff oder den Acylrest einer Carbonsäure bedeutet, wie einer aliphatischen oder aromatischen Carbonsäure mit höchstens 12 C-Atomen, insbesondere einer unsubstituierten oder substituierten, vorzugsweise linearen, $C_{1-5}$-Alkansäure. Unter substituierten Alkansäuren dieser Art sind insbesondere zu nennen: einerseits mono- oder vorzugsweise poly-halogenierte, insbesondere chlorierte oder fluorierte, $C_{1-5}$-Alkansäuren, wie in erster Linie die Trifluoressigsäure, andererseits mono- und dibasische Aminosäuren einschliesslich Monoamide der letzteren, insbesondere α-Aminosäuren vom Typ derjenigen, die als Bausteine von Peptiden und insbesondere in L-Form in der Natur vorkommen; unter diesen ist beispielsweise die Glutaminsäure hervorzuheben, die vorzugsweise mit ihrem γ-Carboxyl die Aminogruppe acyliert. Dieser Darstellung nach bedeutet Symbol $R^4$ vorzugsweise Wasserstoff, Trifluoracetyl oder 7-Glutamyl der Formel HOCOCH($NH_2$)$CH_2CH_2$CO-, wobei beim letzteren sein freies Carboxyl in einer Salzform vorliegen kann.

Vorzugsweise bildet die oben charakterisierte Aminomethylengruppe zusammen mit den benachbarten Symbolen einen gegebenenfalls acylierten Cystein-Rest der Partialformel

$$\begin{array}{c} \text{-S-CH}_2 \\ \qquad\quad | \\ R^4\text{-NH-CH-CO-}R^3 \end{array}$$

bzw. in Kurzform $R^4$-$C_y^{|}$s-$R^3$, worin $R^3$ und $R^4$ die obgenannten allgemeinen und bevorzugten Bedeutungen haben, wobei der L-Cysteinyl-Rest mit der in der Natur vorkommenden Konfiguration am asymmetrischen C-Atom bevorzugt ist. In diesem Fall bedeutet $R^3$ vorzugsweise Hydroxyl, $C_{1-4}$-Alkoxy oder einen am Stickstoff gebundenen, gegebenenfalls durch ein $C_{1-4}$-Alkanol veresterten Glycinrest, und $R^4$ insbesondere Wasserstoff, Trifluoracetyl oder γ-Glutamyl (auch in Salzform).

Die meisten Verbindungen der Formel I können, ihrem individuellen Charakter nach, auch in Form von Salzen vorliegen. Diejenigen davon, welche eine genügende Acidität haben, wie insbesondere die mit freien Carboxylgruppen, können Salze mit Basen, wie insbesondere anorganischen Basen, vorzugsweise physiologisch verträgliche Alkalimetall-Salze, vor allem Natrium- und Kalium-Salze, bilden. Diejenigen der Verbindungen der Formel I, welche eine genügende Basizität haben, wie Ester und Amide von Aminosäuren, können als Säureadditionssalze, insbesondere als physiologisch verträgliche Salze, mit üblichen pharmazeutisch anwendbaren Säuren vorliegen; von anorganischen Säuren sind die Halogenwasserstoffsäuren, wie die Chlorwasserstoffsäure, sowie Schwefelsäure und Phosphor- bzw. Pyrophosphorsäure besonders zu nennen, von den organischen Säuren sind es in erster Linie die Sulfonsäuren, z.B. die aromatischen, wie Benzol- oder p-Toluol-sulfonsäure, Embonsäure und Sulfanilsäure, oder Niederalkansulfonsäuren, wie Methansulfon-, Ethansulfon-, Hydroxyethansulfon- sowie Ethylendisulfonsäure, oder aber auch aliphatische, alicyclische, aromatische oder heterocyclische Carbonsäuren, wie Ameisen-, Essig-, Propion-, Bernstein-, Glykol-, Milch-, Aepfel-, Wein-, Zitronen-, Fumar-, Malein-, Hydroxymalein-, Oxal-, Brenztrauben-, Phenylessig-- Benzoe-, p-Aminobenzoe-, Anthranil-, p-Hydroxybenzoe-, Salicyl- und p-Aminosalicylsäure, sowie Ascorbinsäure. Verbindungen der Formel I, die sowohl basische wie saure funktionelle Gruppen. wie freie Carboxyl- und Aminogruppen, enthalten, können auch als innere Salze vorliegen.

Ganz besonders hervorzuheben sind Verbindungen der Formel I, in welchen der ganze Rest (A) der eingangs erwähnten Meroaptoalkancarbonsäure durch eine der folgenden Formeln dargestellt ist, wobei die Aminosäurereste der "natürlichen" L-Reihe bevorzugt sind:

$$\begin{array}{l} \quad\quad\quad\quad\quad\quad\quad\quad\quad\quad\quad\quad\quad -S-CH_2 \\ HOCO-CH(NH_2)-CH_2-CH_2-CO-NH-CH-CO-NH-CH_2-COOH \end{array} \quad (A\text{-}1),$$

bzw. in Kurzform

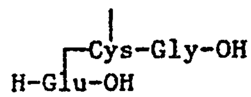

$$\begin{array}{l} \quad\quad\quad -S-CH_2 \\ CF_3-CO-NH-CH-CO-NH-CH_2-COOH \end{array}$$ bzw. in Kurzform $CF_3CO-Cys-Gly-OH$ (A-2),

$$\begin{array}{l} \quad\quad\quad -S-CH_2 \\ CF_3-CO-NH-CH-COOH \end{array}$$ bzw. in Kurzform $CF_3CO-Cys-OH$ (A-3),

$$\begin{array}{l} -S-CH_2 \\ NH_2-CH-CO-NH-CH_2-COOH \end{array}$$ bzw. in Kurzform $H-Cys-Gly-OH$ (A-4),

$$\begin{array}{l} -S-CH_2 \\ NH_2-CH-COOH \end{array}$$ bzw. in Kurzform $H-Cys-OH$ (A-5)

sowie $-S-CH_2-COOH$ (A-6).

Mitinbegriffen sind auch entsprechende Verbindungen, worin die Carboxylgruppen in Form eines primären Amids oder $C_{1-4}$-Alkylesters, oder insbesondere eines Salzes, vorzugsweise eines Alkalimetallsalzes, vorliegen.

In erster Linie sind die in den Beispielen beschriebenen Verbindungen der Formel I hervorzuheben.

Die erfindungsgemässen Thioether können in an sich bekannter Weise hergestellt werden, beispielsweise dadurch, dass man ein dem eingangs definierten Rest (B) entsprechendes ungesättigtes aliphatisches trans -Epoxid von mindestens 12 C-Atomen, welches mindestens in α-Stellung zur Epoxy-Gruppierung eine Doppelbindung trägt, insbesondere ein solches der Formel

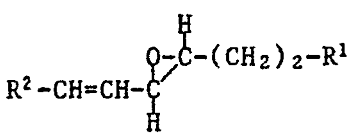

(II)

worin $R^1$ und $R^2$ die oben genannten Bedeutungen haben und beide Wasserstoffe am Oxiran-Ring gegeneinander trans orientiert sind, und in welchem eine gegebenenfalls vorhandene Hydroxylgruppe in einer geschützten Form vorliegen kann, mit einer dem oben definierten Rest (A) entsprechenden Mercaptoalkancarbonsäure, insbesondere einer der Formel

$$HS-CH_2-X-CO-R^3 \quad (III)$$

worin $R^3$ und -X- die obengenannten Bedeutungen haben, in welcher Säure eine gegebenenfalls vorhandene Aminogruppe in einer geschützten Form vorliegen kann, oder mit ihrem Salz oder ihrem Derivat mit umgewandelter Carboxylgruppe umsetzt und wenn notwendig oder erwünscht, die Schutzgruppen der Hydroxyl- und/oder Aminogruppe abspaltet, und/oder eine als Ester vorliegende Verbindung zur freien Säure oder einem Salz davon verseift und, wenn erwünscht, eine erhaltene freie Verbindung mit salzbildenden Eigenschaften in ein Salz davon oder ein erhaltenes Salz in eine freie Verbindung überführt.

Die Umwandlung erfolgt unter an sich bekannten Bedingungen bei Temperaturen von etwa -20 °C bis etwa +50 °C, vorzugsweise bei Raumtemperatur, und vornehmlich in einem basischen Milieu, beispielsweise in Gegenwart eines Amins, insbesondere eines tertiären aliphatischen, arylaliphatischen oder gesättigten heterocyclischen Amins, wie Trialkylamin (z.B. Triethylamin, oder Ethyl-diisopropylamin), Dialkyl-benzylamin

(z.B. N,N-Dimethylbenzylamin), N,N-Dialkylanilin (z.B. N,N-Dimethylanilin) bzw. N-Methyl- oder N-Ethylpiperidin oder N,N'-Dimethylpiperazin. Ueblicherwiese wird die Umsetzung in einem indifferenten organischen Lösungsmittel, wie einem Niederalkanol, z.B. Methanol oder Ethanol, durchgeführt.

Wenn im Ausgangsstoff, insbesondere im Substituent $R^1$ der Formel II ein freies Hydroxyl vorliegt, so kann es während der Umsetzung in einer geschützten, wie veretherten, Form vorliegen. Bevorzugt sind leicht abspaltbare, insbesondere acidolytisch abspaltbare Hydroxyl-Schutzgruppen, wie sie allgemein, insbesondere von der Peptid- und Steroid-Chemie, wohl bekannt sind; dabei werden Schutzgruppen vom Typ tert-Butyl- und insbesondere Tetrahydropyranyl-ether (THP-Ether) besonders bevorzugt. Diese Schutzgruppen können nach erfolgter Hauptreaktion (d.h. Kondensation des Epoxids mit der Mercaptocarbonsäure) in allgemein bekannter Weise, z.B. durch Behandeln mit einer organischen Säure, wie Ameisensäure, Essigsäure, Oxalsäure oder Trifluoressigsäure, oder einem Gemisch davon, und gegebenenfalls in Gegenwart von Wasser und/oder inerter organischer Lösungsmittel, wie Niederalkanole (z.B. Methanol oder Ethanol) und cyclischer Ether (wie Tetrahydrofuran oder Dioxan) unter Freisetzung des Hydroxyls entfernt werden.

Wenn die als Ausgangsstoff eingesetzten Mercaptocarbonsäuren eine freie Aninogruppe enthalten, so kann diese während der Hauptreaktion vorzugsweise in einer geschützten, wie isnbesondere einer acylierten, Form vorliegen. Vorzugsweise werden leicht, insbesondere acidolytisch, abspaltbare Amino-Schutzgruppen angewendet, wie sie allgemein, vor allem in der Peptid-Chemie, einschliesslich Bedingungen für ihre Entfernung wohl bekannt sind. Unter den Amino-Schutzgruppen ist jedoch die Trifluoracetylgruppe besonders hervorzuheben: diese kann nach erfolgter Hauptreaktion im erfindungsgemässen Endstoff weiter bestehen bleiben oder, wenn erwünscht, nachträglich abgespalten werden. Die Abspaltung der N-Trifluoracetylgruppeerfolgt, wie bekannt, vorzugsweise durch Hydrolyse, insbesondere unter basischen Bedingungen, wie mit Alkalimetallcarbonaten (z.B Natrium- oder Kaliumcarbonat) oder verdünnten Alkalilaugen (z.B. Natrium- oder Kalium-hydroxid) in Anwesenheit von Wasser in einem mit Wasser mischbaren organischen Lösungsmittel, wie einem Niederalkanol (z.B. Methanol oder Ethanol) oder cyclischen Ether (z.B. Tetrahydrofuran oder Dioxan) bei Temperaturen von etwa 0-80°C, vorzugsweise bei einer leicht erhöhten Temperatur von etwa 50-60°C. Wenn Estergruppen im zu hydrolysierenden Produkt vorhanden sind, wie eine acylierte Hydroxylgruppe im Hydroxyalkylrest $R^1$ oder eine veresterte Carboxylgruppe im Mercaptosäure-Rest (A), dann werden sie unter diesen Bedingungen gleichzeitig mitverseift.

In der Hauptreaktion (Kondensation mit Epoxid) wird die Mercaptocarbonsäure vornehmlich in Form ihres Esters, vorzugsweise eines $C_{1-4}$-Alkylesters (wie Methyl- oder Ethyl-esters) eingesetzt; falls der erfindungsgemässe Endstoff als freie Säure oder ihr Salz erwünscht ist, dann muss der erhaltene Ester hydrolysiert werden. Die Hydrolyse wird unter den üblichen Bedingungen durchgeführt, z.B. solchen, die vorangehend für die basenkatalysierte hydrolytische Abspaltung der N-Trifluoracetylgruppe geschildert wurden. Man kann jedoch unter milderen Bedingungen, wie insbesondere bei niedriger Temperatur (vorzugsweise bei Raumtemperatur), mit einer äquivalenten stöchiometrischen Menge Alkali und durch verkürzte Reaktionszeit, gegebenenfalls unter analytischer Kontrolle, z.B. durch Dünnschichtchromatographie, die Estergruppe selektiv unter Erhaltung der N-Trifluoracetylgruppe abspalten, eine acylierte Hydroxylgruppe wird dabei jedoch meistenfalls gleichzeitig abgespalten.

Ausgangsstoffe für das erfindungsgemässe Kondensationsverfahren sind entweder an sich bekannt oder nach bekannten Analogieverfahren in an sich bekannter Weise erhältlich. - So sind z.B. die wichtigen Mercaptocarbonsäuren der Formel III beschrieben (vgl. z.B. E.J. Corey et al.: Tetrahedron Letters 1980, 3143), andere analoge Säuren sind auf dieselbe Weise ausgehend von entsprechenden bekannten Ausgangsstoffen zugänglich. Zur Herstellung von Cysteinderivaten werden vorteilhaft analoge bekannte Cystin-Verbindungen eingesetzt und der üblichen reduktiven Spaltung der disulfidischen Bindung unterworfen oder aber als Cystein-Derivate mit geeignet, z.B. durch Trityl oder Acetylaminomethyl, geschützter Mercaptogruppe verarbeitet.

Das als Ausgangsstoff verwendete ungesättigte trans-Epoxid, z.B. jenes der oben definierten Formel II, kann vornehmlich mittels denselben Verfahren hergestellt werden, welche auch bei der Synthese von Leukotrienen verwendet werden. Bei einem typischen allgemeinen Syntheseweg wird z.B. von einem gesättigten aliphatischen Aldehyd (Alkanal) der Formel

$$O{=}CH{-}(CH_2)_2{-}R^1 \qquad (IV)$$

ausgegangen, worin $R^1$ die oben angegebenen Bedeutungen hat, wobei eine gegebenenfalls im Rest $R^1$ vorhandene freie Hydroxylgruppe in einer als Ether geschützten, z.B. einer der oben geschilderten, Form

vorliegt. Diese Verbindung wird mit Formylmethylentriphenylphosphoran (oder einem äquivalenten Reagens) kondensiert, womit der entsprechende $\alpha,\beta$-ungesättigte Aldehyd, 2-trans -alkenal, der Formel

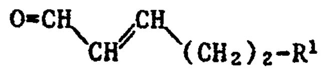

(V)

entsteht, worin $R^1$ die oben angegebenen Bedeutungen hat und eine im Rest $R^1$ gegebenenfalls vorhandene freie Hydroxylgruppe als Ether geschützt ist. Diese Verbindung wird anschliessend in an sich bekannter Weise, vorzugsweie unter schwach alkalischen Bedingungen (z.B. in Gegenwart von Alkalicarbonaten) mit wässrigem Wasserstoffperoxid epoxidiert, wodurch ein trans-Epoxid, 2-(RS),3(SR)-Epoxy-Alkanal der Formel

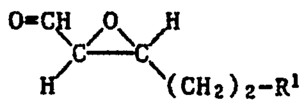

(VI)

resultiert, worin $R^1$ die oben angegebenen Bedeutungen hat und eine im Rest $R^1$ gegebenenfalls vorhandene freie Hydroxylgruppe als Ether geschützt ist. Dieser Epoxyaldehyd kann zum gewünschten ungesättigten Epoxid, z.B. dem der oben definierten Formel II, worin eine im Rest $R^1$ gegebenenfalls vorhandene freie Hydroxylgruppe in geschützter, veretherter Form vorliegt, durch Kondensation mit einem entsprechenden bekannten Alkylidentriphenylphosphoran kondensiert werden. Für mehrfach ungesättigte Epoxide, z.B. diejenigen der Formel II, worin $R^2$ eine oder mehrere Doppelbindungen aufweist, bietet sich eine indirekte Alternative an: anstelle der Wittig-Reaktion mit einem in seiner Kette ungesättigten Yliden-Phoshoran wird der Epoxyaldehyd VI zuerst mit Formylmethylentriphenylphoshoran oder $\gamma$-Triphenylphosphoranylidencrotonaldehyd (4-Triphenylphosphoranyliden-2-trans -butenal) um 2 bzw. 4 Kohlenstoffatome (1 bzw. 2 Doppelbindungen) verlängert, und erst das erhaltene 4(RS),5(RS)-Epoxy-2-alkenal bzw. 6(RS),7(RS)-Epoxy-2,4-alkadienal mit einem einfachen, gesättigten Alkylidentriphenylphosphoran oder einem weniger komplizierten Alkenylidentriphenylphosphoran zum gewünschten mehrfach ungesättigten Epoxid (z.B. einem der Formel II) kondensiert. -

Wenn individuelle Diastereomere erwünscht sind, so kann vorteilhaft an beliebiger Stufe ein individuelles Diastereomeres eines Ausgangsstoffes eingesetzt werden oder aus einem razemischen oder optisch inaktiven Ausgangsstoff durch stereoselektive Reaktionsbedingungen oder optisch aktive Reagentien ein Diastereomeres bevorzugt gebildet werden, oder razemische Diastereomerengemische durch physikalische Trennmethoden, gegebenenfalls unter Anwendung optisch aktiver Hilfsstoffe, in optisch individuelle Diastereomere aufgetrennt werden.

In stereochemischer Hinsicht wird jedoch sowohl die erfindungsgemässe Kondensation der Bildungskomponenten II und III, wie auch die Zubereitung der Ausgangsstoffe vornehmlich so durchgeführt, dass man jeweils stereochemisch einheitliche Ausgangsstoffe einsetzt, die Reaktionen nach Möglichkeit stereoselektiv, z.B. durch Einsatz von optisch aktiven Reagentien und/oder Hilfsstoffen, durchführt und stereochemisch einheitliche Produkte aus Reaktionsgemischen unmittelbar nach der Reaktion isoliert. So werden z.B. bei Herstellung der ungesättigten Ausgangsstoffe gegebenenfalls gebildete Isomere mit cis - und trans - Doppelbindungen sofort voneinander getrennt, wozu die üblichen physikalischen Trennungsmethoden, wie insbesondere Chromatographie, geeignet sind. In der Hauptreaktion wird vornehmlich das Epoxid der Formel II als ein individuelles trans -Stereoisomer, jedoch in razemischer Form (wie es normalerweise durch die Epoxydierung eines Olefins gebildet wird) eingesetzt; die Mercaptoalkansäure der Formel III, sofern sie optisch aktiv ist, wird vorzugsweise als ein individuelles optisches Antipode verwendet (was insbesondere bei Cystein und dessen Derivaten der übliche Fall ist) - diese Massnahme ermöglicht, die beiden gebildeten optisch aktiven Diastereomere einfach durch geläufige physikalische Methoden, wie Chromatographie, voneinander zu trennen; falls eine optisch inaktive Mercaptoalkansäure verwendet wird, ist es zur Gewinnung individueller optisch aktiver Produkte unumgänglich, die Methoden der Spaltung in Antipode mittels optisch aktiver Hilfsstoffe, wie z.B. die Bildung von Salzen mit optisch aktiven Basen, anzuwenden. Alle geeigneten Trennungsverfahren sind an sich bekannt und können auch wiederholt oder untereinander zweckmässig kombiniert werden.

Infolge der engen Beziehung zwischen den neuen Verbindungen in freier Form und in Form ihrer Salze sind im vorausgegangenen und nachfolgend unter den freien Verbindungen oder ihren Salzen sinngemäss

auch die entsprechenden Salze bzw. freien Verbindungen zu verstehen.

Die Erfindung betrifft auch diejenigen Ausführungsformen des Verfahrens, nach denen man von einer auf irgendeiner Stufe des Verfahrens als Zwischenprodukt erhältlichen Verbindung ausgeht und die fehlenden Schritte durchführt oder einen Ausgangsstoff in Form eines Salzes verwendet oder unter den Reaktionsbedingugnen bildet.

Die bei den erfindungsgemässen Verfahren und ihren Vorstufen auftretenden neuen Ausgangsstoffe und Zwischenprodukte bilden ebenfalls Gegenstand der Erfindung.

Vorzugsweise werden solche Ausgangsstoffe verwendet, und die Reaktionsbedingungen so gewählt, dass man zu den vorstehend als besonders bevorzugt aufgeführten Verbindungen gelangt.

Die vorliegende Erfindung betrifft ebenfalls pharmazeutische Zusammensetzungen und Arzneimittel, welche eine der erfindungsgemässen Verbindungen der Formel I oder ein pharmazeutisch verwendbares Salz davon enthalten. Bei den erfindungsgemässen pharmazeutischen Zusammensetzungen handelt es sich insbesondere um solche, die zur lokalen Verabreichung und vor allem zur Inhalationsverabreichung, z.B. in Form eines Aerosols, mikropulverisierten Pulvers oder einer feinversprühten Lösung, an Säuger bestimmt sind und den Wirkstoff allein oder zusammen mit einem pharmazeutisch anwendbaren Trägermaterial enthalten.

Pharmazeutische Präparate für topische und lokale Verwendung sind z.B. für die Behandlung der Haut Lotionen und Cremen, die eine flüssige oder semifeste Oel-in-Wasser- oder Wasser-in-Oel-Emulsion enthalten, und Salben (wobei solche vorzugsweise ein Konservierungsmittel enthalten). Für die Behandlung der Augen eignen sich Augentropfen, welche die aktive Verbindung in wässriger oder öliger Lösung enthalten, und Augensalben, die vorzugsweise in steriler Form hergestellt werden. Für die Behandlung der Nase sind Aerosole und Sprays (ähnlich den weiter unten beschriebenen für die Behandlung der Atemwege), grobe Puder, die durch schnelles Inhalieren durch die Nasenlöcher verabreicht werden, und vor allem Nasentropfen, welche die aktive Verbindung in wässriger oder öliger Lösung enthalten, geeignet; für die lokale Behandlung der Mundhöhle eignen sich auch Lutschbonbons, welche die aktive Verbindung in einer im allgemeinen aus Zucker und Gummiarabikum oder Tragaganth gebildeten Masse enthalten, welcher Geschmacksstoffe beigegeben sein können, sowie Pastillen, die den Aktivstoff in einer inerten Masse, z.B. aus Gelatine und Glycerin oder Zucker und Gummiarabikum, enthalten.

Als pharmazeutische Zusammensetzungen für die Verabreichung in Form von Aerosolen oder Sprays sind geeignete z.B. Lösungen, Suspensionen oder Emulsionen des erfindungsgemässen Wirkstoffs der Formel I mit einem geeigneten pharmazeutisch annehmbaren Lösungsmittel, wie insbesondere Ethanol und Wasser, oder einem Gemisch solcher Lösungsmittel. Sie können nach Bedarf auch andere pharmazeutische Hilfsstoffe, wie nicht-ionische oder anionische oberflächenaktive Mittel, Emulgatoren und Stabilisatoren, sowie Wirkstoffe anderer Art enthalten und vor allem zweckmässig mit einem Treibgas, wie einem inerten Gas unter erhöhtem Druck oder insbesondere mit einer leicht flüchtigen, vorzugsweise unter normalem atmosphärischem Druck unterhalb der üblichen Raumtemperatur (z.B. zwischen etwa -30 und +10 °C) siedenden Flüssigkeit, wie einem mindestens teilweise fluorierten polyhalogenierten Niederalkan, oder einem Gemisch solcher Flüssigkeiten, vermischt ist. Derartige pharmazeutische Zusammensetzungen, welche vorwiegend als Zwischenprodukte oder Vorratsgemische für die Herstellung der entsprechendenden Arzneimittel in fertiger Form verwendet werden, enthalten den Wirkstoff üblicherweise in einer Konzentration von etwa 0,1 bis etwa 10, insbesondere von etwa 0,3 bis etwa 3 Gewichts-%. - Zur Herstellung von Arzneimitteln in fertiger Form wird eine solche pharmazeutische Zusammensetzung in geeignete Behälter, wie Flacons und Druckflaschen, abgefüllt, welche mit einer für solche Zwecke geeigneten Versprühungseinrichtung bzw. Ventil versehen sind. Das Ventil ist vorzugsweise als ein Dosierungsventil konstruiert, welches bei der Betätigung eine vorbestimmte Menge der Flüssigkeit, entsprechend einer vorbestimmten Dosis des Wirkstoffs, freigibt. Bei Herstellung der fertigen Arzneimittelform kann man auch so vorgehen, dass entsprechende Mengen der als Vorratslösung vorliegenden pharmazeutischen Zusammensetzung und des Treibmittels separat in die Behälter abgefüllt werden und erst dort zur Vermischung kommen. Die Dosierung des zu verabreichenden Wirkstoffs der Formel I und die Häufigkeit der Verabreichung hängen ab von der jeweiligen Wirksamkeit bzw. der Länge der Wirkung jeder individueller dieser Verbindungen, von der Stärke der zu behandelnden Krankheit bzw. ihrer Symptome, sowie vom Geschlecht, Alter, Gewicht und individueller Ansprechbarkeit des zu behandelnden Säugers. Im Durchschnitt dürfte die empfohlene tägliche Dosis einer erfindungsgemässen Verbindung der Formel I bei einem 75 kg schweren Säuger (in erster Linie Menschen) im Bereich von etwa 10 bis etwa 500, vorzugsweise zwischen etwa 25 bis etwa 250 mg liegen, wobei die Verabreichung zweckmässig nach Bedarf in mehreren Dosen pro Tag erfolgen kann.

Die Erfindung betrifft auch die Anwendung der erfindungsgemässen Wirkstoffe der Formel I zur Linderung oder Behebung krankhafter Zustände und/oder Symptome des Körpers eines Säugers, insbesondere des Menschen, welche auf die allergogene Einwirkung von Leukotrienen zurückzuführen sind und

insbesondere bei Asthma vorkommen. Diese Anwendung bzw. die entsprechende Heilmethode, ist durch die Behandlung des leidenden Körpers bzw. Körperteils mit einer antiallergisch wirksamen Menge einer Verbindung der Formel I allein oder in Form eines Arzneimittels, insbesondere einer zur Inhalation bestimmten pharmazeutischen Zusammensetzung, charakterisiert. Unter der Bezeichnung "eine antiallergisch wirksame Menge" ist eine solche Menge des Wirkstoffes zu verstehen, die zu einer signifikanten Inhibition der durch Leukotriene verursachten Kontraktionen ausreicht.

Die folgenden Beispiele illustrieren näher die vorliegende Erfindung, ohne sie in ihrem Umfang einzuschränken. Alle Temperaturen sind in Celsiusgraden angegeben. Die Aminosäuren als Bildungskomponenten der beschriebenen Verbindungen sind in der "natürlichen" L-Form.

Beispiel 1:

N-{S-[5(S,R),6(R,S)-5-Hydroxy-7-trans,9-trans,11-cis,14-cis-eicosatetraen-6-yl]-N-trifluoracetylcysteinyl}-glycin-methylester und die individuelen 5(S),6(R)- und 5(R),6(S)-Diastereomeren

Eine Lösung von 103 mg (0.36 mMol) 5(S,R),6(S,R)-5,6-Epoxy-7-trans, 9-trans,11-cis,14-cis-eicosatetraen, 154 mg (0.54 mMol) N-(N'-Trifluoracetylcysteinyl)-glycin-methylester und 145 mg (1.44 mMol) Triethylamin in 0.5 ml Methanol wird in einer Argon-Atmosphäre 4 Stunden lang bei Raumtemperatur gerührt und mit 2 ml Methanol verdünnt. Umkehrphasen-Chromatographie im System Acetonitril/Wasser (2:1) ergibt 212 mg (100 %) eines Diatereomerengemisches, welches durch nachträgliche Hochdruck-Chromatographie an einer Zorbax ODS RP-Säule mit Methanol/Wasser (85:15) in etwa gleiche Anteile beider Diastereomeren aufgetrennt wird. Das 5(S),6(R)-Diastereomere hat unter diesen Bedingungen eine kürzere Retentionszeit als das 5(R),6(S)-Diastereomere.

UV (in Methanol): 5(S),6(R)-Isomer: $\lambda_{max}$ = 271, 281, 290nm
($\epsilon_{281}$ = 40 600)
5(R),6(S)-Isomer: $\lambda_{max}$ = 271, 280, 290nm

IR ($CH_2Cl_2$) ist praktisch identisch für beide Diasteromere:
3570 (breit), 3370 (breit), 3000, 2950, 2920, 2850, 1742, 1720, 1680, 1518, 1460, 1435, 1380-1350, 1208, 1165, 1000 $cm^{-1}$.

$[\alpha]_D^{20}$ [5(S),6(R)-Isomer]: $+59^\circ \pm 1^\circ$ (1.4 % in $CHCl_3$).

Das als Ausgangsstoff eingesetzte razemische 5(S,R),6(S,R)-5,6-Epoxy-7-trans,9-trans,11-cis,14-cis-eicosatetraen kann folgendermassen hergestellt werden:

Eine Lösung von 51.4 ml Pentanal und 147 g Triphenylphosphoranylidenacetaldehyd in 480 ml Chlorofrm wird 5 Tage unter Rückfluss erhitzt, das Lösungsmittel unter vermindertem Druck abdestilliert und der erhaltene Rückstand mit 400 ml eines 3:1-Gemisches von Hexan und Ether verrührt. Das kristallin abgeschiedene Triphenylphosphinoxid wird abgenutscht, das Filtrat im Vakuum eingedampft und der Rückstand im Vakuum destilliert. Man erhält 24.5 g (45.2 %) 2-trans-Heptenal als farbloses Oel, Sdp. 60-65° C/24 mbar.

Zu einer Lösung von 2.24 g 2-trans-Heptenal in 24 ml Methylenchlorid und 44 ml Methanol wird bei 0° C unter Rühren 2.04 ml 30%-ige wässrige $H_2O_2$-Lösung zugegeben, gefolgt von 100 mg festem Kaliumcarbonat. Das so entstandene Reaktionsgemisch wird 3 Stunden lang bei 0° C weitergerührt. Schliesslich wird es mit 100 ml Methylenchlorid versetzt und zweimal mit je 25 ml Phosphatpuffer von pH 8.0 gewaschen. Die wässrigen Anteile werden mit 50 ml Methylenchlorid nachextrahiert. Die vereinigten organischen Anteile werden über Magnesiumsulfat getrocknet und unter vermindertem Druck eingedampft. Chromatographie des so erhaltenen Rohproduktes auf 85 g Merck Kieselgel 60 mit Methylenchlorid als Eluens ergibt insgesamt 1.59 g (62 %) 2(R,S),3(S,R)-2,3-Epoxyheptanal als farbloses Oel [$R_f$ (Kieselgel; Toluol-Ethylacetat 4:1):0.59; IR ($CH_2Cl_2$): 2950, 2925, 2850, 2820, 1721, 1463, 1430, 1378, 1210, 850 $cm^{-1}$].

Zu einer Lösung von 640 mg 2(R,S),3(S,R)-2,3-Epoxyheptanal in 10 ml Methylenchlorid wird bei Raumtemperatur innerhalb 1 Stunde eine Lösung von 1.65 g $\gamma$-Triphenylphosphoranylidencrotonaldehyd in 15 ml Methylenchlorid zugetropft und das resultierende Reaktionsgemisch eine weitere Stunde bei Raumtemperatur gerührt. Zur Aufarbeitung wird das Reaktionsgemisch unter vermindertem Druck eingedampft und der Rückstand auf einer Säule von 40 g Kieselgel in Toluol/Ethylacetat (19:1) chromatographiert. Man erhält ein cis,trans- und trans,trans-Isomerengemisch, welches zur Isomerisierung in 5 ml Methylenchlorid mit wenigen Kristallen Iod gelöst und bei Raumtemperatur 4 Stunden stehen gelassen wird. Die Lösung wird dann im Vakuum eingedampft und, wie oben erwähnt, chromatographiert. Man erhält 511 mg (57 %) 6-

(S,R),7(S,R)-6,7-Epoxy-2-trans,4-transundecadienal als gelbliches Oel. [UV(Ethanol): $\lambda_{max}$ = 276 nm; $\epsilon$ = 29900; IR($CH_2Cl_2$): 2950, 2920, 2850, 2800, 2720, 1678, 1640, 1600,, 1460, 1163, 1120, 1007, 985 $cm^{-1}$; $R_f$ [Kieselgel; Toluol-Ethylacetat (4:1)] = 0.56.

Eine Lösung von 3-cis-Nonen-l-yl-triphenyl-phosphonium-toluolsulfonat [I.Ernest, A.J.Main, R. Menassé, Tetrahedron Letters 23 , 167 (1982)] (378 mg) in 4.2 ml Tetrahydrofuran und 1.26 ml Hexamethylphosphorsäuretriamid wird bei -78°C unter Argon mit 0.31 ml einer 20%-Lösung von Butyllithium in Hexan tropfenweise versetzt und die resultierende Lösung bei -78°C weitere 30 Min. gerührt. Zu der erhaltenen Lösung von Triphenylphosphoranyliden-3-cis-nonen wird bei -78°C eine Lösung von 110 mg 6(S,R),7(S,R)-6,7-Epoxy-2-trans,4-trans-undecadienal in 1.0 ml Tetrahydronfuran zugetropft und das entstandene Reaktionsgemisch 30 Min. bei -78°C weitergerührt. Zur Aufarbeitung wird das Reaktonsgemisch zwischen 100 ml Ether und 30 ml Phosphatpuffer von pH 8.0 verteilt, und beide Phasen werden noch mit Ether, bzw. Pufferlösung, nachextrahiert. Die vereinigten etherischen Anteile werden über Magnesiumsulfat getrocknet und im Vakuum eingedampft, wodurch man 421 mg öliges Rohprodukt bekommt. Dieses wird mit 2-3 ml Hexan-Ether-Gemisch 3:1 verrührt, das kristallin ausgeschiedene Triphenylphsphinoxid abgetrennt und das Filtrat eingedampft. Der erhaltene Rückstand (195 mg) wird an einer Aluminiumoxid-Säule (10 g), präpariert in Hexan mit 0.5 % Triethylamin, mit demselben Eluens chromatographiert. Man erhält 90.6 mg (57 %) 5-(S,R),6(S,R)-5,6-Epoxy-7-trans,9-trans,11-cis,14-cis-eicosatetraen als dickflüssiges, gelbliches Oel. [UV (Methanol):
$\lambda_{max}$ = 270, 280, 291 nm; $\epsilon_{280}$ = 59 600; IR ($CH_2Cl_2$): 3000, 2900, 2850, 1455, 1370, 992 963 $cm^{-1}$].

Beispiel 1A:

N-(S-[5(S),6(R)-5-Hydroxy-7-trans,9-trans,11-cis,14,cis-eicosatetraen-6-yl]-N-trifluoracetylcysteinyl)-glycin-methylester [aus optisch individuellem Epoxyolefin].

Eine Lösung von 130 mg (0.450 mMol) 5(S),6(S)-5,6-Epoxy-7-trans,9-trans,11-cis,14-cis-eicosatetraen, 260 mg (0.902 mMol) N-(N-Trifluoracetylcysteinyl)-glycin-methylester und 250 ml (1.8 mMol) Triethylamin in 1.0 ml Methanol wird unter Argon bei Raumtemperatur 3.5 Stunden gerührt und nachher, nach Verdünnen mit etwas Methanol, auf 12 RP-Platten (Opti-UPC$_{12}$, ANTECH AG Bennwil, Schweiz; 20 x 20 cm) im System Acetonitril: Wasser (1:1) chromatographiert. Die auf diese Weise erhaltene Titelverbindung (216 mg) wird an einer Hochdruck-RP-Säule (Zorbax ODS) in Methanol: Wasser = 87:13 rechromatographiert. Man erhält 122 mg reine Titelverbindung, die mit dem Diastereomeren mit der kürzeren Retentionszeit (s.Beispiel 1) in jeder Hinsicht identisch ist.

Der optisch aktive Ausgangsstoff, d.h. 5(S),6(S)-5,6-Epoxy-7-trans,9-trans,11-cis,14-cis-eicosatetraen, wird auf die folgende Weise hergestellt:

Zu einer Lösung von 10 g Lithiumaluminiumhydrid in 400 ml Ether werden bei 0°C 16.9 g 2-Heptinol in 200 ml Ether innerhalb von 30 Minuten unter Rühren zugetropft, und das entstandene Reaktionsgemisch wird über Nacht unter Rückfluss gekocht. Der Ueberschuss von $LiAlH_4$ wird unter Kühlen in einem Eis-Wasser-Bad durch Zugabe von 40 ml Ethylacetat zerstört und das resultierende Reaktionsgemisch zwischen Ether und kalter IN Schwefelsäure aufgenommen. Die angesäuerte (pH 2) wässrige Schicht wird noch mit Ether nachextrahiert, die vereinigten organischen Extrakte werden über Magnesiumsulfat getrocknet und im Vakuum eingedampft. Destillation des Rückstandes (18 g) unter vermindertem Druck ergibt 13.2 g 2-trans-Heptenol als farbloses Oel, Sdp. 71.5-72°C/13 mbar.

Eine Lösung von 2.28 g (20 mMol) 2-trans-Heptenol in 10 ml Methylenchlorid wird bei -20°C zu einer Lösung von 5.94 ml Tetraisopropylorthotitanat und 4.12 g L-(+)-Weinsäure-diethylester in 210 ml Methylenchlorid zugegeben, gefolgt von 9.75 ml einer 4.1 H-Lösung von tert-Butylhydroperoxid in 1,2-Dichlorethan. Das resultierende Reaktionsgemisch wird bei -20°C über Nacht stehen gelassen. Nach Zugabe von 8 ml Dimethylsulfid wird bei -20° bis -23°C 45 Minuten gerührt, danach mit 50 ml einer 10%-igen wässrigen Lösung von L-(+)-Weinsäure versetzt und 30 Min. bei -20°C und 60 Min. ohne Kühlung weitergerührt. Die organische Phase wird abgetrennt, mit 100 ml Wasser nachgewaschen und nach Trocknen über Magnesiumsulfat unter vermindertem Druck eingedampft. Der Rückstand, gelöst in 150 ml Ether, wird bei 0°C mit 60 ml IN-NaOH 30 Minuten gerührt, die wässrige Phase abgetrennt, mit Ether nachextrahiert, und die vereinigten organischen Extrakte werden mit Kochsalzlösung geschüttelt. Nach Trocknen des organischen Anteils über Magnesiumsulfat und Abdestillieren des Lösungsmittels im Vakuum erhält man 2.3 g 2(S),3(S)-2,3-Epoxyheptanol als farbloses, instabiles Oel. Es wird gleich weiterverarbeitet.

Eine Lösung von 1.2 g 2(S),3(S)2,3-Epoxyheptanol in 28 ml Methylenchlorid wird bei Raumtemperatur

zu einer frisch hergestellten Lösung von 5.5 g Chromtrioxid und 8.76 g Pyridin in 70 ml Methylenchlorid zugegeben und das erhalene Reakticnsgemisch 30 Minuten weitergerührt. Das dunkelfarbige Reaktionsgemisch wird vom ausgeschiedenen Material abdekantiert, das letztere mit 160 ml Methylenchlorid nachgewaschen, und die vereinigten organischen Anteile werden mit 80 ml Phosphatpuffer von pH 8.0 gewaschen. Nach Trocknen über Magnesiumsulfat und Eindampfen unter vermindertem Druck wird das zurückbleibende Rohprodukt auf 90 g Merck Kieselgel 60 mit Toluol/Ethylacetat (4:1) chromatographiert. Man erhält 464 mg 2(R),3(S)-2,3-Epoxyheptanal als farbloses Oel. $[\alpha]_D^{20} = +101^\circ \pm 1^\circ$ (1.225 % in $CHl_3$); IR($CH_2Cl_2$): 2950, 2925, 2860, 2815, 2730, 1722, 1462, 1432, 1380, 1360, 1230, 1156, 850 $cm^{-1}$.]

Eine Lösung von 804 mg $\gamma$-Triphenylphosphoranylidencrotonaldehyd in 10 ml Methylenchlorid wird bei Raumtemperatur innerhalb 1 Stunde tropfenweise mit einer Lösung von 260 mg 2(R),3(S)-2,3-Epoxyheptanal in 10 ml Methylenchlorid versetzt und das resultierende Reaktionsgemisch anschliessend 1.5 Stunden weitergerührt. Die Lösung wird dann unter vermindertem Druck auf ca. 2 ml konzentriert und unmittelbar auf einer Säule von 20 g Kieselgel 60 mit Toluol/Ethylacetat (19:1) chromatographiert. Man erhält 120 mg eines cis,trans- und trans,trans-lsomerengemisches, welches, in 2 ml Methylenchlorid mit 2 mg Iod gelöst, zur Isomerisierung bei Raumtemperatur stehen gelassen wird. Nach 2.5 Stunden wird die Lösung im Vakuum eingeengt und nochmals, wie oben beschrieben, chromatographiert, wodurch man 103 mg reines, optisch aktives 6(S),7(S)-6,7-Epoxy-2-trans,4-trans-undecadienal als gelbes, bei -20° C kristallin erstarrendes Oel erhält. $[\alpha]_D^{20} = -28^\circ \pm 1^\circ$ (0.735 % in $CHCl_3$).

Die Kondensation von 90 mg der letztgenannten Verbindung, gelöst in 2 ml abs. Tetrahydrofuran bei -78° C mit einer Lösung von Triphenylphosporanyliden-3-cis-nonen, hergestellt aus 338 mg des entsprechenden Phosphoniumtoluolsulfonat in Tetrahydrofuran/Hexamethylphosphorsäuretriamid durch Zugabe von Butyllithium in Hexan, und Aufarbeitung auf die für das racemische Produkt beschriebene Weise (s. Beispiel 1) werden 132 mg (91 %) 5(S),6(S)-5,6-Epoxy-7-trans,9-trans,11-cis,14-cis-eicosatetraen als gelbliches Oel erhalten. $[\alpha]_D^{20} = -23^\circ \pm 1^\circ$ (0.81 % in $CHCl_3$).

Beispiel 1B:

N-{S-[5(5),6(R)-5-Hydroxy-7-trans,9-trans,11-cis,14-cis-eicosatetraen-6-yl]-cysteinyl}-glycin-Kaliumsalz

Eine Lösung von 84 mg N-{S[5(S),6(R)-5-Hydroxy-7-trans,9-trans,11-cis,14-cis-eicoratetraen-6-yl]-N-trifluoracetylcysteinyl}-glycinmethylester in 3.8 ml Tetrahydrofuran und 3.8 ml Methanol wird mit einer Lösung von 276 mg Kaliumcarbonat in 11,5 ml Wasser versetzt und das resultierende Gemisch bei Raumtemperatur unter Argon 44 Stunden gerührt. Schliesslich wird es unter vermindertem Druck auf ca. 3 ml eingeengt und auf Umkehrphasen-Platten aufgetragen. Chromatographie im System Acetonitril/Wasser (1:1) ergibt 40 mg Titelverbindung, die in 5.0 ml Ethanol bei -78° C aufbewahrt wird; UV (Ethanol): $\lambda_{max} = 271, 280, 290$ nm; $\epsilon_{280} = 48\ 600$.

Beispiel 1C:

N-{S-[5(R),6(S)-5-Hydroxy-7-trans,9-trans,11-cis,14-cis-eicosatetraen-6-yl]-cysteinyl}-glycin-Kaliumsalz

Analog dem im vorangehenden Beispiel 1B beschriebenen Verfahren wird aus 32 mg N-{S-[5(R),6(S)-5-Hydroxy-7-trans,9-trans,11-cis,14-cis-eicosatetraen-6-yl]-N-trifluoracetylcysteinyl)-glycin-methylester 8.5 mg Titelverbindung erhalten, welche in ethanolischer Lösung bei -78° C aufbewahrt wird: UV (Ethanol): $\lambda_{max} = 270, 280, 290$ nm;
$\epsilon_{280} = 49\ 000$.

Allgemeines Verfahren

Beispiel 2:

N-[S-5(RS),6(SR)-5-Hydroxy-7-cis-heptadecen-6-yl-N-trifluoracetylcysteinyl]-glycin-methylester

Zu einer Lösung von 480 mg 5(RS),6(RS)-5,6-Epoxy-7-cis-heptadecen (El) und 660 mg N-[N-Trifluoracetylcysteinyl]-glycin-methylester [E.J.Corey et al., Tetrahedron Lett. 1980, 3143] in 6 ml Methanol gibt man 0.78 ml Triethylamin. Die Lösung wird 16 Stunden bei Raumtemperatur unter Argon gerührt, das Lösungsmittel im Vakuum abgedampft und der Rückstand durch Chromatographie an Kieselgel mit Dichlormethan/Ethylacetat (85:15) gereinigt. Man erhält die Titelverbindung als farbloses Oel.

IR ($CH_2Cl_2$): 3400, 2940, 2870, 1755, 1740, 1690, 1530 $cm^{-1}$. Auf analoge Weise werden erhalten:

Beispiel 3: N-[S-5(RS),6(SR)-5-Hydroxy-7-cis-undecen-6-yl-N-trifluoracetylcysteinyl]-glycin-methylester, aus 67 mg 5(RS),6(RS)-5,6-Epoxy-7-cis-undecen (E2) und 486 mg N-[N-Trifluoracetylcysteinyl)-glycin-methylester.
IR ($CH_2Cl_2$): 3400, 2960, 2940, 2880, 1750, 1730, 1640, 1525 $cm^{-1}$.

Beispiel 4: N-[S-5(RS),6(SR)-5-Hydroxy-7-cis-tridecen-6-yl-N-trifluoracetyl-cysteinyl]-glycin-methylester, aus 0.5 g 5(RS)-6(RS)-5,6-Epoxy-7-cis-tridecen (E3) und 0.8 g N-(N-Trifluoracetylcysteinyl)-glycin-methylester.
IR ($CH_2Cl_2$):3400, 2980, 2940, 2870, 1760, 1740, 1700, 1535 $cm^{-1}$.

Beispiel 5: S-5(RS),6(SR)-5-Hydroxy-7-cis-tridecen-6-yl-cystein-methylester
aus 0,5 g 5(RS),6(RS)-5,6-Epoxy-7-cis-tridecen (E3) und 0.87 g Cystein-methylester-hydrochlorid.
IR ($CH_2Cl_2$): 3600, 3400, 2970, 2950, 2880, 1750, 1445 $cm^{-1}$.

Beispiel 6: S-5(RS),6(SR)-5-Hydroxy-7-cis-tridecen-6-yl-mercaptoessigsäure-methylester,
aus 0.72 g 5(RS),6(RS)5,6-Epoxy-7-cis-tridecen (E3)- und 0.43 g Mercaptoessigsäuremethylester.
IR (CH,$Cl_2$): 3600, 2980, 2940, 2880, 1745, 1475, 1445 $cm^{-1}$.

Beispiel 7: N-[S-5(RS),6(SR)-5-Hydroxy-7-cis-pentadecen-6-yl-N-trifluoracetyl-cysteinyl]-glycin-methylester,
aus 0.5 g 5(RS),6(RS)-5,6-Epoxy-7-cis-pentadecen (E4) und 0.71 g N-(N-Trifluoracetylcysteinyl)-glycin-methylester.
IR ($CH_2Cl_2$): 3400, 2970, 2950, 2870, 1760, 1740, 1695, 1530 $cm^{-1}$.

Beispiel 7a: S-5 (RS),6(SR)-5-Hydroxy-7-cis-pentadecen-6-yl-N-trifluoracetyl-cystein-methylester,
aus 0.5 g 5(RS),6(RS)-5,6-Epoxy-7-cis-pentadecen (E4) und 0.58 g N-Trifluoracetylcystein-methylester.

Beispiel 8: S-5(RS),6(SR)-5-Hydroxy-7-cis-pentadecen-6-yl-cystein-methylester,
aus 0.5 g 5(RS),6(RS)-5,6-Epoxy-7-cis-pentadecen (E4) und 0,77 g Cystein-methylester-hydrochlorid.
IR ($CH_2Cl_2$): 3600, 3400, 2970, 2940, 2870, 1750, 1475, 1445 $cm^{-1}$.

Beispiel 9: S-5(RS),6(SR)-5-Hydroxy-7-cis-pentadecen-6-yl-mercaptoessigsäure-methylester,
aus 0,9 g 5(RS),6(RS)-Epoxy-7-cis-pentadecen (E4) und 0.47 g 2-Mercaptoessigsäure-methylester.
IR ($CH_2Cl_2$):3600, 2970, 2990, 2870, 1750, 1475, 1445 $cm^{-1}$.

Beispiel 10: S-5(RS),6(SR)-5-Hydroxy-7-cis-heptadecen-6-yl-cysteinyl-methylester,
aus 1 g 5(RS),6(RS)-5,6-Epoxy-7-cis-heptadecen (E1) und 1.4 g Cystein-methylester-hydrochlorid.
IR ($CH_2Cl_2$): 3600, 3400, 2940, 2870, 1750, 1475 $cm^{-1}$.

Beispiel 11: N-[S-5(RS),6(SR)-5-Hydroxy-7-cis-eicosen-6-yl-N-trifluoracetyl-cysteinyl]-glycin-methylester,
aus 1 g 5(RS),6(RS)-S,6-Epoxy-7-cis-eicosen (E5) und 1 g N-(N-Trifluoracetylcysteinyl)-glycin-methylester.
IR ($CH_2Cl_2$): 3400, 2930, 2860, 1750, 1735, 1690, 1530 $cm^{-1}$.

Beispiel 12: N-[S-5(RS),6(SR)-5-Hydroxy-7-cis-tricosen-6-yl-N-trifluoracetyl-cysteinyl]-glycin-methylester,
aus 0.6 g 5(RS),6(RS)-5,6-Epoxy-7-cis-tricosen (E6) und 0.58 g N-(N-Trifluoracetylcysteinyl)-glycin-methylester.
IR ($CH_2Cl_2$): 3400, 2940, 2870, 17560, 1740, 1700, 1530 $cm^{-1}$.

Beispiel 13: N-{3-[4(RS),5(SR)-4-Hydroxy-6-cis-tetradecen-5-yl-thio]-propionyl)-glycin-methylester,
aus 300 mg 4(RS),5(RS)-4,5-Epoxy-6-cis-tetradecen (E7) und 275 mg N-(3-Mercaptopropionyl)-glycin-methylester [S.Okuyama et al, Chem. Pharm. Bull. 30 , 2453 (1982)].
IR ($CH_2Cl_2$): 3450, 2970, 2940, 2870, 1760, 1690, 1525 $cm^{-1}$.

Beispiel 14: N-[S-4(RS),5(SR)-4-Hydroxy-6-cis-tetradecen-5-yl-N-trifluoracetyl-cysteinyl]-glycin-methylester,
aus 0.7 g 4(RS),5(RS)-4,5-Epoxy-6-cis-tetradecen (E7) und 1,2 g N-(N-Trifluoracetylcysteinyl)-glycin-methylester.
IR ($CH_2Cl_2$): 3400, 2980, 2940, 2870, 1760, 1740, 1700, 1530 $cm^{-1}$.

Beispiel 15: N-{3[4(RS),5(SR)-4-Hydroxy-6-cis-nonadecen-5-yl-thio]-propionyl}-glycin-methylester,
aus 250 mg 4(RS),5(RS)-4,5-Epoxy-6-cis-nonadecen (E8) und 175 mg N-(3-Mercaptopropionyl)-glycin-methylester.

IR ($CH_2Cl_2$): 3450, 2980, 2940, 2870, 1760, 1690, 1530 $cm^{-1}$.

Beispiel 16: N-[S-4(RS),5(SR)-4-Hydroxy-6-cis-nonadecen-5-yl-N-trifluoracetyl-cysteinyl]-glycin-methylester, aus 500 mg 4(RS),5(RS)-4,5-Epoxy-6-cis-nonadecen (E8) und 620 mg N-(N-Trifluoracetylcysteinyl)-glycin-methylester.
IR ($CH_2Cl_2$): 3400, 2970, 2940, 2870, 1760, 1740, 1700, 1530 $cm^{-1}$.

Beispiel 17: N-[S-4(RS),5(SR)-4-Hydroxy-6-cis-eicosen-5-yl-N-trifluoracetyl-cysteinyl]-glycin-methylester, aus 680 mg 4(RS),5(RS)-4,5-Epoxy-6-cis-eicosen (E9) und 830 mg N-(N-Trifluoracetylcysteinyl)-glycin-methylester.
IR ($CH_2Cl_2$): 3400, 2940, 2870, 1760, 1740, 1700, 1530 $cm^{-1}$.

Beispiel 17a: N-{3-[4(RS),5(SR)-4-Hydroxy-6-cis-eicosen-5-yl-thio]propionyl}-glycin-methylester, aus 680 mg 4(RS),5(RS)-4,5-Epoxy-6-cis-eicosen (E9) und 720 g N-($\beta$-Mercaptopropionyl)-glycin-methylester.

Beispiel 17b: S-[4(RS),5(SR)-4-Hydroxy-6-cis-eicosen-5-yl-]-N-trifluoracetyl-cystein-methylester, aus 680 mg 4(RS),5(RS)-4,5-Epoxy-6-cis-eicosen (E9) und 620 mg N-Trifluoracetylcystein-methylester.

Beispiel 18: N-[S-6(RS),7(SR)-6-Hydroxy-8-cis-eicosen-7-yl-N-trifluoracetylcysteinyl]-glycin-methylester, aus 0,54 g 6(RS),7(RS)-6,7-Epoxy-8-cis-eicosen (E10) und 0,53 g N-(N-trifluoracetylcysteinyl)-glycin-methylester.
IR ($CH_2Cl_2$): 3400, 2940, 2870, 1760, 1740, 1700, 1530 $cm^{-1}$.

Beispiel 18a: N-{2-[6(RS),7(SR)-6-Hydroxy-8-cis-eicosen-7-yl-thio]-propionyl}-glycin, aus 0,54 g 6(RS),7(RS)-6,7-Epoxy-8-cis-eicosen (E10) und 0,45 g N-($\alpha$-Mercaptopropionyl)-glycin.

Beispiel 18b: S-[6(RS),7(SR)-6-Hydroxy-8-cis-eicosen-7-yl-]-cystein-methylester. aus 0,54 g 6(RS),7(RS)-6,7-Epoxy-8-cis-eicosen (E10) und 0,45 g Cystein-methylester.

Beispiel 19: N-[S-5(RS),6(SR)-5-Hydroxy-1-tetrahydropyranyloxy-7-cis-octadecen-6-yl-N-trifluoracetyl-cysteinyl]-glycin-methylester, aus 0.7 g 5(RS),6(RS)-5,6-Epoxy-1-tetrahydropyranyloxy-7-cis-octadecen (E11) und 0,6 g N(N-Trifluoracetylcysteinyl)-glycin-methylester.
IR ($CH_2Cl_2$):3400, 2940, 2870, 1740, 1700, 1530 $cm^{-1}$.

Beispiel 19a: S-5(RS),6(SR)-5-Hydroxy-1-tetrahydropyranyloxy-7-cis-octadecen-6-yl-mercaptoessigsäure-methylester, aus 0.7 g 5(RS),6(RS)-5,6-Epoxy-1-tetrahydropyranyloxy-7-cis-octadecen (E11) und 0.35 g Mercaptoessigsäure-methylester.

Beispiel 20: N-[S-5(RS),6(SR)-5-Hydroxy-1-tetrahydropyranyloxy-7-cis-eicosen-6-yl-N-trifluoracetyl-cysteinyl]-glycin-methylester, aus 0.8 g 5(RS),6(RS)-5,6-Epoxy-1-tetrahdropyranyloxy-7-cis-eicosen (E12) und 0.64 N-(N-Trifluoracetylcysteinyl)-glycin-methylester.
IR ($CH_2Cl_2$): 3400, 2940, 2860, 1760, 1740, 1700, 1530 $cm^{-1}$.

Beispiel 20a: S-5(RS),6(SR)-5-Hydroxy-1-tetrahydropyranyloxy-7-cis-eicosen-6-yl-mercaptoessigsäure-methylester, aus 0.8 g 5(RS),6(RS)-5,6-Epoxy-1-tetrahydropyranyloxy-7-cis-eicosen (E12) und 0,4 g Mercaptoessigsäure-methylester.

Beispiel 21: N-[S-5(RS),6(SR)-5-Hydroxy-7-trans-9-cis-nonadecadien-6-yl-N-trifluoracetyl-cysteinyl]-glycin-methylester, aus 0.55 g 5(RS),6(RS)-5,6-Epoxy-7-trans-9-cis-nonadecadien (E13) und 0.62 g N-(N-Trifluoracetylcysteinyl)-glycin-methylester.

Beispiel 21a: N-{-3-[5(RS),6(SR)-5-Hydroxy-7-trans-9-cis-nonadecadien-6-yl-thio]-propionyl}-glycin-methylester, aus 0.55 g 5(RS),6(RS)-5,6-Epoxy-7-trans-9-cis-nonadecadien (E13) und 0,53 g N-(3-Mercaptopropionyl)-glycin-methylester.

Beispiels 21b: S-{5(RS),6(SR)-5-Hydroxy-7-trans-9-cis-nonadecadien-6-yl-]-cystein-methylester, aus 0.55 g 5(RS),6(RS)-5,6-Epoxy-7-trans-9-cis-nonadecadien (E13) und 0,40 g Cystein-methylester.

Beispiel 21c: S-[5(RS),6(SR)-5-Hydroxy-7-trans-9-cis-nonadecadien-6-yl-]-mercaptoessigsäure-methylester, aus 0.55 g 5(RS),6(RS)-5,6-Epoxy-7-trans-9-cis-nonadecadien (E13) und 0.32 g Mercaptoessigsäure-methylester.

Beispiel 21d: S-[5(RS),6(SR)-5-Hydroxy-7-trans-9-cis-nonadecadien-6-yl-]-mercaptoessigsäure, aus 0.55 g 5(RS),6(RS)-5,6-Epoxy-7-trans-9-cis-nonadecadien (E13) und 0.30 g Mercaptoesssäure.

Beispiel 21e : N-[S-5-Hydroxy-7-trans-9-cis-eicosadien-6-yl N-trifluoracetyl-cysteinyl]-glycin-methylester; Diastereomere 5(R),6(S)-[A] und 5(S),6(R)- [B] (durch Chromatographie):
aus 0,6 g racemischem 5(RS),6(RS)-5,6-Epoxy-7-trans-9-cis-eicosadien (E13a) und 0,52 g N-(N-Trifluoracetyl-cysteinyl)-glycin-methylester wird ein Gemisch beider Diastereomeren im Verhältnis von etwa 1:1 erhalten, welches zu individuellen Formen [A] und [B] der Titelverbindung durch Chromatographie an

Kieselgel mit Hexan-Ethylacetat (7:3) getrennt wird.

Beispiel 21f: N-[S-4(RS),5(SR)-4-hydroxy-6-trans-8-cis-nonadecadien-5-yl-N-trifluoracetyl-cysteinyl]-glycin-methylester;
aus 0.6 g 4(RS),5(RS)-Epoxy-6-trans-8-cis-nonadecadien (E13b) und 0,52 g N-(N-Trifluoracetyl-cysteinyl)-glycin-methylester.

Beispiel 22: N-[S-5-Hydroxy-1-tetrahydropyranyloxy-7-trans-9-cis-octadecadien-6-yl-N-trifluoracetyl-cysteinyl]-glycin-methylester;
Diastereomere 5(R),6(S)- [A] und 5(S) ,6(R)- [B] (durch Chromatographie):
aus 0,6 g racemischem 5(RS),6(RS)-5,6-Epoxy-1-tetrahydropyranyloxy-7-trans-9-cis-octadecadien (E14) und 0.52 g N-(N-Trifluoracetyl-cysteinyl)-glycin-methylester wird ein Gemisch beider Diastereomeren im Verhältnis von etwa 1:1 erhalten, welches zu individuellen Formen [A] und [B] der Titelverbindung durch Chromatographie an Kieselgel mit Hexan-Ethylacetat (1:1) getrennt wird. Beide Verbindungen weisen ein praktisch identisches Spektrum auf: IR ($CH_2Cl_2$): 3400, 2940, 2870, 1760, 1740, 1695, 1530 $cm^{-1}$.

Beispiel 22a: S-[5(RS),6(SR 5-Hydroxy-1-tetrahydropyranyloxy-7-trans-9-cis-octadecadien-6-yl-]mercaptoessigsäure-methylester:
aus 0,9 g 5(RS),6(RS)-5,6-Epoxy-1-tetrahydropyranyloxy-7-trans-9-cis-octadecadien (E14) und 0,45 g Mercaptoessigsäure-methylester.

Beispiel 23: N-[S-5-Hydroxy-1-tetrahydropyranyloxy-7-trans-9-cis-eicosadien-6-yl-N-trifluoracetyl-cysteinyl]-glycin-methylester:
Diastereomere 5(R),6(S)- [A] und 5(S),6(R)- [B] (durch Chromatographie).
Aus 0.5 g razemischem 5(RS),6(RS)-5,6-Epoxy-1-tetrahydropyranyloxy-7-trans-9-cis-eicosadien (E15) und 0. 4 g N-(N-Trifluoracetyl-cysteinyl)-glycin-methylester wird ein Gemisch beider Diastereomeren im Verhältnis von etwa 1:1 erhalten, welches zu individuellen Formen [A] und [B] der Titelverbindung durch Chromatographie an Kieselgel mit Hexan-Ethylacetat (1:1) getrennt wird. Beide Verbindungen weisen ein praktisch identisches Spektrum auf: IR ($CH_2Cl_2$): 3400, 2940, 2870, 1760, 1740, 1700, 1530 $cm^{-1}$.

Beispiel 23a: S-[5(RS),6(SR)-5-Hydroxy-1-tetrahydropyranyloxy-7-trans-9-cis-eicosadien-6-yl-]mercaptoessigsäure-methylester:
aus 0,91 g 5(RS),6(RS)-5,6-Epoxy-1-tetrahydropyranyloxy-7-trans-9-cis-eicosadien (E15) und 0,45 g Mercaptoessigsäure-methylester:

Beispiel 24: N-[S-5(RS),6(SR)-5-Hydroxy-7,9-trans-11-cis-hexadecatrien-6-yl-N-trifluoracetyl-cysteinyl]-glycin-methylester, aus 1.1 g 5(RS),6(RS)-5,6-Epoxy-7,9-trans-11-cis-hexadecatrien (E16) und 1.3 g N-(N-Trifluoracetyl-cysteinyl)-glycin-methylester
IR ($CH_2Cl_2$): 3400, 2980, 2950, 2880, 1740, 1700, 1530 $cm^{-1}$.

Dieses Diastereomerengemisch wird durch Chromatographie an Kieselgel mit Hexan-Ethylacetat (4:1) in beide optisch einheitliche Formen getrennt: als erstes wird das 5(S),6(R)-Diastereomere eluiert, gefolgt durch das 5(R),6(S)-Diastereomere.

Beispiel 25: N-[S-5(RS),6(SR)-5-Hydroxy-7,11-cis-9-trans-hexadecatrien-6-yl-N-trifluoracetyl-cysteinyl]-glycin-methylester, aus 0.5 g 5(RS),6(RS)-5,6-Epoxy-7,11-cis-9-trans-hexadecatrien (E17) und 0.62 N-(N-Trifluoracetyl-cysteinyl)-glycin-methylester;
IR ($CH_2Cl_2$): 3400, 2970, 2950, 2880, 1740, 1700, 1530 $cm^{-1}$.

Dieses Diastereomerengemisch wird durch Chromatographie an Kieselgel mit Hexan-Ethylacetat (2:1) in beide optisch einheitliche Formen getrennt: als erstes wird das 5(S),6(R)-Diastereomere, $[\alpha]_D^{20}$ = +102.2 ± 4.4°, eluiert, gefolgt durch das 5(R),6(S)-Diastereomere, $[\alpha]_D^{20}$ = -41.4 ± 2.9°; beide Werte werden in Chloroform-Lösung von Konzentration 0.255% (G/V) bzw. 0.35 % (G/V) gemessen.

Beispiel 25a: N-[S-5(RS),6(SR)-5-Hydroxy-7,9-trans-11-cis-octadecatrien-6-yl-N-trifluoracetyl-cysteinyl]-glycin-methylester, aus 0.5 g 5(RS),6(RS)-5,6-Epoxy-7,9-trans-11-cis-octadecatrien (E17a) und 0.62 N-(N-Trifluoracetyl-cysteinyl)-glycin-methylester.

Beispiel 26: N-[S-5(RS),6(SR)-5-Hydroxy-7,9-trans-11-cis-eicosatrien-6-yl-N-trifluoracetyl-cysteinyl]-glycin-methylester, aus 0.54 g 5(RS),6(RS)-5,6-Epoxy-7,9-trans-11-cis-eicosatrien (E18) und 0.58 g N-(N-Trifluoracetyl-cysteinyl)-glycin-methylester.
IR ($CH_2Cl_2$): 3400, 2970, 2940, 2870, 1760, 1740, 1700, 1530 $cm^{-1}$.

Durch Chromatographie an Kieselgel mit Hexan-Ethylacetat (3:2) wird dieses Diastereomerengemisch zu individuellen optisch einheitlichen Formen getrennt; in ersteren Fraktionen wird das 5(S),6(R)-Diastereomere eluiert, gefolgt mit N-[S-5(R),6(S)-5-Hydroxy-7,9-trans-11-cis-eicosatrien-6-yl-N-trifluoracetyl-cysteinyl]-glycin-methylester, welches, sowie sein Diastereomeres, analoge spektrale Eigenschaften wie das Diastereomerengemisch aufweist.

Beide genannten optisch individuellen Verbindungen kann man auch aus entsprechenden optisch einheitlichen 5,6-Epoxiden durch Kondensation mit N-(N-Trifluoracetyl-cysteinyl)-glycin-methylester folgendermassen erhalten:

Beispiel 26A: N-[S-5(R),6(S)-5-Hydroxy-7,9-trans-11-cis-eicosatrien-6-yl-N-trifluoracetyl-cysteinyl]glycin-methylester:

Eine Lösung von 2,35 g 5(R),6(R)-5,6-Epoxy-7,9-trans-11-cis-eicosatrien in 28 ml Methanol wird unter Argon mit 2.48 g Triethylamin und 2.53 g N-(N-Trifluoracetyl-cysteinyl)-glycin-methylester versetzt und 16 Stunden bei 20° gerührt. Flüchtige Anteile werden im Wasserstrahlvakuum entfernt und der Rückstand an Kieselgel chromatographiert. Eluieren mit Hexan-Ethylacetat (3:2) liefert die Titelverbindung als farbloses Oel, welches mit dem oben charakterisierten Produkt identisch ist.

Die optisch aktive Epoxid-Komponente kann unter Anwendung des Verfahrens von Beispiel 1A folgendermassen hergestellt werden:

Unter wasserfreien Bedingungen wird eine gerührte Lösung von 66.3 ml Tetraisopropylorthotitanat und 38.51 ml D-(-)-Weinsäurediethylester in 1.1 1 Methylenchlorid bei -23° C nacheinander mit 25.7 g 2-trans-Heptenol (siehe Beispiel 1A) und 140 ml einer 3.2M-Lösung von tert-Butylhydroperoxid in Toluol versetzt, 16 Stunden bei -20° C gehalten und bei -23° C mit 56 ml 10%iger wässriger L-Weinsäure-Lösung tropfenweise behandelt. Nach weiteren 30 Minuten wird das Gemisch auf +20° C erwärmen gelassen und so lange noch weiter gerührt, bis sich die organische Schicht klar abtrennen lässt. Diese wird mit 1 1 1%iger wässriger Natriumsulfit-Lösung 1 Stunde gerührt, abgetrennt, mit Wasser gewaschen über Natriumsulfat getrocknet und im Wasserstrahlvakuum eingeengt. Der Rückstand wird in 1.6 1 Diethylether gelöst, auf 0° C gekühlt, tropfenweise mit 675 ml N-Natronlauge versetzt und 30 Minuten bei 0° C gerührt. Die abgetrennte organische Phase wird mit gesättigter Natriumchloridlösung gewaschen, getrocknet und eingeengt, womit 2(R),3(R)-2,3-Epoxyheptanol als farblose instabile Flüssigkeit resultiert, welche in der nächsten Stufe sofort verarbeitet wird.

Eine Lösung von 13.3 g der letztgenannten Verbindung in 100 ml Methylenchlorid wird innert 30 Minuten zu einer gerührten Suspension von 110.1 g Pyridiniumchlorochromat und 41.9 g Natriumacetat in 500 ml Methylenchlorid getropft, wobei die Temperatur durch leichtes Kühlen bei 25° C gehalten wird. Nach 3 Stunden wird das Reaktionsgemisch mit 500 ml Diethylether verdünnt und über Kieselgel filtriert. Das Filtrat wird mit Phosphatpuffer vom pH 8 gewaschen, über Natriumsulfat getrocknet und eingedampft. Chromatographie des Rückstandes an Kieselgel mit einem Gemisch von Petrolether (Sdp. 30-45°) und Diethylether (3:2) ergibt 2(S),3(R)-2,3-Epoxyheptanal als farblose Flüssigkeit; das Produkt weist analoge spektrale Eigenschaften wie sein 2(R),3(S)-Antipode (siehe Beispiel 1A) auf.

Eine Lösung von 6,7 g 2(S),3(R)-2,3-Epoxyheptanal in 250 ml Methylenchlorid wird bei 20° C während 1 Stunde mit einer Lösung von 20.85 g $\gamma$-Triphenylphosphoranylidencrotonaldehyd in 200 ml Methylenchlorid tropfenweise versetzt und eine weitere Stunde bei 20° C gerührt. Das Reaktionsgemisch wird mit 240 ml Hexan und 120 ml Ethylacetat verdünnt, über Kieselgel filtriert und eingeengt. Der Rückstand wird in gleiche Volummengen Hexan und Ethylacetat aufgenommen, 15 Minuten gerührt, erneut durch Kieselgel filtriert und eingeengt. Das resultierende ölige Gemisch von cis,trans- und trans,trans-Isomeren wird zwecks Isomerisierung in 200 ml Methanol gelöst, mit 220 mg Jod versetzt und bei 20° C 3 Stunden stehen gelassen. Nach Waschen mit einer wässrigen Natriumthiosulfatlösung und Wasser und Trocknen über Natriumsulfat wird die Lösung eingeengt und der Rückstand an Kieselgel chromatographiert. Eluieren mit Hexan-Ethylacetat (4:1) ergibt das gewünschte 6(R),7(R)-6,7-Epoxy-2,4-trans-undecadienal als gelbliches Oel,

$[\alpha]_D^{20}$ = -21.1 ± 1.3° (0.75 G/V-% in Chloroform), dessen spektrale Eigenschaften sich von denjenigen des 6(S),7(S)-Antipoden (siehe Beispiel 1A) nicht unterscheiden.

Zu einer auf -78° C abgekühlten, gerührten Lösung von 5.15 g Nonyltriphenylphosphoniumbromid in 50 ml Tetrahydrofuran wird unter Argon 6.85 ml einer 1.6M-Lösung von Butyllithium in Toluol zugesetzt. Nach 30 Minuten bei -78° C wird das Gemisch nacheinander durch tropfenweise Zugabe von 15.1 g Hexamethylphosphorsäuretriamid und einer Lösung von 1.52 g (6(R),7(R)-6,7-Epoxy-2,4-trans-undecadienal in 10 ml Tetrahydrofuran behandelt, weitere 15 Minuten bei -78° C gehalten und auf 0° C erwärmen gelassen. Das Reaktionsgemisch wird mit Phosphatpuffer (pH 8) versetzt und mit Ether extrahiert. Die vereinigten Ether-Auszüge werden mit einigen Tropfen Triethylamin stabilisiert, über Natriumsulfat getrocknet und bei 20° C im Vakuum von leicht-flüchtigen Anteilen befreit. Der Rückstand wird mit kleinen Mengen Ether verrührt und vom ausgeschiedenen festen Triphenylphosphinoxid durch Filtration befreit. Aus dem Filtrat entfernt man die letzten Anteile Triphenylphosphinoxids durch Filtration über eine Kieselgelsäule, die durch Auswaschen mit einem Gemisch (4:1) von Ether-Hexan mit einer 2%igen Beimischung von Triethylamin vorpräpariert wurde. Nach Abdestillieren der Lösungsmittel aus dem Filtrat resultiert das gewünschte 5(R),6(R)-5,6-Epoxy-7,9-trans-11-cis-eicosatrien in Form von leicht gelben Kristallen, Smp. 31-32° C.

Beispiel 26B: N-[S-5(S),6(R)-5-Hydroxy-7,9-trans-11-cis-eicosatrien-6-yl-N-trifluoracetyl-cysteinyl]-glycin-methylester erhält man in analoger Weise wie im Beispiel 26A, aber ausgehend vom 5(S),6(S)-5,6-Epoxy-7,9-trans-11-cis-eicosatrien. Die Titelverbindung ist mit dem Produkt, welches durch Chromatographie des Diastereomerengemisches (siehe oben) erhältlich ist, identisch.

Das als Ausgangsstoff benötigte 5(S),6(S)-5,6-Epoxy-7,9-trans-11-cis-eicosatrien kann in analoger Weise wie sein 5(R),6(R)-Antipode erhalten werden, indem man das 6(S),7(S)-6,7-Epoxy-2,4-transundecadienal (siehe Beispiel 1A) mit einem in situ aus Nonyltriphenylphosphoniumbromid und Butyllithium zubereiteten Wittig-Reagens gemäss dem im letzten Absatz des Beispiels 26A beschriebenen Verfahren umsetzt. Die spektralen Eigenschaften dieser Verbindung entsprechen denjenigen des im Beispiel 26A charakterisierten 5(R),6(R)-Antipoden.

Beispiel 27: N-[S-5(RS),6(SR)-5-Hydroxy-7,11-cis-9-trans-eicosatrien-6-yl-N-trifluoracetyl-cysteinyl]-glycin-methylester, aus 0.94 g 5(RS),6(RS)-5,6-Epoxy-7,11-cis-9-trans-eicosatrien (E19) und 1.0 g N-(N-Trifluoracetyl-cysteinyl)-glycin-methylester.

IR ($CH_2Cl_2$): 3400, 2970, 2940, 2870, 1740, 1700, 1535 $cm^{-1}$

Das Diastereomerengemisch kann durch Chromatographie analog Beispiel 25 in individuelle optisch einheitliche Diastereomere getrennt werden (Elutionsmittel: Hexan-Ethylacetat 7:3).

Beispiel 28: N-[S-5(RS),6(SR)-5-Hydroxy-1-tetrahydropyranyloxy-7,9-trans-11-cis-eicosatrien-6-yl-N-trifluoracetyl-cysteinyl]-glycin-methylester, individuelle Diastereomere 5(R),6(S)- [A] und 5(S),6(R)-[B].

Aus 0.78 racemischem 5(RS),6(RS)-5,6-Epoxy-1-tetrahydropyranyloxy-7,9-trans-11-cis-eicosatrien (E20) und 0.63 g N-(N-Trifluoracetyl-cysteinyl)-glycin-methylester wird ein Gemisch von Diastereomeren [A] und [B] im Verhältnis von etwa 1:1 erhalten, aus welchem individuelleFormen der Titelverbindung durch Chromatographie an Kieselgel mit Hexan-Ethylacetat (1:1) isoliert werden. Beide zeigen analoges Spektrum.

IR ($CH_2Cl_2$): 3400, 2940, 2870, 1740, 1695, 1530 $cm^{-1}$.

Beispiel 28a: N-[S-5(RS),6(SR)-1-Acetoxy-5-hydroxy-7,9-trans-11-cis-eicosatrien-6-yl-N-trifluoracetyl-cysteinyl]-glycin-methylester, individuelle Diastereomere 5(R),6(S)- [A] und 5(S),6(R)- [B].

Aus 0.78 racemischem 5(RS),6(RS)-1-Acetoxy-5,6-epoxy-7,9-trans-11-cis-eicosatrien (E20a) und 0.63 g N-(N-Trifluoracetyl-cysteinyl)-glycin-methylester wird ein Gemisch von Diastereomeren [A] und [B] im Verhältnis von etwa 1:1 erhalten, aus welchem individuelle Formen der Titelverbindung durch Chromatographie an Kieselgel mit Hexan-Ethylacetat (1:1) isoliert werden.

Beispiel 29: N-[S-5(RS),6(SR)-5-Hydroxy-7,9-trans-11,14-cis-eicosatetraen-6-yl-N-trifluoracetyl-cysteinyl]-glycin-methylester;

individuelle Diastereomere 5(R),6(S)- [A] und 5(S),6(R)- [B]. Aus 103 mg racemischem 5(RS),6(RS)-5,6-Epoxy-7,9-trans-11,14-cis-eicosatetraen (E21) und 154 mg N-(N-Trifluoracetyl-cysteinyl)-glycin-methylester. wird ein Gemisch von Diastereomeren [A] und [B] im Verhältnis von etwa 1:1 erhalten, aus welchem individuelle Formen der Titelverbindung durch Chromatographie an Kieselgel mit Hexan-Ethylacetat isoliert werden. Beide zeigen analoges Spektrum.

IR ($CH_2Cl_2$): 3360, 2920, 2850, 1740, 1720, 1680, 1520 $cm^{-1}$.

Beispiel 29a: S-5(RS),6(SR)-5-Hydroxy-7,9-trans-11,14-cis-eicosatetraen-6-yl-mercaptoessigsäure-methylester:

aus 103 mg 5(Rs),6(RS)-5,6-Epoxy-7,9-trans-11,14-cis-eicosatetraen (E21) und 54 mg Mercaptoessigsäure-methylester.

Beispiel 30: N-[S-5(RS),6(SR)-5-Hydroxy-1-tetrahydropyranyloxy-7,9-trans-11,14-cis-eicosatetraen-6-yl-N-trifluoracetyl-cysteinyl]-glycin-methylester; individuelle Diastereomere 5(R),6(S)- [A] und 5(S),6(R)- [B]. Aus 0.5 g racemischem 5(RS),6(RS)-5,6-Epoxy-1-tetrahydropyranyloxy-7,9-trans-11,14-cis-eicosatetraen(E22) und 0.42 g N-(N-Trifluoracetyl-cysteinyl)-glycin-methylester wird ein Gemisch von Diastereomeren [A] und [B] im Verhältnis von etwa 1:1 erhalten, aus welchem individuelle Formen der Titelverbindung durch Chromatographie an Kieselgel mit Hexan-Ethylacetat (1:1) isoliert werden. Beide zeigen analoges Spektrum.

IR ($CH_2Cl_2$): 3400, 2940, 2880, 1760, 1740, 1700, 1530 $cm^{-1}$.

Beispiel 31: N-[S-5(RS),6(SR)-1-Acetoxy-5-hydroxy-7,11,14-cis-9-trans-eicosatetraen-6-yl-N-trifluoracetyl-cysteinyl]-glycin-methylester; individuelle Diastereomere 5(R),6(S)- [A] und 5(S),6(R)- [B]. Aus 490 mg racemischem 5(RS),6(RS)-1-Acetoxy-5,6-epoxy-7,11,14-cis-9-trans-eicosatetraen (E23) und 400 mg N-(N-Trifluoracetyl-cysteinyl)-glycin-methylester, wird ein Gemisch von Diastereomeren [A] und [B] im Verhältnis von etwa 1:1 erhalten, aus welchem individuelle Formen der Titelverbindung durch Chromatographie an Kieselgel mit Hexan-Ethylacetat (1:1) isoliert werden. Beide zeigen analoges Spektrum.

IR ($CH_2Cl_2$): 3400, 2940, 2870, 1740, 1700, 1530 $cm^{-1}$. Diastereomeres [A]: $[\alpha]_D^{20}$ = -36.6 ± 2° (0.5 G/V-% in Chloroform)

Diastereomeres [B]: $[\alpha]_D^{20}$ = +63.0 ± 2° (0.5 G/V-% in Chloroform).

Nachträgliche Abspaltung der Hydroxyl-Schutzgruppe

Beispiel 32: N-[S-5(RS),6(SR)-1,5-Dihydroxy-7-cis-eicosen-6-yl-N-trifluoracetyl-cysteinyl]-glycin-methylester.

Die Lösung von 1,2 g 1-Tetrahydropyranylether der Titelverbindung (siehe Beispiel 20) in 70 ml eines Gemisches bestehend aus 4-Volumenteilen Essigsäure, 2 Tetrahydrofuran und 1 Wasser, wird während 6 Stunden bei 45° gerührt. Das Lösungsmittel wird im Vakuum abgedampft, der Rückstand mehrmals in Toluol extrahiert, und die Extrakte werden in Vakuum eingeengt. Nach Chromatographie des Rückstandes an Kieselgel mit Dichlormethan/Hexan (15:1) erhält man die Titelverbindung.
IR ($CH_2Cl_2$): 3620, 3400, 2940, 2870, 1760 1740, 1700, 1530, 1220, 1180 $cm^{-1}$.

Beispiel 33: N-[S-5(RS),6(SR)-1,5-Dihydroxy-7-cis-octadecen-6-yl-N-trifluoracetyl-cysteinyl]-glycin-methylester. Durch das Verfahren des Beispiels 32 wird die Titelverbindung aus 0.7 g ihres entsprechenden 1-Tetrahydropyranylethers (siehe Beispiel 19) erhalten.
IR ($CH_2Cl_2$): 3620, 3400, 2940, 2870, 1760, 1740, 1700, 1535, 1220, 1180 $cm^{-1}$.

Beispiel 33a: S-5(RS),6(SR)-1,5-Dihydroxy-7-cis-octadecen-6-yl-mercaptoessigsäure-methylester. Durch das Verfahren des Beispiels 32 wird die Titelverbindung aus 0.7 g ihres entsprechenden 1-Tetrahydropyranylethers (siehe Beispiel 19a) erhalten.

Beispiel 33b: S-5(RS),6(SR)-1,5-Dihydroxy-7-cis-eicosen-6-yl-mercaptoessigsäure-methylester. Durch das Verfahren des Beispiels 32 wird die Titelverbindung aus 0.7 g ihres entsprechenden 1-Tetrahydropyranylethers (siehe Beispiel 19b) erhalten.

Beispiel 34: N-[S-5(S),6(R)-1,5-Dihydroxy-7-trans-9-cis-octadecadien-6-yl-N-trifluoracetyl-cysteinyl]-glycin-methylester. Durch das Verfahren des Beispiels 32 wird die Titelverbindung aus 0,39 g ihres entsprechenden 1-Tetrahydropyranylethers (siehe Beispiel 22, Diastereomer [B]) erhalten.
IR ($CH_2Cl_2$): 3620, 3400, 2940, 2870, 1760, 1740, 1700, 1530, 1220, 1180 $cm^{-1}$.

Beispiel 35: N-[S-5(R),6(S)-1,5-Dihydroxy-7-trans-9-cis-octadecadien-6-yl-N-trifluoracetyl-cysteinyl]-glycin-methylester. Durch das Verfahren des Beispiels 32 wird die Titelverbindung aus 0.33 g ihres entsprechenden 1-Tetrahydropyranylethers (siehe Beispiel 22, Diastereomer [A]) erhalten.
IR ($CH_2Cl_2$): 3620, 3400, 2940, 2870, 1760, 1740, 1700, 1530, 1220, 1180$cm^{-1}$.

Beispiel 35a: S-5(RS),6(SR)-1,5-Dihydroxy-7-trans-9-cis-octadecadien-6-yl-mercaptoessigsäure-methylester. Durch das Verfahren des Beispiels 32 wird die Titelverbindung aus 0.33 g ihres entsprechenden 1-Tetrahydropyranylethers (siehe Beispiel 22a) erhalten.

Beispiel 36: N-[S-5(S),6(R)-1,5-Dihydroxy-7-trans-9-cis-eicosadien-6-yl-N-trifluoracetyl-cysteinyl]-glycin-methylester. Durch das Verfahren des Beispiels 32 wird die Titelverbindung aus 0,25 g ihres entsprechenden 1-Tetrahydropyranylethers (siehe Beispiel 23, Diastereomer [B]) erhalten.
IR ($CH_2Cl_2$): 3640, 3420, 2940, 2880, 1760, 1740, 1700, 1535, 1220, 1180 $cm^{-1}$.

Beispiel 37: N-[S-5(R),6(S)-1,5-Dihydroxy-7-trans-9-cis-eicosadien-6-yl-N-trifluoracetyl-cysteinyl]-glycin-methylester.
Durch das Verfahren des Beispiels 32 wird die Titelverbindung aus 0.24 g ihres entsprechenden 1-Tetrahydropyranylethers (siehe Beispiel 23, Diastereomer [A]) erhalten.
IR ($CH_2Cl_2$): 3620, 3400, 2940, 2870, 1760, 1740, 1700, 1535, 1220, 1180 $cm^{-1}$.

Beispiel 37a: S-5(RS),6(SR)-1,5-Dihydroxy-7-trans-9-cis-eicosadien-6-yl-mercaptoessigsäure-methylester. Durch das Verfahren des Beispiels 32 wird die Titelverbindung aus 0,25 g ihres entsprechenden 1-Tetrahydropyranylethers (siehe Beispiel 23a, Diastereomer [B]) erhalten.

Beispiel 38: N[S-5(S),6(R)-1,5-Dihydroxy-7,9-trans-11-cis-eicosatrien-6-yl-N-trifluoracetyl-cysteinyl]-glycin-methylester.
Durch das Verfahren des Beispiels 32 wird die Titelverbindung aus 0,56 g ihres entsprechenden 1-Tetrahydropyranylethers (siehe Beispiel 28, Diastereomer [B]) erhalten.
IR ($CH_2Cl_2$): 3620, 3400, 2940, 2860, 1760, 1740, 1695, 1535, 1220, 1180 $cm^{-1}$.

Beispiel 39: N-[S-5(R),6(S)-1,5-Dihydroxy-7,9-trans-11-cis-eicosatrien-6-yl-N-trifluoracetyl-cysteinyl]-glycin-methylester.
Durch das Verfahren des Beispiels 32 wird die Titelverbindung aus 49 mg ihres entsprechenden 1-Tetrahydropyranylethers (siehe Beispiel 28, Diastereomer [A]) erhalten.
IR ($CH_2Cl_2$): 3620, 3400, 2940, 2860, 1760, 1740, 1695, 1535, 1220, 1180 $cm^{-1}$.

Beispiel 40: N-[S-5(S),6(R)-1,5-Dihydroxy-7,9-trans-11,14-cis-eicosatetraen-6-yl-N-trifluoracetyl-cysteinyl]-glycin-methylester.
Durch das Verfahren des Beispiels 32 wird die Titelverbindung aus 140 mg ihres entsprechenden 1-Tetrahydropyranylethers (siehe Beispiel 30, Diastereomer [B]) erhalten.
IR ($CH_2Cl_2$): 3610, 3400, 2930, 2880, 1750, 1725, 1685, 1525, 1215, 1170 $cm^{-1}$.

Beispiel 41: N-[S-5(R),6(S)-1,5-Dihydroxy-7,9-trans-11,14-cis-eicosatetraen-6-yl-N-trifluoracetyl-cysteinyl]-glycin-methylester.
Durch das Verfahren des Beispiels 32 wird die Titelverbindung aus 140 mg ihres entsprechenden 1-Tetrahydropyranylethers (siehe Beispiel 30, Diastereomer [A]) erhalten.

Nachträgliche Verseifung der endständigen Estergruppe

Beispiel 42: N-[S-(5(RS),6(SR)-5-Hydroxy-7-cis-heptadecen-6-yl-N-trifluoracetyl-cysteinyl)-glycin-Natriumsalz. Die Lösung von 800 mg Methylester der Titelverbindung (siehe Beispiel 2) in 70 ml Methanol wird mit 22 ml 0.1-N wässriger Natronlauge versetzt und 16 Std. bei Raumtemperatur gerührt. Das Methanol wird im Vakuum bei 20° C abgedampft und der wässrige Rückstand mit 80 ml Acetonitril versetzt. Die Lösung wird über wenig Kieselgel filtriert und im Vakuum bei 20° C vom Lösungsmittel befreit. Der Rückstand wird mehrmals mit Chloroform extrahiert, und die Auszüge werden im Vakuum eingeengt. Nach Trocknen im Hochvakuum lässt sich der Rückstand, bestehend aus der Titelverbindung, pulverisieren.
IR ($CH_2Cl_2$):3320, 2980, 2940, 2870, 1730, 1675, 1400 $cm^{-1}$.

Beispiel 43: N-[S-5(RS),6(SR)-5-Hydroxy-7-cis-undecen-6-yl-N-trifluoracetyl-cysteinyl]-glycin-Natriumsalz. Gemäss dem in Beispiel 41 beschriebenen Verfahren gewinnt man die Titelverbindung aus 1.74 g des entsprechenden Methylesters (siehe Beispiel 3).
IR ($CH_2Cl_2$): 3320, 2980, 2940, 2880, 1730, 1675, 1610, 1400 $cm^{-1}$.

Beispiel 44: N-[S-5(RS),6(SR)-5-Hydroxy-7-cis-tridecen-6-yl-N-trifluoracetyl-cysteinyl]-glycin-Natriumsalz.
Gemäss dem in Beispiel 41 beschriebenen Verfahren gewinnt man die Titelverbindung aus 800 mg des entsprechenden Methylesters (siehe Beispiel 4).
IR ($CH_2Cl_2$): 3300, 2980, 2940, 2870, 1730, 1670, 1600, 1400 $cm^{-1}$.

Beispiel 45: S-5(RS),6(SR)-5-Hydroxy-7-cis-tridecen-6-yl-cystein-Natriumsalz.
Gemäss dem in Beispiel 41 beschriebenen Verfahren gewinnt man die Titelverbindung aus 300 mg des entsprechenden Methylesters (siehe Beispiel 5).
IR ($CH_2Cl_2$): 2970, 2940, 2860, 1740, 1620, 1430 $cm^{-1}$.

Beispiel 46: S-5(RS),6(SR)-5-Hydroxy-7-cis-tridecen-6-yl-mercaptoessigsäure-Natriumsalz. Gemäss dem in Beispiel 41 beschriebenen Verfahren gewinnt man die Titelverbindung aus 700 mg des entsprechenden Methylesters (siehe Beispiel 6).
IR ($CH_2Cl_2$): 3300, 2960, 2930, 2860, 1600, 1400 $cm^{-1}$.

Beispiel 47: N-[S-5(RS),6(SR)-5-Hydroxy-7-cis-pentadecen-6-yl-N-trifluoracetyl-cysteinyl]-glycin-Natriumsalz.
Gemäss dem in Beispiel 41 beschriebenen Verfahren gewinnt man die Titelverbindung aus 710 mg des entsprechenden Methylesters (siehe Beispiel 7).
IR ($CH_2Cl_2$): 3300, 2970, 2940, 2860, 1730, 1670, 1610, 1400 $cm^{-1}$.

Beispiel 47a: S-5(RS),6(SR)-5-Hydroxy-7-cis-pentadecen-6-yl-N-trifluoracetyl-cystein-Kaliumsalz.
Gemäss dem in Beispiel 41 beschriebenen Verfahren gewinnt man die Titelverbindung aus 710 mg des entsprechenden Methylesters (siehe Beispiel 7a).

Beispiel 48: S-5(RS),6(SR)-5-Hydroxy-7-cis-pentadecen-6-yl-cystein-Natriumsalz.
Gemäss dem in Beispiel 41 beschriebenen Verfahren gewinnt man die Titelverbindung aus 250 mg des entsprechenden Methylesters (siehe Beispiel 8).
IR ($CH_2Cl_2$): 2970, 2940, 2870, 1740, 1640, 1590, 1410 $cm^{-1}$.

Beispiel 49: S-5(RS),6(SR)-5-Hydroxy-7-cis-pentadecen-6-yl-mercaptoessigsäure-Natriumsalz.
Gemäss dem in Beispiel 41 beschriebenen Verfahren gewinnt man die Titelverbindung aus 950 mg des entsprechenden Methylesters (siehe Beispiel 9).
IR ($CH_2Cl_2$): 3300, 2960, 2930, 2860, 1600, 1400 $cm^{-1}$.

Beispiel 50: S-5(RS),6(SR)-5-Hydroxy-7-cis-heptadecen-6-yl-cystein-Natriumsalz.
Gemäss dem in Beispiel 41 beschriebenen Verfahren gewinnt man die Titelverbindung aus 500 mg des entsprechenden Methylesters (siehe Beispiel 10).
IR ($CH_2Cl_2$): 3300, 2940, 2870, 1600, 1410 $cm^{-1}$.

Beispiel 51: N-[S-5(RS),6(SR)-5-Hydroxy-7-cis-eicosen-6-yl-N-trifluoracetyl-cysteinyl]-glycin-Natriumsalz.
Gemäss dem in Beispiel 41 beschriebenen Verfahren gewinnt man die Titelverbindung aus 1.64 g des entsprechenden Methylesters (siehe Beispiel 11).
IR ($CH_2Cl_2$): 3300, 2930, 2860, 1730, 1670, 1600, 1400 $cm^{-1}$.

Beispiel 52: N-[S-5(RS),6(SR)-5-Hydroxy-7-cis-tricosen-6-yl-N-trifluoracetyl-cysteinyl]-glycin-Natriumsalz.
Gemäss dem in Beispiel 41 beschriebenen Verfahren gewinnt man die Titelverbindung aus 950 mg des entsprechenden Methylesters (siehe Beispiel 12).

Beispiel 53: N-{3-[4(RS),5(SR)-4-hydroxy-6-cis-tetradecen-5-yl-thio]-propionyl}-glycin-Natriumsalz.
Gemäss dem in Beispiel 41 beschriebenen Verfahren gewinnt man die Titelverbindung aus 200 mg des entsprechenden Methylesters (siehe Beispiel 13).

Beispiel 54: N-[S-4(RS),5(SR)-4-hydroxy-6-cis-tetradecen-5-yl-N-trifluoracetyl-cysteinyl]-glycin-Natriumsalz.
Gemäss dem in Beispiel 41 beschriebenen Verfahren gewinnt man die Titelverbindung aus 600 mg des entsprechenden Methylesters (siehe Beispiel 14).
IR ($CH_2Cl_2$): 3300, 2980, 2940, 2870, 1720, 1660, 1610, 1560 $cm^{-1}$.

Beispiel 54a: N-[S-4(RS),5(SR)-4-hydroxy-6-cis-nonadecen-5-yl-N-trifluoracetyl-cysteinyl]-glycin-Natriumsalz.
Gemäss dem in Beispiel 41 beschriebenen Verfahren gewinnt man die Titelverbindung, Smp. 69-73 °C, aus 600 mg des entsprechenden Methylesters (siehe Beispiel 16).

Beispiel 55: N-[S-4(RS),5(SR)-4-hydroxy-6-cis-eicosen-5-yl-N-trifluoracetyl-cysteinyl]-glycin-Natriumsalz.
Gemäss dem in Beispiel 41 beschriebenen Verfahren gewinnt man die Titelverbindung aus 420 mg des entsprechenden Methylesters (siehe Beispiel 17).

Beispiel 55a: N-{3-[4(RS),5(SR)-4-hydroxy-6-cis-eicosen-5-yl-thio]-propionyl-glycin-Natriumsalz.
Gemäss dem in Beispiel 41 beschriebenen Verfahren gewinnt man die Titelverbindung aus 420 mg des entsprechenden Methylesters (siehe Beispiel 17a).

Beispiel 55b: S-4(RS),5(SR)-4-hydroxy-6-cis-eicosen-5-yl-N-trifluoracetyl-cystein-Natriumsalz.
Gemäss dem in Beispiel 41 beschriebenen Verfahren gewinnt man die Titelverbindung aus 420 mg des entsprechenden Methylesters (siehe Beispiel 17b).

Beispiel 56: N-[S-6(RS),7(SR)-6-hydroxy-8-cis-eicosen-7-yl-N-trifluoracetyl-cysteinyl]-glycin-Natriumsalz.
Gemäss dem in Beispiel 41 beschriebenen Verfahren gewinnt man die Titelverbindung aus 570 mg des entsprechenden Methylesters (siehe Beispiel 18).
IR ($CH_2Cl_2$): 3300, 2940, 2870, 1730, 1675, 1620, 1400 $cm^{-1}$.

Beispiel 56a: S-5(RS),6(SR)-1,5-Dihydroxy-7-cis-octadecen-6-yl-mercaptoessigsäure-Natriumsalz.
Gemäss dem in Beispiel 41 beschriebenen Verfahren gewinnt man die Titelverbindung aus 570 mg des entsprechenden Methylesters (siehe Beispiel 33a).

Beispiel 56b: N-[S-5(RS),6(SR)-1,5-Dihydroxy-7-cis-eicosen-6-yl-N-trifluoracetyl-cysteinyl]-glycin-Kaliumsalz.
Gemäss dem in Beispiel 41 beschriebenen Verfahren gewinnt man die Titelverbindung aus 570 mg des entsprechenden Methylesters (siehe Beispiel 32).

Beispiel 56c: S-5(RS),6(SR)-1,5-Dihydroxy-7-cis-eicosen-6-yl-mercaptoessigsäure-Natriumsalz.
Gemäss dem in Beispiel 41 beschriebenen Verfahren gewinnt man die Titelverbindung aus 570 mg des entsprechenden Methylesters (siehe Beispiel 33b).

Beispiel 57: N-[S-5(RS),6(SR)-5-Hydroxy-7-trans-9-cis-nonadecadien-6-yl-N-trifluoracetyl-cysteinyl]-glycin-Natriumsalz.
Gemäss dem in Beispiel 41 beschriebenen Verfahren gewinnt man die Titelverbindung aus 410 mg des entsprechenden Methylesters (siehe Beispiel 21).
IR ($CH_2Cl_2$): 3300, 2940, 2860, 1730, 1670, 1610, 1400 $cm^{-1}$.

Beispiel 57a: N-{3-[5(RS),6(SR)-5-Hydroxy-7-trans-9-cis-nonadecadien-6-yl-thio]-propionyl}-glycin-Natriumsalz.
Gemäss dem in Beispiel 41 beschriebenen Verfahren gewinnt man die Titelverbindung aus 410 mg des entsprechenden Methylesters (siehe Beispiel 21a).

Beispiel 57b: N-[S-5(R),6(S)-5-Hydroxy-7-atrans-9-cis-eicosadien-6-yl-N-trifluoracetyl-cysteinyl]-glycin-Kaliumsalz.
Gemäss dem in Beispiel 41 beschriebenen Verfahren gewinnt man die Titelverbindung aus 410 mg des entsprechenden Methylesters (siehe Beispiel 21e, Diastereomeres [A]).

Beispiel 57c: N-[S-5(S),6(R)-5-Hydroxy-7-trans-9-cis-eicosadien-6-yl-N-trifluoracetyl-cysteinyl]-glycin-Kaliumsalz.
Gemäss dem in Beispiel 41 beschriebenen Verfahren gewinnt man die Titelverbindung aus 410 mg des entsprechenden Methylesters (siehe Beispiel 21e, Diastereomeres [B].

Beispiel 57d: N-[S-4(RS),5(SR)-4-Hydroxy-6-trans-8-cis-nonadecadien-5-yl-N-trifluoracetyl-cysteinyl]-glycin-Natriumsalz.

Gemäss dem in Beispiel 41 beschriebenen Verfahren gewinnt man die Titelverbindung aus 410 mg des entsprechenden Methylesters (siehe Beispiel 21f). Smp. 52-54° C.

Beispiel 57e: S-5(RS),6(SR)-1,5-Dihydroxy-7-trans-9-cis-octadecadien-6-yl-mercaptoessigsäure-Natriumsalz.

Gemäss dem in Beispiel 41 beschriebenen Verfahren gewinnt man die Titelverbindung aus 410 mg des entsprechenden Methylesters (siehe Beispiel 35a).

Beispiel 57f: N-[S-5(S),6(R)-1,5-Dihydroxy-7-trans-9-cis-eicosadien-6-yl-N-trifluoracetyl-cysteinyl]glycin-Kaliumsalz.

Gemäss dem in Beispiel 41 beschriebenen Verfahren gewinnt man die Titelverbindung aus 410 mg des entsprechenden Methylesters (siehe Beispiel 36).

Beispiel 57g : N-[S-5(R),6(S)-1,5-Dihydroxy-7-trans-9-cis-eicosadien-6-yl-N-trifluoracetyl-cysteinyl]-glycin-Kaliumsalz.

Gemäss dem in Beispiel 41 beschriebenen Verfahren gewinnt man die Titelverbindung aus 410 mg des entsprechenden Methylesters (siehe Beispiel 37).

Beispiel 58: N-[S-5(RS),6(SR)-5-Hydroxy-7,9-trans-11-cis-hexadecatrien-6-yl-N-trifluoracetyl-cysteinyl]-glycin-Natriumsalz.

Gemäss dem in Beispiel 41 beschriebenen Verfahren gewinnt man die Titelverbindung aus 1.17 g des entsprechenden Methylesters (siehe Beispiel 24).

IR ($CH_2Cl_2$): 3300, 2970, 2940, 2880, 1730, 1670, 1620, 1410 $cm^{-1}$.

Beispiel 59: N-[S-5(RS),6(SR)-5-Hydroxy-7,11-cis-9-trans-hexadecatrien-6-yl-N-trifluoracetyl-cysteinyl]-glycin-Natriumsalz.

Gemäss dem in Beispiel 41 beschriebenen Verfahren gewinnt man die Titelverbindung aus 730 mg des entsprechenden Methylesters (siehe Beispiel 25).

IR ($CH_2Cl_2$): 3300, 2970, 2940, 2880, 1730, 1675, 1625, 1410 $cm^{-1}$.

Beispiel 60: N-[S-5(RS),6(SR)-5-Hydroxy-7,9-trans-11-cis-eicosatrien-6-yl-N-trifluoracetyl-cysteinyl]-glycin-Natriumsalz.

Gemäss dem in Beispiel 41 beschriebenen Verfahren gewinnt man die Titelverbindung aus 230 mg des entsprechenden Methylesters (siehe Beispiel 26).

IR ($CH_2Cl_2$): 3350, 2960, 2930, 2860, 1720, 1675, 1540 $cm^{-1}$.

In analoger Weise, ausgehend von entsprechenden optisch individuellen Diastereomeren (siehe Beispiele 26A und 26B), können optisch individuelle Produkte erhalten werden:

Beispiel 60A: N-[S-5(R),6(S)-5-Hydroxy-7,9-trans-11-cis-eicosatrien-6-yl-N-trifluoracetyl-cysteinyl]-glycin-Natriumsalz:

Eine Lösung von 3.1 g N-[S-5(R),6(S)-5-Hydroxy-7,9-trans-11-cis-eicosatrien-6-yl-N-trifluoracetyl-cysteinyl]-glycin-methylester (siehe Beispiel 26A) in 50 ml Methanol wird unter Argon bei 0-5° C mit 26.8 ml 0.2N-Natronlauge tropfenweise versetzt, 20 Stunden bei 20° C gerührt und bei dieser Temperatur im Vakuum eingeengt. Durch "reversed-phase"-Chromatographie an Adsorbent Merck RP8 mit Methanol-Wasser (3:1) und Abdestillieren der Lösungsmittel im Vakuum resultiert die Titelverbindung als weisses amorphes Pulver.

Beispiel 60B: N-[S-5(S),6(R)-5-Hydroxy-7,9-trans-11-cis-eicosatrien-6-yl-N-trifluoracetyl-cysteinyl]-glycin-Natriumsalz:

Unter den Reaktionsbedingungen des Beispiels 60A und mit analogen Mengen der Reaktionspartner und Hilfschemikalien, jedoch ausgehend von N-[S-5(S),6(R)-5-Hydroxy-7,9-trans-11-cis-eicosatrien-6-yl-N-trifluoracetyl-cysteinyl]-glycin-methylester (siehe Beispiel 26B), wird die Titelverbindung in Form eines weissen amorphen Pulvers erhalten.

Beispiel 61: N-[S-5(RS),6(SR)-5-Hydroxy-7,11-cis-9-trans-eicosatrien-6-yl-N-trifluoracetyl-cysteinyl]-glycin-Natriumsalz.

Gemäss dem in Beispiel 41 beschriebenen Verfahren gewinnt man die Titelverbindung aus 370 mg des entsprechenden Methylesters (siehe Beispiel 27).

IR ($CH_2Cl_2$): 3300, 2960, 2430, 2860, 1720, 1660, 1620, 1400 $cm^{-1}$.

In analoger Weise, ausgehend von entsprechenden optisch individuellen Diastereomeren (siehe die chromatographische Trennung im Beispiel 27), können optisch individuelle Produkte, d.h. das 5(S),6(R)-Diastereomere [A] und das 5(R),6(S)-Diastereomere [B] erhalten werden.

Beispiel 61a: N-[S-5(S),6(R)-1-Acetoxy-5-hydroxy-7,11-cis-9-trans-eicosatrien-6-yl-N-trifluoracetyl-cysteinyl]-glycin-Kaliumsalz und N-[S-5(S),6(R)-1,5-Dihydroxy-7,11-cis-9-trans-eicosatrien-6-yl-N-trifluoracetyl-cysteinyl]-glycin-Kaliumsalz.

Gemäss dem in Beispiel 41 beschriebenen Verfahren wird das entsprechende optisch individuelle Methylester-1-Acetat (siehe Beispiel 28) umgesetzt und das rohe Reaktionsgemisch durch Reverse-phase-chromatographie (Eluieren mit Methanol:Wasser 3:1) getrennt. Zunächst wird die Diol-verbindung eluiert,

gefolgt durch das 1-Acetat.

Beispiel 61b: N-[S-5(R),6(S)-1-Acetoxy-5-hydroxy-7,11-cis-9-trans-eicosatrien-6-yl-N-trifluoracetyl-cysteinyl]-glycin-Kaliumsalz und N-[S-5(R),6(S)-1,5-Dihydroxy-7,11-cis-9-trans-eicosatrien-6-yl-N-trifluoracetyl-cysteinyl]-glycin-Kaliumsalz.

Gemäss dem in Beispiel 41 beschriebenen Verfahren wird das entsprechende optisch individuelle Methylester-1-Acetat (siehe Beispiel 28) umgesetzt und das rohe Reaktionsgemisch durch Reverse-phase-chromatographie (Eluieren mit Methanol:Wasser 3:1) getrennt. Zunächst wird die Diol-verbindung eluiert, gefolgt durch das 1-Acetat.

Beispiel 61c: N-[S-5(R),6(S)-5-Hydroxy-7,9-trans-11,14-cis-eicosatetraen-6-yl-N-trifluoracetyl-cysteinyl]-glycin-Kaliumsalz.

Gemäss dem in Beispiel 41 beschriebenen Verfahren gewinnt man die Titelverbindung aus 370 mg des entsprechenden Methylesters (siehe Beispiel 29, Diastereomeres [A]).

Beispiel 61d: N-[S-5(S),6(R)-5-Hydroxy-7,9-trans-11,14-cis-eicosatetraen-6-yl-N-trifluoracetyl-cysteinyl]-glycin-Kaliumsalz.

Gemäss dem in Beispiel 41 beschriebenen Verfahren gewinnt man die Titelverbindung aus 370 mg des entsprechenden Methylesters (siehe Beispiel 29, Diastereomeres [B]).

Beispiel 61e: S-5(RS),6(SR)-5-Hydroxy-7,9-trans-11,14-cis-eicosatetraen-6-yl-mercaptoessigsäure-Kaliumsalz

Gemäss dem in Beispiel 41 beschriebenen Verfahren gewinnt man die Titelverbindung aus 370 mg des entsprechenden Methylesters (siehe Beispiel 29a).

Beispiel 61f: N-[S-5(S),6(R)-1,5-Dihydroxy-7,11,14-cis-9-tans-eicosatetraen-6-yl-N-trifluoracetyl-cysteinyl]-glycin-Kaliumsalz.

Gemäss dem in Beispiel 41 beschriebenen Verfahren gewinnt man die Titelverbindung aus 370 mg des entsprechenden Methylesters (siehe Beispiel 31, Dioastereomeres [B]).

Beispiel 61g: N-[S-5(R),6(S)-1,5-Dihydroxy-7,11,14-cis-9-tans-eicosatetraen-6-yl-N-trifluoracetyl-cysteinyl]-glycin-Kaliumsalz.

Gemäss dem in Beispiel 41 beschriebenen Verfahren gewinnt man die Titelverbindung aus 370 mg des entsprechenden Methylesters (siehe Beispiel 31, Diastereomeres [A]).

Nachträgliche Abspaltung der N-Trifluoracetylgruppe

Beispiel 62: N-[S-5(RS),6(SR)-5-Hydroxy-7-cis-heptadecen-6-yl-cysteinyl]-glycin-Natriumsalz.

Die Lösung von 590 mg $N^{cys}$-Trifluoracetylderivat der Titelverbindung (siehe Beispiel 41) in 15 ml Methanol wird mit einer Lösung von 1.7 g Natriumcarbonat in 15 ml Wasser versetzt. Die entstandene Suspension wird 20 Std. bei 60 °C gerührt. Das Reaktionsgemisch wird filtriert und das Filtrat im Vakuum eingedampft. Der Rückstand wird in Methanol/Dichlormethan (1:1) gelöst und erneut filtriert, und das Filtrat im Vakuum vom Lösungsmittel befreit. Der Rückstand ergibt durch Umkehrphasen-Chromatographie an Kieselgel mit Methanol/Wasser (3:1) die gewünschte Titelverbindung.

IR ($CH_2Cl_2$): 3300, 2940, 2870, 1680, 1600, 1400 $cm^{-1}$.

Beispiel 63: N-[S-5(Rs),6(SR)-5-Hydroxy-7-cis-undecen-6-yl-cysteinyl]-glycin-Natriumsalz.

Gemäss dem in Beispiel 62 beschriebenen allgemeinen Verfahren erhält man die Titelverbindung aus 1.74 g des entsprechenden N-Trifluoracetyl-Derivats (siehe Beispiel 42).

IR ($CH_2Cl_2$): 3300, 2980, 2940, 2880, 1730, 1670, 1610, 1400 $cm^{-1}$.

Beispiel 64: N-[S-5(RS),6(SR)-5-Hydroxy-7-cis-tridecen-6-yl-cysteinyl]-glycin-Natriumsalz.

Gemäss dem in Beispiel 62 beschriebenen allgemeinen Verfahren erhält man die Titelverbindung aus 320 mg des entsprechenden N-Trifluoracetyl-Derivats (siehe Beispiel 43).

IR ($CH_2CH_2$): 3400, 2960, 2930, 2860, 1680, 1600, 1400 $cm^{-1}$.

Beispiel 65: N-[S-5(RS),6(SR)-5-Hydroxy-7-cis-pentadecen-6-yl-cysteinyl]-glycin-Natriumsalz.

Gemäss dem in Beispiel 62 beschriebenen allgemeinen Verfahren erhält man die Titelverbindung aus 400 mg des entsprechenden N-Trifluoracetyl-Derivats (siehe Beispiel 46).

IR ($CH_2Cl_2$): 3380, 2960, 2930, 2860, 1650, 1600, 1400 $cm^{-1}$.

Beispiel 66: N-[S-5(RS),6(SR)-5-Hydroxy-7-cis-eicosen-6-yl-cysteinyl]-glycin-Natriumsalz.

Gemäss dem in Beispiel 62 beschriebenen allgemeinen Verfahren erhält man die Titelverbindung aus 1.64 g des entsprechenden N-Trifluoracetyl-Derivats (siehe Beispiel 50).

IR ($CH_2Cl_2$): 3300, 2930, 2860, 1730, 1670, 1600, 1400 $cm^{-1}$.

Beispiel 67: N-[S-5(RS),6(SR)-5-Hydroxy-7-cis-tricosen-6-yl-cysteinyl]-glycin-Natriumsalz

Gemäss dem in Beispiel 62 beschriebenen allgemeinen Verfahren erhält man die Titelverbindung aus 960

mg des entsprechenden N-Trifluoracetyl-Derivats (siehe Beispiel 51).
IR ($CH_2Cl_2$): 3250, 2940, 2870, 1680, 1600, 1400 $cm^{-1}$.

Beispiel 67a: N-[S-4(RS),5(SR)-4-Hydroxy-6-cis-nonadecen-5-yl-cysteinyl]-glycin-Natriumsalz.
Gemäss dem in Beispiel 62 beschriebenen allgemeinen Verfahren erhält man die Titelverbindung aus 200 mg des entsprechenden N-Trifluoracetyl-Derivats (siehe Beispiel 54a); Smp 182°C.

Beispiel 68: N-[S-4(RS),5(SR)-4-Hydroxy-6-cis-eicosen-5-yl-cysteinyl]-glycin-Natriumsalz.
Gemäss dem in Beispiel 62 beschriebenen allgemeinen Verfahren erhält man die Titelverbindung aus 200 mg des entsprechenden N-Trifluoracetyl-Derivats (siehe Beispiel 54).
IR ($CH_2Cl_2$): 3420, 2940, 2860, 1720, 1670, 1620, 1400 $cm^{-1}$.

Beispiel 68a: S-4(RS),5(SR)-4-Hydroxy-6-cis-eicosen-5-yl-cystein-Natriumsalz.
Gemäss dem in Beispiel 62 beschriebenen allgemeinen Verfahren erhält man die Titelverbindung aus 200 mg des entsprechenden N-Trifluoracetyl-Derivats (siehe Beispiel 55a); Smp. 153-156°C.

Beispiel 68b: N-[S-6(RS),7(SR)-6-Hydroxy-8-cis-eicosen-7-yl-cysteinyl]-glycin-Kaliumsalz.
Gemäss dem in Beispiel 62 beschriebenen allgemeinen Verfahren erhält man die Titelverbindung aus 200 mg des entsprechenden N-Trifluoracetyl-Derivats (siehe Beispiel 56).

Beispiel 68c: S-6(RS),7(SR)-6-Hydroxy-8-cis-eicosen-7-yl-cystein-Natriumsalz.
Gemäss dem in Beispiel 62 beschriebenen allgemeinen Verfahren erhält man die Titelverbindung aus 200 mg des entsprechenden N-Trifluoracetyl-Derivats (siehe Beispiel 18b).

Beispiel 68d: N-[S-5(RS),6(SR)-5-Hydroxy-7-trans-9-cis-nonadecadien-6-yl-cysteinyl]-glycin-Natriumsalz.
Gemäss dem in Beispiel 62 beschriebenen allgemeinen Verfahren erhält man die Titelverbindung aus 200 mg des entsprechenden N-Trifluoracetyl-Derivats (siehe Beispiel 57).

Beispiel 68e: N-[S-5(R),6(S)-5-Hydroxy-7-trans-9-cis-eicosadien-6-yl-cysteinyl]-glycin-Kaliumsalz.
Gemäss dem in Beispiel 62 beschriebenen allgemeinen Verfahren erhält man die Titelverbindung aus 200 mg des entsprechenden N-Trifluoracetyl-Derivats (siehe Beispiel 57b).

Beispiel 68f: N-[S-5(S),6(R)-5-Hydroxy-7-trans-9-cis-eicosadien-6-yl-cysteinyl]-glycin-Kaliumsalz.
Gemäss dem in Beispiel 62 beschriebenen allgemeinen Verfahren erhält man die Titelverbindung aus 200 mg des entsprechenden N-Trifluoracetyl-Derivats (siehe Beispiel 57c).

Beispiel 68g: N-[S-4(RS),5(SR)-4-Hydroxy-6-trans-8-cis-nonadecadien-5-yl-cysteinyl]-glycin-Natriumsalz.
Gemäss dem in Beispiel 62 beschriebenen allgemeinen Verfahren erhält man die Titelverbindung aus 200 mg des entsprechenden N-Trifluoracetyl-Derivats (siehe Beispiel 57d); Smp. 98-102°C.

Beispiel 68h: N-[S-5(S),6(R)-5-Hydroxy-7,9-trans-11-cis-eicosatrien-6-yl-cysteinyl]-glycin-Kaliumsalz.
Gemäss dem in Beispiel 62 beschriebenen allgemeinen Verfahren erhält man die Titelverbindung aus 200 mg des entsprechenden N-Trifluoracetyl-Derivats (siehe Beispiel 60B).

Beispiel 68i: N-[S-5(R),6(S)-5-Hydroxy-7,9-trans-11-cis-eicosatrien-6-yl-cysteinyl]-glycin-Kaliumsalz.
Gemäss dem in Beispiel 62 beschriebenen allgemeinen Verfahren erhält man die Titelverbindung aus 200 mg des entsprechenden N-Trifluoracetyl-Derivats (siehe Beispiel 60A).

Beispiel 68j: N-[S-5(S),6(R)-5-Hydroxy-7-cis-9-trans-11-cis-eicosatrien-6-yl-cysteinyl]-glycin-Kaliumsalz.
Gemäss dem in Beispiel 62 beschriebenen allgemeinen Verfahren erhält man die Titelverbindung aus 200 mg des entsprechenden N-Trifluoracetyl-Derivats (siehe Beispiel 61).

Beispiel 68k: N-[S-5(R),6(S)-5-Hydroxy-7-cis-9-trans-11-cis-eicosatrien-6-yl-cysteinyl]-glycin-Kaliumsalz.
Gemäss dem in Beispiel 62 beschriebenen allgemeinen Verfahren erhält man die Titelverbindung aus 200 mg des entsprechenden N-Trifluoracetyl-Derivats (siehe Beispiel 61).

Beispiel 68l: N-[S-5(S),6(R)-1,5-Dihydroxy-7,9-trans-11-cis-eicosatrien-6-yl-cysteinyl]-glycin-Kaliumsalz.
Gemäss dem in Beispiel 62 beschriebenen allgemeinen Verfahren erhält man die Titelverbindung aus 200 mg des entsprechenden N-Trifluoracetyl-Derivats (siehe Beispiel 61a).

Beispiel 68m: N-[S-5(R),6(S)-1,5-Dihydroxy-7,9-trans-11-cis-eicosatrien-6-yl-cysteinyl]-glycin-Kaliumsalz.
Gemäss dem in Beispiel 62 beschriebenen allgemeinen Verfahren erhält man die Titelverbindung aus 200 mg des entsprechenden N-Trifluoracetyl-Derivats (siehe Beispiel 61b).

Nachträgliche simultane Abspaltung der N-Trifluacetylgruppe und Verseifung der terminalen Estergruppe

Beispiel 69: N-[S-5(S),6(R)-1,5-Dihydroxy-7,9-trans-11,14-cis-eicosatetraen-6-yl-cysteinyl]-glycin-Kaliumsalz.
Zu einer Lösung von 50 mg $N^{cys}$-Trifluoracetyl-methylester der Titelverbindung [siehe Beispiel 39, Diastereomer 5(S),6(R)] in 4 ml Methanol gibt man die Lösung von 170 mg Kaliumcarbonat in 10 ml Wasser. Die Reaktionslösung wird 3 Tage unter Argon gerührt und im Vakuum bei Raumtemperatur eingedampft. Der Rückstand wird wiederholt in Chloroform aufgenommen und im Vakuum eingedampft. Nach Umkehrphasen-Chromatographie an Kieselgel im System Methanol-Wasser (3:1) gewinnt man die Titelverbindung.

Beispiel 69a: N-[S-5(RS),6(SR)-1,5-Dihydroxy-7-cis-octadecen-6-yl-cysteinyl]-glycin-Kaliumsalz. Gemäss dem im Beispiel 69 beschriebenen allgemeinen Verfahren erhält man die Titelverbindung aus 30 mg des entsprechenden N-Trifluoracetyl-methylesters (siehe Beispiel 33).

Beispiel 69b: N-[S-5(RS),6(SR)-1,5-Dihydroxy-7-cis-eicosen-6-yl-cysteinyl]-glycin-Kaliumsalz. Gemäss dem im Beispiel 69 beschriebenen allgemeinen Verfahren erhält man die Titelverbindung aus 30 mg des entsprechenden N-Trifluoracetyl-methylesters (siehe Beispiel 32).

Beispiel 69c: N-[S-5(S),6(R)-1,5-Dihydroxy-7-trans-9-cis-octadecadien-6-yl-cysteinyl]-glycin-Kaliumsalz. Gemäss dem im Beispiel 69 beschriebenen allgemeinen Verfahren erhält man die Titelverbindung aus 30 mg des entsprechenden N-Trifluoracetyl-methylesters (siehe Beispiel 34).

Beispiel 69d: N-[S-5(S),6(R)-5-Hydroxy-7,9-trans-11-cis-hexadecatrien-6-yl-cysteinyl]-glycin-Kaliumsalz. Gemäss dem im Beispiel 69 beschriebenen allgemeinen Verfahren erhält man die Titelverbindung aus 30 mg des entsprechenden N-Trifluoracetyl-methylesters (siehe Beispiel 24).

Beispiel 69e: N-[S-5(R),6(S)-5-Hydroxy-7,9-trans-11-cis-hexadecatrien-6-yl-cysteinyl]-glycin-Kaliumsalz. Gemäss dem im Beispiel 69 beschriebenen allgemeinen Verfahren erhält man die Titelverbindung aus 30 mg des entsprechenden N-Trifluoracetyl-methylesters (siehe Beispiel 24).

Beispiel 69f: N-[S-5(S),6(R)-5-Hydroxy-7,11-cis-9-trans-hexadecadien-6-yl-cysteinyl]-glycin-Kaliumsalz. Gemäss dem im Beispiel 69 beschriebenen allgemeinen Verfahren erhält man die Titelverbindung aus 30 mg des entsprechenden N-Trifluoracetyl-methylesters (siehe Beispiel 25).

Beispiel 69g: N-[S-5(R),6(S)-5-Hydroxy-7,11-cis-9-trans-hexadecadien-6-yl-cysteinyl]-glycin-Kaliumsalz. Gemäss dem im Beispiel 69 beschriebenen allgemeinen Verfahren erhält man die Titelverbindung aus 30 mg des entsprechenden N-Trifluoracetyl-methylesters (siehe Beispiel 25).

Beispiel 70: N-[S-5(R),6(S)-1,5-Dihydroxy-7,9-trans-11,14-cis-eicosatetraen-6-yl-cysteinyl]-glycin-Kaliumsalz.

Gemäss dem im Beispiel 69 beschriebenen allgemeinen Verfahren erhält man die Titelverbindung aus 30 mg des entsprechenden N-Trifluoracetyl-methylesters (siehe Beispiel 40, Diastereomer [5(R),6(S)].

Beispiel 71: N-[S-5(S),6(R)-5-Hydroxy-7,9-trans-11,14-cis-eicosatetraen-6-yl-cysteinyl]-glycin-Kaliumsalz. Gemäss dem im Beispiel 69 beschriebenen allgemeinen Verfahren erhält man die Titelverbindung aus 33 mg des entsprechenden N-Trifluoracetyl-methylesters (siehe Beispiel 29, Diastereomer [B]).
UV ($CH_3OH$): $\lambda_{max}$ = 280 nm ($\epsilon$ = 48 600).

Beispiel 72: N-[S-5(R),6(S)-5-Hydroxy-7,9-trans-11,14-cis-eicosatetraen-6-yl-cysteinyl]-glycin-Kaliumsalz. Gemäss dem im Beispiel 69 beschriebenen allgemeinen Verfahren erhält man die Titelverbindung aus 8 mg des entsprechenden N-Trifluoracetyl-methylesters (siehe Beispiel 29, Diastereomer [A]),
UV ($CH_3OH$): $\lambda_{max}$ - 280 nm ($\epsilon$ = 48600).

Beispiel 73: N-[S-5(S),6(R)-1,5-Dihydroxy-7,11,14-cis-9-trans-eicosatetraen-6-yl-cysteinyl]-glycin-Kaliumsalz. (Simultane Abspaltung von 3 Schutzgruppen).

Eine Lösung von 160 mg N-[S-5(S),6(R)-1-Acetoxy-5-hydroxy-7,11,14-cis-9-trans-eicosatetraen-6-yl-N-trifluoracetyl-cysteinyl]-glycinmethylester versetzt man mit einer Lösung von 700 mg Kaliumcarbonat in 50 ml Wasser, rührt 3 Tage unter Argon bei Raumtemperatur und dampft im Vakuum bei Raumtemperatur ein. Der Rückstand wird in mehrere Portionen Chloroform aufgenommen und der Extrakt im Vakuum eingedampft. Nach Filtration über Kieselgel in einer Lösung in Dichlormethan/Methanol (1:3) erhält man die gewünschte Titelverbindung.
IR ($CH_2Cl_2$): 3400, 2940, 1690, 1600, 1440, 1220, 1190 $cm^{-1}$.

Beispiel 74: N-[S-5(R),6(S)-1,5-Dihydroxy-7,11,14-cis-9-trans-eicosatetraen-6-yl-cysteinyl]-glycin-Kaliumsalz. (Simultane Abspaltung von 3 Schutzgruppen)

In analoger Weise wie im vorangehenden Beispiel wird 160 mg N-[S-5(R),6(S)-1-Acetoxy-5-hydroxy-7-11,14-cis-9-trans-eicosatetraen-6-yl-N-trifluoracetyl-cysteinyl]-glycin-methylester zur Titelverbindung hydrolysiert.
IR ($CH_2Cl_2$): 3420, 2940, 1685, 1615, 1420, 1220, 1190 $cm^{-1}$.

Beispiel 74a: N-[S-5(R),6(S)-1,5-Dihydroxy-7-tans-9-cis-eicosadien-6-yl-cysteinyl]-glycin-Kaliumsalz. (Simultane Abspaltung von 3 Schutzgruppen).
In analoger Weise wie im Beispiel 73 wird 160 mg N-[S-5(R),6(S)-1-Acetoxy-5-hydroxy-7-trans-9-cis-eicosaien-6-yl-N-trifluoracetyl-cysteinyl]-glycin-methylester vom Beispiel 23a zur Titelverbindung hydrolysiert.

Beispiel 75: N-[S-5(RS),6(SR)-5-Hydroxy-7-cis-heptadecen-6-yl-cysteinyl]-glycin.
Die Lösung von 250 mg Natriumsalz der Titelverbindung (siehe Beispiel 62) in 15 ml Dichlormethan wird mit 15 ml 5-prozentiger wässriger Essigsäure während 10 Minuten bei Raumtemperatur intensiv geschüttelt. Die organische Phase wird abgetrennt, mit Wasser gewaschen, über Natriumsulfat getrocknet und im Vakuum vom Lösungsmittel befreit. Es verbleiben 220 mg der Titelverbindung als amorpher Rückstand.
IR ($CH_2Cl_2$): 3300, 2960, 2870, 1680, 1620, 1410 $cm^{-1}$.

Anhang: Die in Beispielen 2 - 74 eingesetzten Ausgangsstoffe können folgendermassen hergestellt werden:

A. Ungesättigte Aldehyde:

A1. 2-trans-Heptenal:

Die Lösung von 44.8 ml Valeraldehyd und 6.3 g Formylmethylentriphenylphosphoran (S.Trippett und D.M.Walker, J.Chem. Soc. 1961 , 1266) in 400 ml Tetrahydrofuran und 150 ml Chloroform wird unter Argon 24 Std. rückfliessend erhitzt. Die rote Lösung wird im Vakuum bei Raumtemperatur vom Lösungsmittel befreit und der Rückstand mit Ether/Hexan (1:1) verrührt. Der feste Anteil wird abfiltriert und viermal mit Ether/Hexan (1;1) nachgewaschen. Das Filtrat wird im Vakuum eingedampft und der Rückstand im Vakuum destilliert. Man gewinnt die Titelverbindung als farblose Flüssigkeit (Kp. = 55-57° C /22mbar).

Auf analoge Weide werden erhalten:

A2. 2-trans-Hexenal (Kp. = 33° C/14 mbar)
aus 30.2 ml Butyraldehyd und 51 g Formylmethylentriphenylphosphoran.

A3. 2-trans-Octenal (Kp. = 65-69° C/16 mbar)
aus 20 g Capronaldehyd und 42.6 g Formylmethylentriphenylphosphoran.

A4. 7-Tetrahydropyranyloxy-2-trans-heptenal (Kp. = 106° C/5 mbar)
aus 16 g 5-Tetrahydropyranyloxypentanal [E.J. Core et al, J. Am. Chem. Soc. 92 , 6635 (1970)] und 26.1 g Formylmethylentriphenylphosphoran.

A5. 7-Acetoxy-2-trans-heptenal (Oel),
aus 3.9 g 5-Acetoxypentanal [H.C. Brown et al., Synthesis 1980 , 151] und 8.2 g Formylmethylentriphenylphosphoran, nach Chromatographie des Rohproduktes an Kieselgel mit Hexan/Ethylacetat (3:1).

B. Epoxyaldehyde

B1. 2(RS ),3(SR )-2,3-Epoxyheptanal

Die Lösung von 9 g 2-trans-Heptenal (A1) in 200 ml Dichlormethan/Methanol (1:1) wird mit 28 ml 30-proz. wässrigem Wasserstoffperoxid und mit 800 mg Kaliumcarbonat versetzt und 6 Std. bei Raumtemperatur gerührt. Man gibt 100 ml Phosphatpuffer (pH = 8) zu und trennt die organische Phase ab. Die wässrige Phase wird noch dreimal mit je 50 ml Dichlormethan extrahiert. Die vereinigten organischen Phasen werden mit 20 ml Phosphatpuffer gewaschen und über Magnesiumsulfat getrocknet. Die Lösung wird über wenig Florisil filtriert und im Vakuum eingedampft. Man erhält die Titelverbindung als farblose Flüssigkeit.
IR ($CH_2Cl_2$): 2950, 2920, 2850, 1720, 1460, 850 $cm^{-1}$.

Auf analoge Weise werden erhalten:

B2. 2(RS ),3(SR)-2,3-Epoxyhexanal
aus 16.2 g 2-trans -Hexenal (A2) als Oel,
IR ($CH_2Cl_2$): 2980, 2950, 2890, 1740, 1475, 860 $cm^{-1}$.

B3. 2(RS ),3(SR )-2,3-Octanal aus 9.0 g 2-trans -Octenal (A3) als Oel,
IR ($CH_2Cl_2$): 2970, 2950, 2870, 1740, 1475, 1025 $cm^{-1}$.

B4. 2(RS ),3(SR)-2,3-Epoxy-7-tetrahydropyranyloxyheptanal
aus 8.1 g 7-Tetrahydropyranyloxy-2-trans -heptenal (A4) durch Chromatographie des Rohproduktes an Kieselgel mit Hexan/Ethylacetat (2:1) als Oel,
IR ($CH_2Cl_2$): 2950, 2880, 1735, 1140, 1130, 1080, 1040 $cm^{-1}$.

B5. 2(RS ),3 (SR )-7-Acetoxy-2,3-epoxyheptanal
aus 2.2 g 7-Acetoxy-2-trans -heptenal (A5) nach Chromatographie des Rohproduktes an Kieselgel mit Dichlormethan/Ethylacetat (93:7) als Oel,
IR ($CH_2Cl_2$): 2960, 1740, 1375, 1240, 1050, 860 $cm^{-1}$.

C. Einfach ungesättigte Epoxyaldehyde

C1. 4(RS ),5(RS )-4,5-Epoxy-2-trans-nonenal

Die Lösung von 7.4 g 2(RS),3(SR)-Epoxyheptanal (B1) und 17.6 g Formylmethylentriphenylphosphoran in 250 ml Tetrahydrofuran und 100 ml Chloroform wird unter Argon 1,5 Std. rückfliessend erhitzt. Die erkaltete Lösung wird im Vakuum bei Raumtemperatur vom Lösungsmittel befreit und der Rückstand mit Ether/Hexan (4:1) verrührt. Die Suspension wird über wenig Kieselgel filtriert und mit Ether/Hexan (4:1) gewaschen. Das Filtrat wird im Vakuum eingeengt und der Rückstand mit Hexan/Ethylacetat (5:1, mit 1 % Triethylamin) an Kieselgel chromatographiert. Man erhält die Titelverbindung als farbloses Oel,
IR ($CH_2Cl_2$): 2970, 2940, 2870, 1690, 1650, 1115, 990 $cm^{-1}$.

Auf analoge Weise wird erhalten:
C1a. 4-(R S),5(RS )-4,5-Epoxy-2-trans-octenal
aus 2(RS),3(SR)-2,3-Epoxyhexanal (B2) als Oel.
C2. 4(RS ),5(RS)-4,5-Epoxy-9-tetrahydropyranyloxy-2-trans-nonenal
aus 2 g 2(RS),3(SR)-2,3-Epoxy-7-tetrahydropyranyloxyheptanal (B4) als Oel,
IR ($CH_2Cl_2$): 2950, 2880, 1700, 1125, 1040 $cm^{-1}$.
C2a. 4(RS),5(RS)-9-Acetoxy-4,5-epoxy-2-trans-nonenal
aus 2(RS),3(SR)-7-Acetoxy-2,3-epoxy-heptanal (B5) als Oel.
D. Zweifach ungesättigte Epoxyaldehyde
D1. 6(R S),7(RS )-6,7-Epoxy-2,4-undecadienal: 2-trans-4-trans-Isomeres (D1a) und 2-trans-4-cis-Isomeres (D1b ).
Zu einer Lösung von 1.75 g 2(RS),3(SR)-2,3-Epoxyheptanal (B1) in 40 ml Dichormethan tropft man unter Rühren und unter Argon die Lösung von 4.6 g 4-Triphenylphosphoranyliden-2-trans-butenal [M.J. Berenguer et al., Tetrahedron Lett. 1971, 495] innert 1 Stunde bei Raumtemperatur zu. Man rührt noch 1 Stunde nach und dampft das Lösungsmittel im Vakuum bei Raumteperatur ab. Der Rückstand wird mit Ether/Hexan (4:1) verrührt, über wenig Kieselgel filtriert und mit Ether/Hexan (4:1) nachgewaschen. Nach Abdampfen des Lösungsmittels im Vakuum wird der Rückstand an Kieselgel mit Hexan/Ethylacetat (4:1, mit 1 % Triethylamin) chromatographiert. Man erhält als hellgelbe Oele etwa gleiche Mengen des 2-trans -4-cis - (D1b) und 2-trans -4-trans -(D1a)-Isomeren der Titelverbindung, die beide analoge Spektralmaxima aufweisen:
IR ($CH_2Cl_2$): 2950, 2920, 1680, 1640, 1110, 990 $cm^{-1}$.
Auf analoge Weise werden erhalten:
D2. 6(RS),7(RS )-6,7-Epoxy-11-tetrahydropyranyloxy-2,4-trans-undecadienal
aus 1.4 g 2(RS),3(SR)-2,3-Epoxy-7-tetrahydropyranyloxyheptanal (B4) wird ein Isomerengemisch erhalten, welches dann in Dichlormethanlösung bis zur bleibenden Färbung mit Iod versetzt und anschliessend 5 Stunden bei Raumtemperatur gerührt wird. Nach Filtrieren über Kieselgel wird das Lösungsmittel abdestilliert und die Titelverbindung als Oel isoliert:
IR ($CH_2Cl_2$): 2960, 2880, 1690, 1650, 1580, 1120, 1040 $cm^{-1}$.
D3. 6(Rs ),7(RS)-11-Acetoxy-6,7-epoxy-2,4-undecadienal:
2-trans-4-trans-Isomeres (D3a) und 2-trans-4-cis-Isomeres (D3b). Ein Gemisch beider Isomeren wird aus 0.54 g 2(RS),3(SR)-7-Acetoxy-2,3-epoxy-heptanal (B5) im Verhältnis von etwa 1:2 (D3a: D3b) erhalten und chromatographisch aufgetrennt.
E. Epoxyolefine der Formel II
E1. 5(RS),6(RS)-5,6-Epoxy-7-cis-heptadecen
Zu einer auf -30 ° C gekühlten Lösung von 9.4 g n-Decyltriphenylphosphoniumbromid (C.T. Eyles und S. Trippett, J. Chem. Soc. (C) 1966 , 67) in 50 ml Tetrahydrofuran tropft man 12.2 ml einer 1.6-molaren Lösung von n-Butyllithium in Hexan unter Rühren und unter Argonatmosphäre zu, wobei die Temperatur zwischen -25 und -30 ° C gehalten wird. Man lässt die rote Lösung auf Raumtemperatur auftauen und rührt noch 10 Minuten bei dieser Temperatur. Nach Abkühlen auf -78 ° C tropft man die Lösung von 2 g 2(RS),3(SR)-2,3-Epoxy-heptanal (B1) in 10 ml Tetrahydrofuran innerhalb 15 Minuten zu. Man lässt die Lösung auf Raumtemperatur erwärmen und rührt noch 1 Stunde. Das Lösungsmittel wird im Vakuum bei 40 ° C abgedampft und der Rückstand in Dichlormethan gelöst. Die Lösung wird mit Kieselgel versetzt (so viel, dass alles Lösungsmittel aufgesaugt wird), dann mit Ether aufgeschlämmt und filtriert. Man wäscht viermal mit Ether/Hexan (1:1) nach und engt das Filtrat im Vakuum ein. Der Rückstand wird durch Chromatographie an Kieselgel mit Hexan/Ethylacetat (30:1, mit 1 % Triethylamin) gereinigt. Man erhält die Titelverbindung als farbloses Oel.
IR ($CH_2Cl_2$): 2980, 2940, 2870, 1475, 875 $cm^{-1}$.
Auf analoge Weise werden erhalten:
E2. 5(RS ),6(RS)-5,6-Epoxy-7-cis-undecen
aus 1.3 g 2(RS),3(SR)-2,3-Epoxy-heptanal (B1) und 5.5 g n-Butyltriphenylphosphoniumbromid [R. Mechoulam und F. Sondheimer, J. Am. Chem. Soc. 80 , 4386 (1958)].
IR ($CH_2Cl_2$): 2970, 2940, 2880, 1470, 875 $cm^{-1}$.
E3. 5(RS),6(RS)-5,6-Epoxy-7-cis-tridecen
aus 1.2 g 2(RS),3(SR)-2,3-Epoxy-heptanal (B1) und 5 g n-Hexyltriphenylphoshoniumbromid [C.F. Hauser et al., J. Org. Chem. 28 , 372 (1963)].
IR ($CH_2Cl_2$): 2970, 2940, 2870, 1475, 875 $cm^{-1}$.
E4 5(RS)6(RS )-5,6-Epoxy-7-cis-pentadecen
aus 1.25 g 2(RS),3(SR)-2,3-Epoxy-heptanal (B1) und 5.6 g n-Octyltriphenylphosphoniumbromid

[C.T.Eyles und S.Trippett, J.Chem.Soc. (C) 1966 , 67].
IR ($CH_2Cl_2$): 2970, 2930, 2870, 1475, 875 $cm^{-1}$.
E5 5(RS),6(RS)-5,6-Epoxy-7-cis-eicosen
aus 2 g 2(RS),3(SR)-2,3-Epoxy-heptanal (B1) und 12.3 g n-Tridecyltriphenylphosphoniumbromid [J. Gigg et al. J. Chem. Soc. 1966 , 1872].
IR ($CH_2Cl_2$): 2970, 2940, 2860, 1475, 875 $cm^{-1}$.
E6 5(RS),6(RS)-5,6-Epoxy-7-cis-tricosen
aus 1 g 2(RS),3(SR)-2,3-Epoxy-heptanal (B1) und 5.5 g n-Hexadecyltriphenylphosphoniumbromid [D. Jerchel und J.Kimmig, Chem. Ber.83 , 277 (1950)].
IR ($CH_2Cl_2$): 2970, 2935, 2870, 1470, 875 $cm^{-1}$.
E7 4(RS),5(RS)-4,5-Epoxy-6-cis-tetradecen
aus 2 g 2(RS),3(SR)-2,3-Epoxy-hexanal (B2) und 10 g n-Octyltriphenylphosphoniumbromid.
IR ($CH_2Cl_2$): 2990, 2960, 2880, 1480, 910 $cm^{-1}$.
E8 4(RS),5(RS)-4,5-Epoxy-6-cis-nonadecen
aus 1 g 2(RS),3(SR)-2,3-Epoxy-hexanal (B2) und 3.6 g n-Tridecyltriphenylphosphoniumbromid.
IR ($CH_2Cl_2$): 2980, 2940, 2870, 1475, 905 $cm^{-1}$.
E9 4(RS ),5(RS)-4,5-Epoxy-6-cis-eicosen
aus 0.58 g 2(RS),3(SR)-2,3-Epoxy-hexanal (B2) und 3.8 g n-Tetradecyltriphenylphosphoniumbromid [E.J.Reist und P.H. Christie, J.Org.Chem. 35 , 3521 (1970)].
IR ($CH_2Cl_2$): 2940, 2870, 1470, 905 $cm^{-1}$.
E10 6(RS),7(RS)-6,7-Epoxy-8-cis-eicosen
aus 1 g 2(RS),3(SR)-2,3-Epoxy-octanal (B3) und 4.1 g n-Dodecyltriphenylphosphoniumbromid [D. Jerchel und J Kimmig, Chem. Ber. 83 , 277 (1950)].
IR ($CH_2Cl_2$): 2940, 2870, 1465, 875 $cm^{-1}$.
E11 5(RS),6(RS )-5,6-Epoxy-1-tetrahydropyranyloxy-7-cis-octadecen
aus 1 g 2(RS),3(SR)-2,3-Epoxy-7-tetrahydropyranyloxy-heptanal (B4) und 6.6 g n-Undecyltriphenylphosphoniumbromid (hergestellt analog n-Decyltriphenylphosphoniumbromid).
IR ($CH_2Cl_2$): 2930, 2860, 1465, 1040 $cm^{-1}$.
E11a 5(RS ),6(RS)-1-Acetoxy-5,6-epoxy-7-cis-octadecen
unter denselben Bedingungen wie im vorangehenden Beispiel, jedoch aus 2(RS),3(SR)-7-Acetoxy-2,3-epoxy-heptanal (B5) anstelle des THP-Derivats B4 : Oel.
E12 5(RS ),6(RS )-5,6-Epoxy-1-tetrahdropyranyloxy-7-cis-eicosen
aus 1 g 2(RS),3(SR)-2,3-Epoxy-7-tetrahydropyranyloxy-heptanal (B4) und 6.6 g n-Tridecyltriphenylphosphoniumbromid.
IR ($CH_2Cl_2$): 2930, 2860, 1465, 1040 $cm^{-1}$.
E12a 5(RS ),6(RS)-1-Acetoxy-5,6-epoxy-7-cis-eicosen
unter denselben Bedingungen wie im vorangehenden Beispiel, jedoch aus 2(RS),3(SR)-7-Acetoxy-2,3-epoxy-heptanal (B5) anstelle des THP-Derivats B4 : Oel.
E13 5(RS),6(RS)-5,6-Epoxy-7-trans-9-cis-nonadecadien
aus 1 g 4(RS),5(RS)-4,5-Epoxy-2-trans-nonenal (C1) und 3.8 g n-Decyltriphenylphosphoniumbromid.
E13a 5(RS),6(RS )-5,6-Epoxy-7-trans-9-cis-eicosadien
aus 1 g 4(RS),5(RS)-4,5-Epoxy-7-trans-nonenal (C1) und 3.9 g n-Undecyltriphenylphosphoniumbromid : Oel.
E13b 4(RS),5(RS)-4,5-Epoxy-6-trans-8-cis-nonadecadien
aus 1 g 4(RS),5(RS)-4,5-Epoxy-7-trans-octenal (C1a) und 3.9 g n-Undecyltriphenylphosphoniumbromid : Oel.
E14 5(RS ),6(RS)-5,6-Epoxy-1-tetrahydropyranyloxy-7-trans-9-cis-octadecadien
aus 0.8 g 4(RS),5(RS)-4,5-Epoxy-9-tetrahydropyranyloxy-2-trans -nonenal (C2) und 2.2 g n-Nonyltriphenylphosphoniumbromid [G. Ohloff et al. Helv. Chim. Acta 60 , 1161 (1977)]
IR ($CH_2Cl_2$): 2940, 2870, 1585, 1460, 1040 $cm^{-1}$.
E14a 5(RS ),6(RS )-1-Acetoxy-5,6-epoxy-7-trans-9-cis-octadecadien
unter denselben Bedingungen wie im vorangehenden Beispiel, jedoch aus 4(RS),5(RS)-9-Acetoxy-4,4-epoxy-2-trans-nonenal (C2a) anstelle des THP-Derivats C2 : Oel.
E15 5 (RS ),6(RS )-5,6-Epoxy-1-tetrahydropyranyloxy-7-trans-9-cis-eicosadien
aus 0.8 g 4(RS),5(RS)-4,5-Epoxy-9-tetrahydropyranyloxy-2-trans -nonenal (C2) und 2.4 g n-Undecyltriphenylphosphoniumbromid.
IR ($CH_2Cl_2$): 2940, 2870, 1585, 1450, 1040 $cm^{-1}$.
E15a 5(RS),6(RS)-1-Acetoxy-5,6-epoxy-7-trans-9-cis-eicosadien

unter denselben Bedingungen wie im vorangehenden Beispiel, jedoch aus 4(RS),5(RS)-9-Acetoxy-4,5-epoxy-2-trans-nonenal (C2a) anstelle des THP-Derivats C2 : Oel.

E16 5(RS ),6(RS )-5,6-Epoxy-7,9-trans-11-cis-hexadecatrien

aus 0.95 g 6(RS),7(RS)-6,7-Epoxy-2,4-trans-undecadienal (D1a) und 2.8 g n-Pentyltriphenylphosphoniumbromid [L. Jaenicke et al., Liebigs Ann. Chem. 1973 , 1252].

IR ($CH_2Cl_2$): 2980, 2940, 2880, 1470, 1000, 870 $cm^{-1}$.

E17 5(R S),6(RS)-5,6-Epoxy-7,11-cis-9-trans-hexadecatrien

aus 0.78 g 6(RS),7(RS)-6,7-Epoxy-2-trans-4-cis-undecadienal (D1b) und 2.3 g n-Pentyltriphenylphosphoniumbromid.

IR ($CH_2Cl_2$): 2975, 2940, 2880, 1470, 1000, 870 $cm^{-1}$.

E17a 5(RS),6(RS)-5,6-Epoxy-7,9-trans-11-cis-octadecatrien

unter denselben Bedingungen wie im vorangehenden Beispiel, jedoch aus Heptyltriphenylphosphoniumbromid anstelle von Pentylderivat : Oel.

E18 5(RS ),6(RS )-5,6-Epoxy-7,9-trans-11-cis-eicosatrien

aus 0.65 g 6(RS),7(RS)-6,7-Epoxy-2,4-trans-undecadienal (Da) und 4.65 g n-Nonyltriphenylphosphoniumbromid.

E19 5(RS ),6(RS )-5,6-Epoxy-7,11-cis-9-trans-eicosatrien

aus 0.65 g 6(RS),7(RS)-6,7-Epoxy-2-trans-4-cis-undecadienal (D1b) und 4.65 g n-Nonyltriphenylphosphoniumbromid.

IR ($CH_2Cl_2$):

E20 5(RS ),6(RS )-5,6-Epoxy-1-tetrahydropyranyloxy-7,9-trans-11-cis-eicosatrien

aus 0.66 g 6(RS),7(RS)-6,7-Epoxy-11-tetrahydropyranyloxy-2,4-trans -undecadienal (D2) und 2.8 g n-Nonyltriphenylphosphoniumbromid

E20a 5(RS ),6(RS )-1-Acetoxy-5,6-epoxy-7,9-trans-11-cis-eicosatrien

unter denselben Bedingungen wie im vorangehenden Beispiel, jedoch aus 6(RS),7(RS)-11-Acetoxy-6,7-epoxy-2,4-trans-undecadienal (D3a) anstelle des THP-Derivats D2 : Oel.

E21 5(RS),6 (RS)-5,6-Epoxy-7,9-trans-11,14-cis-eicosatetraen

aus 125 mg 6(RS),7(RS)-6,7-Epoxy-2,4-trans-undecadienal (D1a) und 435 mg 3-cis-Nonenyltriphenylphosphoniumjodid [E.J.Corey, et al., J. Am. Chem. Soc. 101 , 6748 (1979)]

IR ($CH_2Cl_2$): 2910, 2840, 1450, 990 $cm^{-1}$.

E22 5(RS ),6(RS)-5,6-Epoxy-1-tetrahydropyranyloxy-7,9-trans-11,14-cis-eicosatetraen

aus 0.5 g 6(RS),7(RS)-6,7-Epoxy-11-tetrahydropyranyloxy-2,4-trans-undecadienal (D2) und 2.1 g 3-cis -Nonenyltriphenylphosphoniumjodid.

E23 5(RS ),6(RS)-1-Acetoxy-5,6-epoxy-7,11,14-cis-9-trans-eicosatetraen

aus 0.3 g 6(RS),7(RS)-11-Acetoxy-6,7-epoxy-2-trans-4-cis-undecadienal (D3b) und 0.8 g 3-cis-Nonenyltriphenylphosphoniumjodid.

Beispiele der pharmazeutischen Zusammensetzungen
und entsprechender fertiger Arzneimittelformen.

Unter den Ausdruck "Wirkstoff" ist nachstend eine erfindungsgemässe Verbindung der Formel I zu verstehen, insbesondere eine solche, die als Produkt in Beispielen 1-75 beschrieben ist, wie z.B. S-[5(RS)-,6(SR)-5-Hydroxy-7-cis-pentadecen-6-yl]-cystein-methylester; N-{S-[5(RS),6(SR)-5-Hydroxy-7-cis-heptadecen-6-yl]-N-trifluoracetyl-cysteinyl}-glycin-methylester; N-{S-[5(RS),6(SR)-5-Hydroxy-7-trans-9-cis-nonadecadien-6-yl]-N-trifluoracetyl-cysteinyl}-glycin-Natriumsalz; N-{S-[5(RS),6(SR)-5-Hydroxy-7,9-trans-11-cis-eicosatrien-6-yl]-N-trifluoracetyl-cysteinyl}-glycin-methylester; N-{S-[5(RS),6(SR)-5-Hydroxy-7,9-trans-11,14-cis-eicosatetraen-6-yl]-cysteinyl}-glycin (auch in optischer aktiver Form); sowie N-{S-[5(RS),6(SR)-1,5-Dihydroxy-7,9-trans-11,14-cis-eicosatetraen-6-yl]-cysteinyl}-glycin-Kaliumsalz (auch in optisch aktiver Form).

Beispiel A : Eine treibmittelhaltige, feststoffaerosolbildende Inhalationssuspension enthaltend 0,1 Gew.-% Wirkstoff.

| Zusammensetzung: | Gew.-% |
|---|---|
| Wirkstoff, mikronisiert | 0,1 |
| Sorbitantrioleat | 0,5 |
| Treibmittel A | |
| (Trichlortrifluorethan) | 4,4 |
| Treibmittel B | |
| (Dichlordifluormethan und | 15,0 |
| 1,2-Dichlortetrafluorethan) | 80,0 |

Herstellung : Der Wirkstoff wird unter Feuchtigkeitsausschluss mit Hilfe eines üblichen Homogenisators unter Zusatz des Sorbitantrioleats im Trichlortrifluorethan suspendiert, die Suspension in einen mit Dosierventil versehenen Aerosolbehälter abgefüllt; dieser wird verschlossen und unter Druck mit dem Treibmittel B aufgefüllt.

Beispiel B: Eine zur Inhalation geeignete, etwa 2%-ige wässrige Lösung eines Wirkstoffes in Form dessen Natrium- oder Kalium-salzes.

| Zusammensetzung | | |
|---|---|---|
| Wirkstoff (K- oder Na-Salz) | | 2000 mg |
| Ethylendiamintetraessigsäure- | | |
| Dinatriumsalz | | 10 mg |
| Benzalkoniumchlorid | | 10 mg |
| Wasser, frisch destilliert | ad | 100 ml |

Herstellung : Der Wirkstoff wird in etwa 60 ml frisch destilliertem Wasser gelöst und der Stabilisator (Ethylendiamintetraessigsäure-Dinatriumsalz) und das Konservierungsmittel (Benzalkoniumchlorid) hinzugegeben. Nach vollständiger Auflösung aller Komponenten wird die erhaltene Lösung auf 100 ml aufgefüllt, in Druckfläschchen abgefüllt und diese gasdicht verschlossen. Das Treibmittel wird nach Bedarf gasförmig unter Druck oder in flüssiger Form zugegeben.

ANHANG - PHARMAKOLOGISCHE TESTMETHODEN

Meerschweinchen-Bronchokonstriktionstest (in vivo, Aerosol ):

Man anästhetisiert männliche, 400-700 g schwere Meerschweinchen intraperitoneal mit 1.4 g/kg Urethan und führt eine Polyethylenkanüle in die Vena jugularis ein. Eine zweite Polyethylenkanüle wird in die Trachea eingeführt. Mittels einer in die Speiseröhre eingeführten Kanüle, welche mit einem Statham-Druck-Transduktor verbunden ist, wird der Druck in der Speiseröhre aufgezeichnet. Das Tier wird in eine luftdicht verschliessbare Plexiglaskammer gelegt, die mit einer Fleisch'schen Röhre Nr. 000 und einem Validyne-Transducer MP 45-1 verbunden ist. Mit dieser Anordnung wird der Flow gemessen.

Nach der chirurgischen Präparierung der Versuchstiere wartet man eine gewisse Zeit, damit die pulmonalen Funktionen sich stabilisieren können. Die zu testende Verbindung wird anschliessend gemäss dem nachfolgenden Protokoll verabreicht. Die Versuchstiere werden während einer Minute einer 1-prozentigen Aerosollösung der zu testenden Verbindung (Gew/Vol) oder destilliertem Wasser (zu Kontrollzwecken) ausgesetzt. Für alle Testverbindungen, welche durch Inhalation verabreicht werden, verwendet man ein Monaghan-Ultraschall-Sprühgerät (Modell 670) dessen Partikelgrösse sich zwischen 1 und 8 Mikron bewegt

mit einem Hauptanteil von 3 Mikron.

Wässrige Lösungen werden jeweils frisch hergestellt und mit einem On-stream drug vial in die Kammer des Sprühgeräts eingeführt. Der produzierte Sprühnebel wird den Versuchstieren über eine Glaskammer von 65 ml Inhalt, die mit einer Kanüle mit der Trachea verbunden wird, verabreicht. Nach Ablauf der Behandlungszeit wird mit einem zweiten Monaghan-Ultraschall-Sprühgerät (Modell 670) und über eine gleiche Glaskammer $LTD_4$ (0,3 µg/ml) während 2 Minuten verabreicht.

Es wird die Abnahme der Compliance in der 3. Minute nach $LTD_4$-Applikation abgelesen und zwar wird der Mittelwert von drei Tieren mit dem Mittelwert von drei Kontrolltieren verglichen und die prozentuale Hemmung der Compliance nach folgender Formel errechnet:

$$\% \text{ Hemmung} = 100 - \frac{(100 - \text{Compliance Präparat}) \cdot 100}{(100 - \text{Compliance Kontrolle})}$$

Werden unterschiedliche Wirkstoffkonzentrationen untersucht, so wird die prozentuale Hemmung für jede Konzentration aufgezeichnet, und zwar wird der log Konzentration auf der Abszisse gegen die prozentuale Hemmung auf der Ordinate aufgetragen. Die $IC_{50}$ wird dann durch lineare Regressionsanalyse ermittelt.

<u>In vitro-Test zur Bestimmung der Hemmung der Phospholipase $A_2$ aus menschlichen Leukozyten</u>

Neutrophile polymorphkernige menschliche Leukozyten werden ausgehend von "Buffy coats" durch mehrstufige fraktionierte Sedimentation isoliert und tiefgefroren. Die Phospholipase $A_2$ wird aus der Zellsuspension durch Homogenisieren unter Zusatz von eiskalter 0.36 N $H_2SO_4$ in 2N NaCl extrahiert und der nach Zentrifugation bei 10'000 x g erhaltene Ueberstand gegen Natriumacetatpuffer pH 4.5 dialysiert

Für die Bestimmung der Enzymaktivität wird Enzym (10-30 µg Protein) in 0.1 M Tris/HCl-Puffer pH 7 unter Zusatz von 1 mM $CaCl_2$ und Substrat, bestehend aus biosynthetisch mit $^{14}C$-Oelsäure radioaktiv markiertem Phospholipiden (2µM ) von Escherichia coli bei 37° während 1 Stunde inkubiert. Die Reaktion wird abgestoppt durch Zugabe von Dole-Reagens (Isopropanol/Heptan/1N $H_2SO_4$ 40:10:1, v/v) und die durch Phospholipase $A_2$ selektiv freigesetzte $^{14}C$-Oelsäure extrahiert. Ebenfalls mitextrahiertes Substrat wird durch Filtrationdes Extraktes durch eine Säule von Kieselgel vollständig entfernt. Die Bestimmung der $^{14}C$-Oelsäure im Eluat erfolgt durch Radiometrie.

Zur Ermittlung einer Hemmwirkung von Prüfsubstanzen auf die Phospholipase $A_2$ werden diese als Lösungen in Wasser, Dimethylsulfoxid (Endkonzentration im Ansatz bis 5 %, v/v) oder Ethanol (Endkonzentration im Ansatz bis 2.5 % v/v) dem Inkubationsumsatz zugesetzt. Die Wirkungsstärke der Prüfsubstanzen wird ausgedrückt durch die $IC_{50}$, d.h. die Konzentration, welche eine Hemmung von 50 % der Kontrollaktivität bewirkt. Die $IC_{50}$ wird graphisch ermittelt durch Auftragen der prozentualen Hemmung auf der Ordinate gegen den log der Konzentration (µM) auf der Abszisse.

Unter den beschriebenen Versuchsbedingungen hemmt Mepacrin die Phospholipase $A_2$ mit einer $IC_{50}$ von 1600 µM.

<u>In vitro-Test zur Bestimmung der Hemmung der Phospholipase C aus menschlichen Thrombozyten</u>

Menschliche Thrombozyten werden aus "Buffy coats" durch fraktionierte Zentrifugation gewonnen und anschliessend tiefgefroren. Die Phospholipase C wird durch Ultraschallbehandlung der Zellsuspension freigesetzt und findet sich nach Ultrazentrifugation (150'000 x g während 1 Stunde) in löslicher Form im Ueberstand.

Für die Bestimmung der Enzymaktivität wird Enzym (20-100 µg Protein) in 0.025 M Tris/Maleat-Puffer pH 6 unter Zusatz von 0.2 mM $CaCl_2$ und 0.02 mM radioaktiv markiertem Substrat, Phosphatidyl-[$^{14}C$]-inosit, bei 37° während 5 Minuten inkubiert. Die Reaktion wird abgestoppt durch Ausschütteln mit $CHCl_3/CH_3OH$ 2:1 (v/v). Dabei wird unverbrauchtes Substrat in die organische Phase extrahiert, während das Reaktionsprodukt $^{14}C$-Inositphophat in der wässrigen Phase verbleibt und durch Radiometrie eines Aliquots gemessen werden kann.

Zur Ermittlung einer Hemmwirkung von Prüfsubstanzen auf die Phospholipase C werden diese als Lösungen in Wasser, Dimethylsulfoxid (Endkonzentration im Ansatz bis 5 % v/v) oder Ethanol

(Endkonzentration im Ansatz bis 2.5 %, v/v) dem Inkubationsansatz zugesetzt. Die Wirkungsstärke der Prüfsubstanzen wird ausgedruckt durch die $IC_{50}$, d.h. die Konzentration, welche eine Hemmung von 50 % der Kontrollaktivität bewirkt. Die $IC_{50}$ wird graphisch ermittelt durch Auftragen der prozentualen Hemmung auf der Ordinate gegen den log der Konzentration ($\mu$M) auf der Abszisse.

Unter den beschriebenen Versuchsbedingungen hemmt Mepacrin die Phospholipase C mit einer $IC_{50}$ von 20 $\mu$M.

## Ansprüche

1. Eine Verbindung der Formel

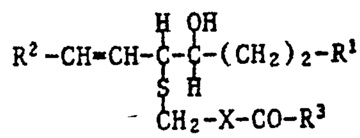

(I)

worin

$R^1$ ein $C_{1-3}$-Alkyl oder ein $C_{1-3}$-Hydroxyalkyl, dessen Hydroxylgruppe in veresterter Form vorliegen kann,

$R^2$ einen gesättigten oder ungesättigten aliphatischen Rest mit 5-15 C-Atomen,

$R^3$ Hydroxyl, Alkoxy oder eine gegebenenfalls substituierte Aminogruppe, und

-X- eine Einfachbindung, eine Methylengruppe oder eine gegebenenfalls N-acylierte primäre Aminomethylengruppe

darstellt, wobei das O-Atom der Hydroxylgruppe mit dem S-Atom zueinander in der relativen trans -Konfiguration stehen, sowie Salze solcher Verbindungen mit salzbildenden Eigenschaften.

2. Eine Verbindung gemäss Anspruch 1, worin in Formel I $R^1$ Methyl, Ethyl, Propyl oder ein gegebenenfalls durch eine $C_{1-4}$-Alkansäure verestertes $\beta$-Hydroxyethyl, $R^2$ ein lineares $C_{5-15}$-Alkyl oder einen entsprechenden Rest mit 1-3 Doppelbindungen, $R^3$ Hydroxyl, $C_{1-7}$-Alkoxy oder den Rest der Partialformel $-NH-CH_2-COR^3{}_b$ (wobei $R^3{}_b$ Hydroxyl oder $C_{1-7}$-Alkoxy ist), und -X- eine Einfachbindung, die Methylengruppe oder die Gruppe der Partialformel

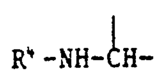

darstellt, worin $R^4$ Wasserstoff oder den Acylrest einer gegebenenfalls halogenierten Carbonsäure mit höchstens 12 C-Atomen oder einer $\alpha$-Aminosäure bedeutet, sowie Salze von Verbindungen mit salzbildenden Eigenschaften.

3. Eine Verbindung gemäss Anspruch 2, worin in Formel I $R^1$ und $R^2$ die dort angegebenen Bedeutungen haben und die Gruppierung $-S-CH_2-X-CO-R^3$ einen durch das S-Atom gebundenen Rest der Mercaptoessigsäure, $\beta$-Mercaptopropionsäure, N-($\beta$-Mercaptopropionyl)-glycins, oder eines durch Trifluoressigsäure gegebenenfalls N-acylierten L-Cysteins oder einen gegebenenfalls N-acylierten N-(L-Cysteinyl)-glycyl-Rest der Formel

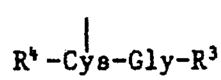

,

in welcher $R^3$ Hydroxyl oder $C_{1-4}$-Alkoxy und $R^4$ Wasserstoff, Trifluoracetyl oder $\gamma$-Glutamyl ist, darstellt.

4. N-{S-[5(RS),6(SR)-5-Hydroxy-7,9-trans-11-cis-eicosatrien-6-yl]-N-trifluoracetyl-cysteinyl}-glycin und sein individuelles 5(S),6(R)-Diastereomeres, jeweils in Form von Methylester, freier Säure oder eines Alkalimetallsalzes, als Verbindungen gemäss Anspruch 1.

5. N-{S-[5(R),6(S)-5-Hydroxy-7,9-trans-11-cis-eicosatrien-6-yl]-N-trifluoracetyl-cysteinyl}-glycin in Form des Natrium- oder Kaliumsalzes, als Verbindungen gemäss Anspruch 1.

6. N-{S-[5(RS),6(SR)-5-Hydroxy-7,9-trans-11,14-cis-eicosatetraen-6-yl]-cysteinyl}-glycin und dessen individuelle 5(S) ,6(R)- und 5(R),6(S)-Diastereomere, jeweils in Form von Methylester, freier Säure oder eines Alkalimetallsalzes, als Verbindungen gemäss Anspruch 1.

7. Eine Verbindung gemäss einem der Ansprüche 1-6 zur Verwendung als Leukotrien-Antagonist und/oder zur Hemmung von Phospholipasen.

8. Verfahren zur Herstellung von Verbindungen der in Anspruch 1 definierten Formel I, dadurch gekennzeichnet, dass man ein trans-Epoxid der Formel

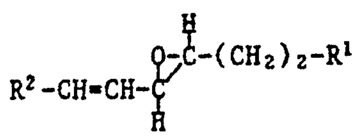

(II)

worin $R^1$ und $R^2$ die im Anspruch 1 angegebenen Bedeutungen haben und in welchem eine gegebenenfalls vorhandene Hydroxylgruppe in einer geschützten Form vorliegen kann, mit einem Mercaptoalkancarbonsäure-Derivat der Formel

$$HS\text{-}CH_2\text{-}X\text{-}CO\text{-}R^3 \qquad \text{(III)}$$

worin $R^3$ und -X- die im Anspruch 1 angegebenen Bedeutungen haben und in welchem eine gegebenenfalls vorhandene Aminogruppe in einer geschützten Form vorliegen kann, umsetzt und wenn notwendig oder erwünscht, die Schutzgruppe(n) der Hydroxyl- und/oder Aminogruppe(n) abspaltet und/oder eine als Ester vorliegende Verbindung zur freien Säure oder einem Salz verseift und/oder eine erhaltene freie Verbindung mit salzbildenden Eigenschaften in ein Salz umwandelt und/oder eine Verbindung aus einer entsprechenden Salzform freisetzt.

9. Pharmazeutische Zusammensetzungen enthaltend als Wirkstoff mindestens eine der Verbindungen gemäss einem der Ansprüche 1-7 zusammen mit mindestens einem pharmazeutisch annehmbaren Trägermaterial.

10. Verwendung der Verbindungen gemäss einem der Ansprüche 1-34 zur Herstellung einer pharmazeutischen Zusammensetzung.

Patentansprüche für folgenden Vertragsstaat: AT

1. Verfahren zur Herstellung einer Verbindung der Formel

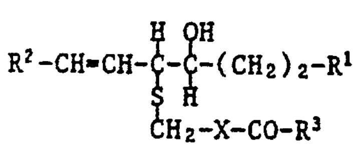

(I)

worin

$R^1$ ein $C_{1-3}$-Alkyl oder ein $C_{1-3}$-Hydroxyalkyl, dessen Hydroxylgruppe in veresterter Form vorliegen kann,

$R^2$ einen gesättigten oder ungesättigten aliphatischen Rest mit 5-15 C-Atomen,
$R^3$ Hydroxyl, Alkoxy oder eine gegebenenfalls substituierte Aminogruppe, und
-X- eine Einfachbindung, eine Methylengruppe oder eine gegebenenfalls N-acylierte primäre Aminomethylengruppe

darstellt, wobei das O-Atom der Hydroxylgruppe mit dem S-Atom zueinander in der relativen trans -Konfiguration stehen, oder eines Salzes solcher Verbindung mit salzbildenden Eigenschaften, dadurch gekennzeichnet, dass man ein trans-Epoxid der Formel

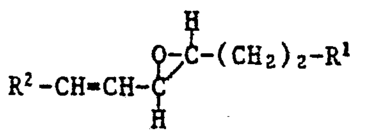

(II)

worin $R^1$ und $R^2$ die oben angegebenen Bedeutungen haben und in welchem eine gegebenenfalls vorhandene Hydroxylgruppe in einer geschützten Form vorliegen kann, mit einem Mercaptoalkancarbonsäure-Derivat der Formel

$$HS-CH_2-X-CO-R^3 \qquad (III)$$

worin $R^3$ und -X- die oben angegebenen Bedeutungen haben und in welchem eine gegebenenfalls vorhandene Aminogruppe in einer geschützten Form vorliegen kann, umsetzt und wenn notwendig oder erwünscht, die Schutzgruppe(n) der Hydroxyl- und/oder Aminogruppe(n) abspaltet und/oder eine als Ester vorliegende Verbindung zur freien Säure oder einem Salz verseift und/oder eine erhaltene freie Verbindung mit salzbildenden Eigenschaften in ein Salz umwandelt und/oder eine Verbindung aus einer entsprechenden Salzform freisetzt.

**2.** Verfahren gemäss Anspruch 1, dadurch gekennzeichnet, dass man eine Verbindung der Formel I herstellt, worin $R^2$ ein lineares Alkyl, 1-Alkenyl oder 1,3-Alkadienyl mit 5-12 C-Atomen, oder ein lineares 1,3,6-Alkatrienyl mit 8-12 C-Atomen darstellt.

**3.** Verfahren gemäss Anspruch 1, dadurch gekennzeichnet, dass man eine Verbindung der Formel I herstellt, worin $R^1$ Methyl, Ethyl, Propyl oder ein gegebenenfalls durch eine $C_{1-4}$-Alkansäure verestertes $\beta$-Hydroxyethyl, $R^2$ ein lineares $C_{5-15}$-Alkyl oder einen entsprechenden Rest mit 1-3 Doppelbindungen, $R^3$ Hydroxyl, $C_{1-7}$-Alkoxy oder den Rest der Partialformel $-NH-CH_2-COR^3_b$ (wobei $R^3_b$ Hydroxyl oder $C_{1-7}$-Alkoxy ist), und -X- eine Einfachbindung, die Methylengruppe oder die Gruppe der Partialformel

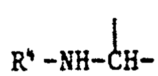

darstellt, worin $R^4$ Wasserstoff oder den Acylrest einer gegebenenfalls halogenierten Carbonsäure mit höchstens 12 C-Atomen oder einer $\alpha$-Aminosäure bedeutet, sowie Salze von Verbindungen mit salzbildenden Eigenschaften.

**4.** Verfahren gemäss einem der Ansprüche 1-3, dadurch gekennzeichnet, dass man eine Verbindung der Formel I herstellt, worin $R^1$ und $R^2$ die dort angegebenen Bedeutungen haben und die Gruppierung $-S-CH_2-X-CO-R^3$ einen durch das S-Atom gebundenen Rest der Mercaptoessigsäure, der $\beta$-Mercaptopropionsäure, des N-($\beta$-Mercaptopropionyl)-glycinsoder eines durch Trifluoressigsäure gegebenenfalls N-acylierten L-Cysteinyls oder einen gegebenenfalls N-acylierten N-(L-Cysteinyl)-glycyl-Rest der Formel

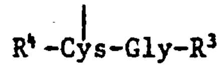

,

in welcher $R^3$ Hydroxyl oder $C_{1-4}$-Alkoxy und $R^4$ Wasserstoff, Trifluoracetyl oder $\gamma$-Glutamyl ist,

darstellt.

5. Verfahren gemäss Anspruch 1, dadurch gekennzeichnet, dass man N-{S-[5(RS),6(SR)-5-Hydroxy-7,9-trans-11-cis-eicosatrien-6-yl]-N-trifluoracetyl-cysteinyl}-glycin oder sein individuelles 5(S),6(R)-5-Diastereomeres in Form von Methylester, freier Säure oder eines Alkalimetallsalzes herstellt.

6. Verfahren gemäss Anspruch 1, dadurch gekennzeichnet, dass man N-{S-[5(R),6(S)-5-Hydroxy-7,9-trans-11-cis-eicosatrien-6-yl]-N-trifluoracetyl-cysteinyl}-glycin in Form das Natrium oder Kaliumsalzes herstellt.

7. Verfahren gemäss Anspruch 1, dadurch gekennzeichnet, dass man N-{S-[5(RS),6(SR)-5-Hydroxy-7,9-trans-11,14-cis-eicosatetraen-6-yl]-cysteinyl}-glycin oder dessen individuelles 5(S) ,6(R)- oder 5(R),6(S)-Diastereomeres in Form von Methylester, freier Säure oder eines Alkalimetallsalzes herstellt.

8. Verfahren gemäss Anspruch 1, dadurch gekennzeichnet, dass man ein physiologisch verträgliches Salz einer der Verbindungen gemäss einem der Ansprüche 1-5 und 7, welche mindestens eine freie Carboxylgruppe aufweist, herstellt.

9. Verfahren gemäss Anspruch 1, dadurch gekennzeichnet, dass man eine Verbindung gemäss einem der Ansprüche 1-8 zur Verwendung als Leukotrien-Antagonist und/oder zur Hemmung von Phospholipasen herstellt.

10. Verfahren zur Herstellung einer pharmazeutischen Zusammensetzung enthaltend als Wirkstoff mindestens eine der Verbindungen gemäss einem der Ansprüche 1-8 zusammen mit mindestens einem pharmazeutisch annehmbaren Trägermaterial, dadurch gekennzeichnet, dass man die entsprechenden Bestandteile auf nicht-chemischem Wege vermischt und, wenn eine fertige Arzneimittelform erwünscht ist, in geeignete Behälter in abgemessenen Mengen abfüllt.

## Claims

1. A compound of the formula

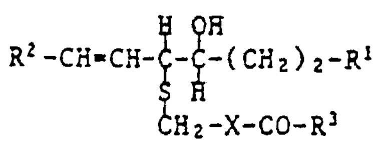

(I)

in which

- $R^1$ represents a $C_{1-3}$-alkyl radical or a $C_{1-3}$-hydroxyalkyl radical of which the hydroxy group may be in esterified form,
- $R^2$ represents a saturated or unsaturated aliphatic radical having from 5 to 15 carbon atoms,
- $R^3$ represents hydroxy, alkoxy or an optionally substituted amino group, and
- -X- represents a single bond, a methylene group or an optionally N-acylated primary aminomethylene group

wherein the O-atom of the hydroxy group is in the trans-configuration relative to the S-atom, and salts of such compounds having salt-forming properties.

2. A compound according to claim 1, wherein, in formula I, $R^1$ represents methyl, ethyl, propyl, or a $\beta$-hydroxyethyl group optionally esterified by a $C_{1-4}$-alkanoic acid, $R^2$ represents a linear $C_{5-15}$-alkyl radical or a corresponding radical having from 1 to 3 double bonds, $R^3$ represents hydroxy, $C_{1-7}$-alkoxy or a radical of the partial formula $-NH-CH_2-COR^3_b$ (in which $R^3_b$ represents hydroxy or $C_{1-7}$-alkoxy) and -X- represents a single bond, a methylene group or a group of the partial formula

$$R^4-NH-CH-$$

in which $R^4$ represents hydrogen or the acyl radical of an optionally halogenated carboxylic acid having a maximum of 12 carbon atoms or of an α-amino acid, and salts of compounds having salt-forming properties.

**3.** A compound according to claim 2, wherein, in formula I, $R^1$ and $R^2$ have the meanings given therein and the grouping $-S-CH_2-X-CO-R^3$ represents a residue, bonded by the S-atom, of mercaptoacetic acid, of β-mercaptopropionic acid, of N-(β-mercaptopropionyl)-glycine, or of an L-cysteine optionally N-acylated by trifluoroacetic acid, or represents an optionally N-acylated N-(L-cysteinyl)-glycyl residue of the formula

$$R^4-Cys-Gly-R^3$$

in which $R^3$ represents hydroxy or $C_{1-4}$-alkoxy and $R^4$ represents hydrogen, trifluoroacetyl or γ-glutamyl.

**4.** N-{S-[5(RS),6(SR)-5-Hydroxy-7,9-trans-11-cis-icosatrien-6-yl]-N-trifluoroacetylcysteinyl}-glycine and its individual 5(S),6(R)-diastereoisomer, each in the form of the methyl ester, free acid or an alkali metal salt, as compounds according to claim 1.

**5.** N-{S-[5(R),6(S)-5-Hydroxy-7,9-trans-11-cis-icosatrien-6-yl]-N-trifluoroacetylcysteinyl}-glycine in the form of the sodium or potassium salt, as compounds according to claim 1.

**6.** N-{S-[5(RS),6(SR)-5-Hydroxy-7,9-trans-11,14-cis-icosatetraen-6-yl]-cysteinyl}-glycine and its individual 5(S),6(R)- and 5(R),6(S)-diastereoisomers, each in the form of the methyl ester, free acid or an alkali metal salt, as compounds according to claim 1.

**7.** A compound according to any one of claims 1 to 6 for use as a leucotriene-antagonist and/or for the inhibition of phospholipases.

**8.** A process for the manufacture of compounds of the formula I defined in claim 1, characterized in that a trans-epoxide of the formula

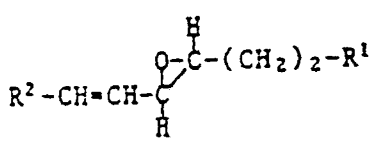

(II)

in which $R^1$ and $R^2$ have the meanings given in claim 1 and in which a hydroxy group, if present, may be in protected form is reacted with a mercaptoalkanecarboxylic acid derivative of the formula

$$HS-CH_2-X-CO-R^3 \qquad (III)$$

in which $R^3$ and -X- have the meanings given in claim 1 and in which an amino group, if present, may be in protected form and, if necessary or desired, the protecting group(s) of the hydroxy and/or amino group(s) is (are) removed and/or a compound present in the form of an ester is hydrolysed to the free acid or a salt, and/or a resulting free compound having salt-forming properties is converted into a salt and/or a compound is freed from a corresponding salt form.

**9.** Pharmaceutical compositions containing as active ingredient at least one of the compounds according to

any one of claims 1 to 7 together with at least one pharmaceutically acceptable carrier.

**10.** The use of the compounds according to any one of claims 1 to 34 for the manufacture of a pharmaceutical composition.

Claims for the following Contracting State: AT

**1.** A process for the manufacture of a compound of the formula

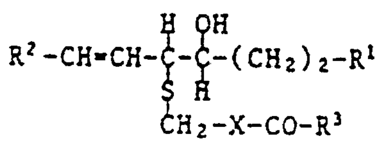

(I)

in which

$R^1$ represents a $C_{1-3}$-alkyl radical or a $C_{1-3}$-hydroxyalkyl radical of which the hydroxy group may be in esterified form,

$R^2$ represents a saturated or unsaturated aliphatic radical having from 5 to 15 carbon atoms,

$R^3$ represents hydroxy, alkoxy or an optionally substituted amino group, and

-X- represents a single bond, a methylene group or an optionally N-acylated primary aminomethylene group

wherein the O-atom of the hydroxy group is in the trans-configuration relative to the S-atom, or a salt of such a compound having salt-forming properties, characterised in that a trans-epoxide of the formula

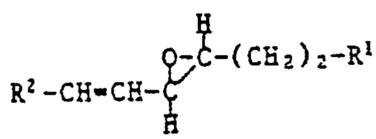

(II)

in which $R^1$ and $R^2$ have the meanings given above and in which a hydroxy group, if present, may be in protected form is reacted with a mercaptoalkanecarboxylic acid derivative of the formula

$$HS-CH_2-X-CO-R^3 \quad (III)$$

in which $R^3$ and -X- have the meanings given above and in which an amino group, if present, may be in protected form and, if necessary or desired, the protecting group(s) of the hydroxy and/or amino group(s) is (are) removed and/or a compound present in the form of an ester is hydrolysed to the free acid or a salt, and/or a resulting free compound having salt-forming properties is converted into a salt and/or a compound is freed from a corresponding salt form.

**2.** A process according to claim 1, characterised in that a compound of formula I in which $R^2$ represents a linear alkyl, 1-alkenyl or 1,3-alkadienyl having from 5 to 12 carbon atoms, or a linear 1,3,6-alkatrienyl having from 8 to 12 carbon atoms, is manufactured.

**3.** A process according to claim 1, characterised in that a compound of formula I in which $R^1$ represents methyl, ethyl, propyl, or a $\beta$-hydroxyethyl group optionally esterified by a $C_{1-4}$-alkanoic acid, $R^2$ represents a linear $C_{5-15}$-alkyl radical or a corresponding radical having from 1 to 3 double bonds, $R^3$ represents hydroxy, $C_{1-7}$-alkoxy or a radical of the partial formula $-NH-CH_2-COR^3{}_b$ (in which $R^3{}_b$ represents hydroxy or $C_{1-7}$-alkoxy) and -X- represents a single bond, a methylene group or a group of the partial formula

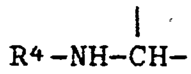

in which $R^4$ represents hydrogen or the acyl radical of an optionally halogenated carboxylic acid having a maximum of 12 carbon atoms or of an $\alpha$-amino acid, and salts of compounds having salt-forming properties, are manufactured.

4. A process according to any one of claims 1 to 3, characterised in that a compound of formula I in which $R^1$ and $R^2$ have the meanings given therein and the grouping $-S-CH_2-X-CO-R^3$ represents a residue, bonded by the S-atom, of mercaptoacetic acid, of $\beta$-mercaptopropionic acid, of N-($\beta$-mercaptopropionyl)-glycine, or of an L-cysteinyl optionally N-acylated by trifluoroacetic acid, or represents an optionally N-acylated N-(L-cysteinyl)-glycyl residue of the formula

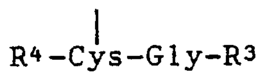

in which $R^3$ represents hydroxy or $C_{1-4}$-alkoxy and $R^4$ represents hydrogen, trifluoroacetyl or $\gamma$-glutamyl, is manufactured.

5. A process according to claim 1, characterised in that N-{S-[5(RS),6(SR)-5-hydroxy-7,9-trans-11-cis-icosatrien-6-yl]-N-trifluoroacetylcysteinyl}-glycine or its individual 5(S),6(R)-diastereoisomer, in the form of the methyl ester, free acid or an alkali metal salt, is manufactured.

6. A process according to claim 1, characterised in that N-{S-[5(R),6(S)-5-hydroxy-7,9-trans-11-cis-icosatrien-6-yl]-N-trifluoroacetylcysteinyl}-glycine in the form of the sodium or potassium salt is manufactured.

7. A process according to claim 1, characterised in that N-{S-[5(RS),6(SR)-5-hydroxy-7,9-trans-11,14-cis-icosatetraen-6-yl]-cysteinyl}-glycine or its individual 5(S),6(R)- or 5(R),6(S)-diastereoisomer, in the form of the methyl ester, free acid or an alkali metal salt, is manufactured.

8. A process according to claim 1, characterised in that a physiologically tolerable salt of a compound according to any one of claims 1 to 5 and 7 that contains at least one free carboxy group is manufactured.

9. A process according to claim 1, characterised in that a compound according to any one of claims 1 to 8 for use as a leucotriene-antagonist and/or for the inhibition of phospholipases is manufactured.

10. A process for the manufacture of a pharmaceutical composition containing as active ingredient at least one compound according to any one of claims 1 to 8 together with at least one pharmaceutically acceptable carrier, characterised in that the corresponding constituents are mixed by non-chemical methods and, if a ready-prepared form of medicament is desired, filled in measured amounts into suitable containers.

**Revendications**

1. Un composé de formule

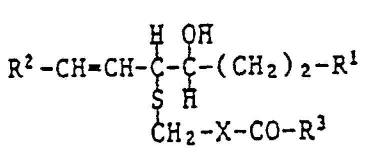

(I)

dans laquelle

$R^1$ représente un groupe alkyle en C1-C3 ou hydroxyalkyle en C1-C3 dont le groupe hydroxy peut être à l'état estérifié,
$R^2$ représente un radical aliphatique saturé ou insaturé en C5-C15,
$R^3$ représente un groupe hydroxy, alcoxy ou un groupe amino éventuellement substitué, et
-X- représente une liaison simple, un groupe méthylène ou un groupe (amino primaire)-méthylène éventuellement acylé sur l'azote,
l'atome d'oxygène du groupe hydroxy étant en configuration relative trans avec l'atome de soufre, et les sels de ces composés qui sont salifiables.

2. Un composé selon la revendication 1, pour lequel, dans la formule I, $R^1$ représente un groupe méthyle, éthyle, propyle, ou un groupe bêta-hydroxyéthyle éventuellement estérifié par un acide alcanoïque en C1-C4, $R^2$ représente un groupe alkyle linéaire en C5-C15 ou un groupe correspondant contenant 1 à 3 doubles liaisons, $R^3$ représente un groupe hydroxy, alcoxy en C1-C7 ou le radical de formule partielle $-NH-CH_2-COR^3{}_b$ (dans lequel $R^3{}_b$ représente un groupe hydroxy ou alcoxy en C1-C7) et -X- représente une liaison simple, un groupe méthylène ou un groupe de formule partielle

$$R^4-NH-CH-$$

dans laquelle $R^4$ représente l'hydrogène ou le radical acyle d'un acide carboxylique éventuellement halogéné à 12 atomes de carbone au maximum ou d'un alpha-aminoacide, et les sels de ces composés qui sont salifiables.

3. Un composé selon la revendication 2, pour lequel, dans la formule I, $R^1$ et $R^2$ ont les significations indiquées en référence à cette formule et le groupement $-S-CH_2-X-CO-R^3$ est un radical, relié par l'intermédiaire de l'atome de S, de l'acide mercapto-acétique, de l'acide bêta-mercapto-propionique, de la N-(bêta-mercaptopropionyl)-glycine ou d'une L-cystéine éventuellement acylée à l'azote par l'acide trifluoracétique, ou un radical N-(L-cystéinyl)-glycyle éventuellement acylé à l'azote, de formule

$$R^4-Cys-Gly-R^3$$

dans laquelle $R^3$ représente un groupe hydroxy ou alcoxy en C1-C4 et $R^4$ représente l'hydrogène, un groupe trifluoracétyle ou gamma-glutamyle.

4. La N- S-[5(RS),6(SR)-5-hydroxy-7,9-trans-11-cis-éicosatriène-6-yl]-N-trifluoracétyl-cystéinyl -glycine et son diastéréoisomère individuel 5(S),6(R), l'un comme l'autre à l'état d'ester méthylique, d'acide libre ou de sel de métal alcalin, en tant que composés selon la revendication 1.

5. La N- S-[5(R),6(S)-5-hydroxy-7,9-trans-11-cis-éicosatriène-6-yl]-N-trifluoracétyl-cystéinyl -glycine à l'état de sel de sodium ou de potassium, en tant que composé selon la revendication 1.

6. La N- S-[5(RS),6(SR)-5-hydroxy-7,9-trans-11, 14-cis-éicosatétraène-6-yl]-cystéinyl -glycine et ses diastéréoisomères individuels 5(S),6(R) et 5(R),6(S), les uns comme les autres à l'état d'esters méthyliques, d'acides libres ou de sels de métaux alcalins, en tant que composés selon la revendication 1.

7. Un composé selon l'une des revendications 1 à 6 pour l'utilisation en tant qu'antagoniste des leucotriènes et/ou pour l'inhibition des phospholipases.

8. Procédé de préparation des composés de formule I de la revendication 1, caractérisé en ce que l'on fait réagir un trans-époxyde de formule

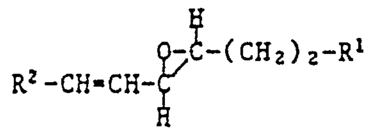

(II)

dans laquelle $R^1$ et $R^2$ ont les significations indiquées dans la revendication 1, un groupe hydroxy éventuellement présent pouvant être à l'état protégé, avec un dérivé d'acide mercaptoalcanoïque de formule

$$HS-CH_2-X-CO-R^3 \qquad (III)$$

dans laquelle $R^3$ et -X- ont les significations indiquées dans la revendication 1, un groupe amino éventuellement présent pouvant être à l'état protégé, et lorsque c'est nécessaire ou lorsqu'on le désire, on élimine le ou les groupes protecteurs des groupes hydroxy et/ou amino, et/ou on saponifie un composé à l'état d'ester en l'acide libre ou en un sel et/ou on convertit un composé obtenu à l'état libre et salifiable en un sel et/ou on libère un composé à partir d'un sel correspondant.

**9.** Compositions pharmaceutiques contenant en tant que substance active au moins l'un des composés selon l'une des revendications 1 à 7 avec au moins un véhicule acceptable pour l'usage pharmaceutique.

**10.** Utilisation des composés selon l'une des revendications 1 à 34 pour la préparation d'une composition pharmaceutique.

Revendications pour l'Etat contractant suivant: AT

**1.** Procédé de préparation d'un composé de formule

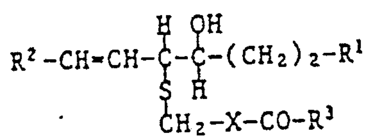

(I)

dans laquelle

- $R^1$ représente un groupe alkyle en C1-C3 ou hydroxyalkyle en C1-C3 dont le groupe hydroxy peut être à l'état estérifié,
- $R^2$ représente un radical aliphatique saturé ou insaturé en C5-C15,
- $R^3$ représente un groupe hydroxy, alcoxy ou un groupe amino éventuellement substitué, et
- -X- représente une liaison simple, un groupe méthylène ou un groupe (amino primaire)-méthylène éventuellement acylé à l'azote,

l'atome d'oxygène du groupe hydroxy étant en configuration relative trans par rapport à l'atome de S, ou d'un sel d'un tel composé qui est salifiable, caractérisé en ce que l'on fait réagir un trans-époxyde de formule

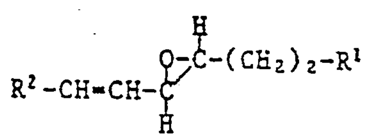

(II)

dans laquelle $R^1$ et $R^2$ ont les significations indiquées ci-dessus, un groupe hydroxy éventuellement présent pouvant être à l'état protégé, avec un dérivé d'acide mercapto-alcanoïque de formule

$$HS-CH_2-X-CO-R^3 \qquad (III)$$

dans laquelle $R^3$ et -X- ont les significations indiquées ci-dessus, un groupe amino éventuellement présent pouvant être à l'état protégé, et lorsque c'est nécessaire ou lorsqu'on le désire, on élimine le ou les groupes protecteurs des groupes hydroxy et/ou amino et/ou on saponifie un composé à l'état d'ester en l'acide libre ou en sel et/ou on convertit un composé obtenu à l'état libre et salifiable en un sel et/ou on libère un composé à partir d'un sel correspondant.

2. Procédé selon la revendication 1, caractérisé en ce que l'on prépare un composé de formule I dans laquelle $R^2$ représente un groupe alkyle, 1-alcényle ou 1,3-alcadiényle linéaire en C5-C12, ou un groupe 1,3,6-alcatriényle linéaire en C8-C12.

3. Procédé selon la revendication 1, caractérisé en ce que l'on prépare un composé de formule I dans laquelle $R^1$ représente un groupe méthyle, éthyle, propyle ou un groupe bêta-hydroxyéthyle éventuellement estérifié par un acide alcanoïque en C1-C4, $R^2$ représente un groupe alkyle linéaire en C5-C15 ou un groupe correspondant contenant 1 à 3 doubles liaisons, $R^3$ représente un groupe hydroxy, alcoxy en C1-C7 ou le radical de formule partielle $-NH-CH_2-COR^3_b$ (dans lequel $R^3_b$ représente un groupe hydroxy ou alcoxy en C1-C7) et -X- représente une liaison simple, le groupe méthylène ou le groupe de formule partielle

$$R^4-NH-\overset{|}{C}H-$$

dans laquelle $R^4$ représente l'hydrogène ou le radical acyle d'un acide carboxylique éventuellement halogéné contenant au maximum 12 atomes de carbone ou d'un alpha-amino-acide, ainsi que les sels de ces composés qui sont salifiables.

4. Procédé selon l'une des revendications 1 à 3, caractérisé en ce que l'on prépare un composé de formule I dans laquelle $R^1$ et $R^2$ ont les significations indiquées ci-dessus et le groupement $-S-CH_2-X-CO-R^3$ représente un radical, relié par l'intermédiaire de l'atome de S, de l'acide mercaptoacétique, de l'acide bêta-mercaptopropionique, de la N-(bêta-mercaptopropionyl)-glycine ou d'un groupe L-cystéinyle éventuellement acylé à l'azote par l'acide trifluoracétique, ou un radical de N-(L-cystéinyle)-glycyle éventuellement acylé à l'azote et répondant à la formule

$$R^4-\overset{|}{Cys}-Gly-R^3$$

dans laquelle $R^3$ représente un groupe hydroxy ou alcoxy en C1-C4 et $R^4$ représente l'hydrogène, un groupe trifluoracétyle ou gamma-glutamyle.

5. Procédé selon la revendication 1, caractérisé en ce que l'on prépare la N- S-[5(RS),6(SR)-5-hydroxy-7,9-trans-11-cis-éicosatriène-6-yl]-N-trifluoracétyl-cystéinyl -glycine ou son diastéréoisomère individuel 5(S)-,6(R)-5 à l'état d'ester méthylique, d'acide libre ou de sel de métal alcalin.

6. Procédé selon la revendication 1, caractérisé en ce que l'on prépare la N- S-[5(R),6(S)-5-hydroxy-7,9-trans-11-cis-éicosatriène-6-yl]-N-trifluoracétyl-cystéinyl -glycine à l'état de sel de sodium ou de potassium.

7. Procédé selon la revendication 1, caractérisé en ce que l'on prépare la N- S-[5(RS),6(SR)-5-hydroxy-7,9-trans-11,14-cis-éicosatétraène-6-yl]-cystéinyl -glycine ou son diastéréoisomère individuel 5(S),6(R) ou 5-(R),6(S) à l'état d'ester méthylique, d'acide libre ou de sel de métal alcalin.

8. Procédé selon la revendication 1, caractérisé en ce que l'on prépare un sel acceptable pour l'usage

pharmaceutique de l'un des composés selon l'une des revendications 1 à 5 et 7 contenant au moins un groupe carboxyle libre.

**9.** Procédé selon la revendication 1, caractérisé en ce que l'on prépare un composé selon l'une des revendications 1 à 8 pour l'utilisation en tant qu'antagoniste des leucotriènes et/ou pour l'inhibition des phospholipases.

**10.** Procédé de préparation d'une composition pharmaceutique contenant en tant que substance active au moins l'un des composés selon l'une des revendications 1 à 8 avec au moins un véhicule acceptable pour l'usage pharmaceutique, caractérisé en ce que l'on mélange les composants appropriés par voie non chimique et, lorsqu'on désire une forme médicamenteuse prête à l'emploi, on introduit le mélange en quantités dosées dans des récipients appropriés.